(19) **Europäisches Patentamt / European Patent Office / Office européen des brevets**

(11) **EP 2 177 230 A1**

(12) # EUROPEAN PATENT APPLICATION
published in accordance with Art. 153(4) EPC

(43) Date of publication:
**21.04.2010 Bulletin 2010/16**

(21) Application number: **08792162.3**

(22) Date of filing: **04.08.2008**

(51) Int Cl.:
*A61K 39/395* (2006.01)   *A61K 9/127* (2006.01)
*A61K 47/24* (2006.01)   *A61K 47/28* (2006.01)
*A61K 47/42* (2006.01)   *A61K 47/44* (2006.01)
*A61P 29/00* (2006.01)   *A61P 35/00* (2006.01)
*A61P 37/02* (2006.01)

(86) International application number:
**PCT/JP2008/063958**

(87) International publication number:
**WO 2009/020093 (12.02.2009 Gazette 2009/07)**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **09.08.2007   JP 2007208664**

(71) Applicant: **Daiichi Sankyo Company, Limited**
**Chuo-ku**
**Tokyo 103-8426 (JP)**

(72) Inventors:
• **MORITA, Koji**
  **c/o Daiichi Sankyo Co. LTD.**
  **Tokyo 140-8710 (JP)**

• **NIWA, Takako**
  **c/o Daiichi Sankyo Co LTD**
  **Tokyo 140-8710 (JP)**
• **ICHIKAWA, Kimihisa**
  **c/o Daiichi Sankyo CO LTD**
  **ToKyo 134-8630 (JP)**
• **Yoshida, Hiroko**
  **c/o Daiichi Sankyo Co Ltd**
  **Tokyo 134-8630 (JP)**

(74) Representative: **Arends, William Gerrit**
**Marks & Clerk LLP**
**90 Long Acre**
**London**
**WC2E 9RA (GB)**

(54) **IMMUNOLIPOSOME INDUCING APOPTOSIS INTO CELL EXPRESSING DEATH DOMAIN-CONTAINING RECEPTOR**

(57)    The present invention relates to an immunoliposome preparation having a therapeutic effect on cancer, autoimmune disease, or inflammatory disease. Specifically, the present invention relates to an immunoliposome comprising, as a constituent, an antibody capable of inducing the apoptosis of cells expressing a death domain-containing receptor.

EP 2 177 230 A1

**Description**

Technical Field

The present invention relates to an immunoliposome which contains an antibody binding to a cell surface receptor involved in apoptosis induction, has an apoptosis-inducing effect on cells expressing the cell surface receptor, and is useful as a therapeutic and/or preventive agent for tumors. The present invention also relates to a method for treating and/or preventing cancer, autoimmune disease, or inflammatory disease using the liposome.

Background Art

Liposomes have attracted a lot of interest as drug carriers, particularly, as carriers of the drug delivery system (DDS) for intravenous injection, since they can contain water-soluble or hydrophobic substances in large amounts (D.D. Lasic, "Liposomes: From Physics to Applications", Elsevier Science Publishers, (1993)). In recent years, liposomes surface-modified with a hydrophilic polymer, for example, polyethylene glycol (PEG), have been applied even to drugs having low in-vivo stability, since they are less aggregated and less captured by the reticulo-endothelial system *in vivo* than conventional liposomes. Thus, these surface-modified liposomes have been put to practical use in pharmaceutical techniques that achieve high stability in blood (half-life of the drug in blood: 20-40 hr). Furthermore, many attempts have been made in recent years to bind functional molecules such as proteins, peptides, or sugars onto the surface of liposomes (or to allow the surface of liposomes to contain these functional molecules) for the purpose of imparting functionality thereto.

Methods which comprise encapsulating drugs into liposomes and binding antibodies onto the surface thereof have been proposed as means for delivering drugs to particular sites *in vivo*. Particularly, in the field of cancer treatment, antibody-conjugated liposomes containing an antitumor agent encapsulated therein have been reported to be effective. Such liposomes containing an antibody are called immunoliposomes. In many cases, the pharmacological effects of the immunoliposomes are attributed to the antitumor agent encapsulated in the liposomes. By contrast, the antibody contained in the immunoliposomes is utilized for delivering these immunoliposomes to tumor cells through its binding to an antigen specifically expressed in the tumor cells. Thus, the antibody contained in the immunoliposomes generally needs only to have the function of binding to the tumor cell-specific antigen, and the antibody itself does not necessarily function as a therapeutic agent. For the liposomes thus rendered targetable (even PEG-liposomes), the majority of those administered are captured and degraded by immune cells in the liver or the like before arriving at the target cells, or a large amount of drug is lost in a solution. Therefore, only a limited amount of drug can actually arrive at the target sites and exert its activity. Thus, many attempts have been made to develop liposomes which are provided with the function of arriving at the target cells while more stably containing drugs, and efficiently releasing the encapsulated drugs at the target sites. However, such liposomes have not been put in practical use so far. Moreover, theoretically, every drug should be applied to liposomes. However, stable drug retention in blood largely depends on the physical interaction (compatibility) between lipid components of a liposome and the encapsulated drug.
Under present circumstances, limited types of drugs are actually applicable. Thus, for obtaining a higher pharmacological effect of a liposome which has a limited delivery and contains one of the limited types of drug encapsulated therein, the liposome needs to be provided with a further pharmacological effect in a new manner.

Cell surface receptors involved in apoptosis induction, typified by death receptors, are known to biologically trigger the induction of intracellular apoptotic signals through their local multimerization on the cell membranes caused by ligand binding (Cell Death and Differentiation, 10: 66-75 (2003)). Antibodies capable of binding to the cell surface receptors involved in apoptosis induction are now under clinical development as therapeutic drugs and are expected to have a therapeutic effect that acts agonistically in a manner specific for cells expressing the receptor (cancer cells/ immunological disease-associated cells) to kill these cells. The action of these antibodies is considered to be based on the mechanism through which the antibodies are multimerized through cross-linking before or after binding to the receptors, thereby causing the multimerization of the antigen receptors (i.e., apoptosis induction). The exhibition of their activities probably requires, in in-vitro experiments, artificially cross-linking the present antibodies by the addition of secondary antibodies thereto and requires, *in vivo,* the mechanism of action through which the antibodies are cross-linked by Fc receptors on immunological effector cells. Attempts have been made in recent years to further enhance the original functions of the antibodies by structurally modifying the antibodies. For example, it has been reported that affinity for Fc receptors is improved by removing a particular sugar chain structure on the antibodies. However, the amount or function of immunological effector cells varies among individuals. Moreover, the amount or function of immunological effector cells is largely reduced in drug-treated cancer patients or immunological disease patients. The existing antibodies might not produce a sufficient pharmacological effect on such cancer or immunological disease patients. Thus, it is required to further enhance the functions of the antibodies themselves.

Disclosure of the Invention

An object of the present invention is to provide a pharmaceutical agent having a therapeutic effect on cancer. Another object of the present invention is to provide a liposome preparation containing an antibody capable of inducing the apoptosis of cells.

The present inventors have conducted diligent studies to attain the objects and consequently completed the present invention by finding that a liposome preparation containing an antibody capable of inducing the apoptosis of cells can exhibit a more significant apoptosis-inducing ability than that exhibited by the antibody alone. The liposome of the present invention functions similarly to a biologically cross-linked antibody, owing to the presence of the antibody at a high density on the liposome. As a result, the immunoliposome more effectively exerts an apoptosis-inducing ability by itself, independently of the presence of secondary antibodies *in vitro* or effector cells *in vivo*. This brings about an effective therapeutic effect even in patients that cannot obtain a sufficient therapeutic effect by the antibody alone. Moreover, the present immunoliposome containing a drug encapsulated therein produces an efficient drug targeting function to target cells as well as double pharmacological effects, i.e., the therapeutic effect of the drug and the therapeutic effect of the enhanced antibody activity. This can bring about a therapeutic effect that would be infeasible by a drug-encapsulated liposome alone or by an immunoliposome of an antibody having only a targeting ability.

Specifically, the present invention encompasses the following inventions:

(1) An immunoliposome which contains the following components (a) and (b) and specifically binds to a cell surface receptor involved in apoptosis induction:

(a) an antibody specifically binding to the cell surface receptor involved in apoptosis induction, a functional fragment of the antibody, or a polypeptide comprising heavy and light chain complementarity-determining regions of the antibody and specifically binding to the cell surface receptor; and
(b) an amphiphilic vesicle-forming lipid.

(2) The immunoliposome according to (1), **characterized in that** the immunoliposome exhibits an apoptosis-inducing activity against a cell expressing the cell surface receptor involved in apoptosis induction.

(3) The immunoliposome according to (1) or (2), **characterized in that** the immunoliposome exhibits, in vitro against a cell expressing the cell surface receptor involved in apoptosis induction, an apoptosis-inducing activity equivalent to or stronger than that exhibited in vitro, through cross-linking by a secondary antibody or an antibody-binding protein such as protein G or A, of full-length molecules of the antibody specifically binding to the cell surface receptor.

(4) The immunoliposome according to any one of (1) to (3), **characterized in that** the immunoliposome exhibits, against a cell expressing the cell surface receptor involved in apoptosis induction, a concentration for 50% cell viability that is 1/2.5 or smaller than that exhibited *in vitro,* through cross-linking by a secondary antibody or an antibody-binding protein such as protein G or A, of full-length molecules of the antibody specifically binding to the cell surface receptor.

(5) The immunoliposome according to any one of (1) to (4), **characterized in that** the immunoliposome exhibits, against a cell expressing the cell surface receptor involved in apoptosis induction, a concentration for 50% cell viability that is 1/10 or smaller than that exhibited in vitro, through cross-linking by a secondary antibody or an antibody-binding protein such as protein G or A, of full-length molecules of the antibody specifically binding to the cell surface receptor.

(6) The immunoliposome according to any one of (1) to (5), **characterized in that** the immunoliposome contains an antibody specifically binding to the cell surface receptor involved in apoptosis induction, wherein the antibody is a full-length antibody molecule.

(7) The immunoliposome according to (6), **characterized in that** the immunoliposome contains the full-length antibody molecule at an antibody density of 0.000014 mol% to 0.23 mol% with respect to the number of moles of total lipids.

(8) The immunoliposome according to (7), **characterized in that** the immunoliposome contains the full-length antibody molecule at an antibody density of 0.002 mol% to 0.14 mol% with respect to the number of moles of total lipids.

(9) The immunoliposome according to any one of (1) to (5), **characterized in that** the functional fragment of the antibody specifically binding to the cell surface receptor involved in apoptosis induction is $F(ab')_2$.

(10) The immunoliposome according to (9), **characterized in that** the immunoliposome contains the $F(ab')_2$ at an antibody density of 0.000014 mol% to 0.23 mol% with respect to the number of moles of total lipids.

(11) The immunoliposome according to (10), **characterized in that** the immunoliposome contains the $F(ab')_2$ at an antibody density of 0.006 mol% to 0.11 mol% with respect to the number of moles of total lipids.

(12) The immunoliposome according to any one of (1) to (5), **characterized in that** the functional fragment of the antibody specifically binding to the cell surface receptor involved in apoptosis induction is Fab'.

(13) The immunoliposome according to (12), **characterized in that** the immunoliposome contains the Fab' at an antibody density of 0.000014 mol% to 0.94 mol% with respect to the number of moles of total lipids.

(14) The immunoliposome according to (13), **characterized in that** the immunoliposome contains the Fab' at an antibody density of 0.005 mol% to 0.56 mol% with respect to the number of moles of total lipids.

(15) The immunoliposome according to any one of (1) to (14), **characterized in that** the antibody specifically binding to the cell surface receptor involved in apoptosis induction is a chimeric antibody.

(16) The immunoliposome according to any one of (1) to (14), **characterized in that** the antibody specifically binding to the cell surface receptor involved in apoptosis induction is a humanized antibody.

(17) The immunoliposome according to any one of (1) to (14), **characterized in that** the antibody specifically binding to the cell surface receptor involved in apoptosis induction is a human antibody.

(18) The immunoliposome according to any one of (1) to (5), **characterized in that** the polypeptide specifically binding to the cell surface receptor involved in apoptosis induction is a single-chain variable fragment antibody.

(19) The immunoliposome according to (18), **characterized in that** the immunoliposome contains the single-chain variable fragment antibody at an antibody density of 0.000014 mol% to 1.8 mol% with respect to the number of moles of total lipids.

(20) The immunoliposome according to (19), **characterized in that** the immunoliposome contains the single-chain variable fragment antibody at an antibody density of 0.005 mol% to 0.56 mol% with respect to the number of moles of total lipids.

(21) The immunoliposome according to any one of (1) to (20), **characterized in that** the cell surface receptor involved in apoptosis induction is a death domain-containing receptor.

(22) The immunoliposome according to (21), wherein the death domain-containing receptor is selected from the group consisting of Fas, DR4, DR5, and a TNF receptor.

(23) The immunoliposome according to (22), wherein the death domain-containing receptor is DR5.

(24) The immunoliposome according to (22), wherein the death domain-containing receptor is DR4.

(25) The immunoliposome according to (22), wherein the death domain-containing receptor is Fas.

(26) The immunoliposome according to (23), **characterized in that** the antibody molecule or the functional fragment of the antibody comprises a heavy chain variable region sequence consisting of amino acid residues 1 to 118 of the amino acid sequence of SEQ ID NO: 1 described in the sequence listing and a light chain variable region sequence consisting of amino acid residues 1 to 107 of the amino acid sequence of SEQ ID NO: 2 described in the sequence listing.

(27) The immunoliposome according to (24), **characterized in that** the antibody molecule or the functional fragment of the antibody comprises heavy and light chain variable regions of an antibody selected from an antibody produced by hybridoma m2E12, an antibody binding to the same epitope as that for the antibody, and a humanized antibody of the antibody.

(28) The immunoliposome according to (25), **characterized in that** the antibody molecule or the functional fragment of the antibody comprises a heavy chain variable region sequence consisting of amino acid residues 1 to 139 of the amino acid sequence of SEQ ID NO: 3 described in the sequence listing and a light chain variable region sequence consisting of amino acid residues 1 to 131 of the amino acid sequence of SEQ ID NO: 4 described in the sequence listing.

(29) An immunoliposome which contains the following components (a) and (b) and specifically binds to a cell surface receptor involved in apoptosis induction:

(a) an endogenous ligand specifically binding to the cell surface receptor and having an activity of inducing the apoptosis of a cell via the receptor, or a functional fragment of the endogenous ligand; and
(b) an amphiphilic vesicle-forming lipid.

(30) The immunoliposome according to (29), **characterized in that** the immunoliposome exhibits an apoptosis-inducing activity stronger than that of the endogenous ligand against a cell expressing the cell surface receptor.

(31) The immunoliposome according to (29) or (30), **characterized in that** the cell surface receptor is a death domain-containing receptor.

(32) The immunoliposome according to (31), wherein the death domain-containing receptor is selected from the group consisting of Fas, DR4, DR5, and a TNF receptor.

(33) The immunoliposome according to (32), wherein the endogenous ligand for the death domain-containing receptor is TRAIL or a Fas ligand.

(34) The immunoliposome according to any one of (1) to (33, **characterized in that** the immunoliposome contains a sterol and a phospholipid as constituent lipids.

(35) The immunoliposome according to (34), **characterized in that** the sterol as one of the constituent lipids of the immunoliposome is cholesterol.

(36) The immunoliposome according to (35), **characterized in that** the immunoliposome contains the cholesterol as one of the constituent lipids of the immunoliposome at a content of 20 mol% to 50 mol% with respect to the number of moles of total lipids.

(37) The immunoliposome according to any one of (34) to (36), **characterized in that** the phospholipid as one of the constituent lipids of the immunoliposome is phosphatidylcholine.

(38) The immunoliposome according to (37), **characterized in that** the phosphatidylcholine as the constituent lipid of the immunoliposome contains an acyl group having 14 to 22 carbon atoms.

(39) The immunoliposome according to (38), **characterized in that** the phosphatidylcholine as the constituent lipid of the immunoliposome contains an acyl group having 14 to 18 carbon atoms.

(40) The immunoliposome according to any one of (34) to (39), wherein the immunoliposome further contains a hydrophilic polymer or a lipid derivative of the hydrophilic polymer as a constituent lipid.

(41) The immunoliposome according to (40), **characterized in that** the hydrophilic polymer in the lipid derivative of the hydrophilic polymer has an average molecular weight of 500 to 20000.

(42) The immunoliposome according to (41), **characterized in that** the hydrophilic polymer in the lipid derivative of the hydrophilic polymer has an average molecular weight of 2000 to 5000.

(43) The immunoliposome according to any one of (40) to (42), **characterized in that** the immunoliposome contains the lipid derivative of the hydrophilic polymer at a content of 0.1 mol% to 10 mol% with respect to the number of moles of total lipids.

(44) The immunoliposome according to (43), **characterized in that** the immunoliposome contains the lipid derivative of the hydrophilic polymer at a content of 1 mol% to 6 mol% with respect to the number of moles of total lipids.

(45) The immunoliposome according to any one of (40) to (44), **characterized in that** the lipid derivative of the hydrophilic polymer as one of the constituent lipids of the immunoliposome is a polyethylene glycol-modified lipid.

(46) The immunoliposome according to any one of (34) to (44), **characterized in that** the liposome is conjugated with the antibody via a thioether linkage using a lipid or a hydrophilic polymer having a terminal maleimide group.

(47) The immunoliposome according to any one of (1) to (46), **characterized in that** the immunoliposome has an average particle size of 30 to 1000 nm.

(48) The immunoliposome according to (47), **characterized in that** the immunoliposome has an average particle size of 30 to 200 nm.

(49) The immunoliposome according to any one of (1) to (48), **characterized in that** a drug having an antitumor activity is encapsulated in the liposome.

(50) The immunoliposome according to any one of (1) to (48), **characterized in that** a drug having a therapeutic effect on autoimmune disease or inflammatory disease is encapsulated in the liposome.

(51) A pharmaceutical composition comprising an immunoliposome according to any one of (1) to (50) as an active ingredient.

(52) An antitumor agent comprising an immunoliposome according to any one of (1) to (49) as an active ingredient.

(53) A therapeutic agent for autoimmune disease or inflammatory disease comprising an immunoliposome according to any one of (1) to (48) and (50) as an active ingredient.

Brief Description of the Drawings

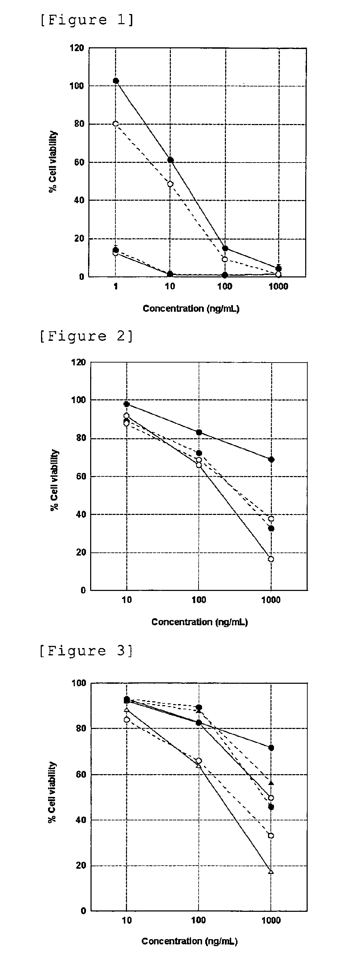

Figure 1 is a diagram showing the apoptosis-inducing activities of immunoliposomes prepared in Examples 1, 2, and 3 against Jurkat cells. In the diagram, the white circle with a dotted line, the black circle with a dotted line, and the white circle with a solid line represent the activities of the liposomes obtained in Examples 1, 2, and 3, respectively. The black circle with a solid line represents the activity of secondarily cross-linked hTRA-8;

Figure 2 is a diagram showing the apoptosis-inducing activities of immunoliposomes prepared in Examples 19, 20, and 21 against A375 cells. In the diagram, the white circle with a solid line, the white circle with a dotted line, and the black circle with a dotted line represent the activities of the liposomes obtained in Examples 19, 20, and 21, respectively. The black circle with a solid line represents the activity of secondarily cross-linked hTRA-8;

Figure 3 is a diagram showing the apoptosis-inducing activities of immunoliposomes prepared in Examples 8, 10, 12, 14, and 16 against A375 cells. In the diagram, the white triangle with a solid line, the black triangle with a dotted line, the white circle with a solid line, the black circle with a dotted line, and the white circle with a dotted line represent the activities of the liposomes obtained in Examples 8, 10, 12, 14, and 16, respectively. The black circle with a solid line represents the activity of secondarily cross-linked hTRA-8;

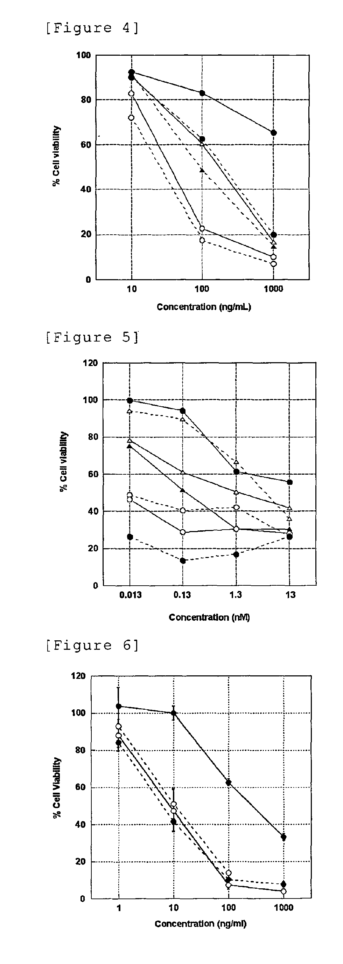

Figure 4 is a diagram showing the apoptosis-inducing activities of immunoliposomes prepared in Examples 7, 9, 11, 13, and 15 against A2058 cells. In the diagram, the white triangle with a solid line, the black triangle with a dotted line, the white circle with a solid line, the black circle with a dotted line, and the white circle with a dotted line represent the activities of the liposomes obtained in Examples 7, 9, 11, 13, and 15, respectively. The black circle with a solid line represents the activity of secondarily cross-linked hTRA-8;

Figure 5 is a diagram showing the apoptosis-inducing activities of immunoliposomes prepared in Examples 26, 27, 28, 29, 30, and 31 against Jurkat cells. In the diagram, the white triangle with a dotted line, the white triangle with a solid line, the black triangle with a solid line, the white circle with a dotted line, the white circle with a solid line, and the black circle with a dotted line represent the activities of the liposomes obtained in Examples 26, 27, 28, 29, 30, and 31, respectively. The black circle with a solid line represents the activity of secondarily cross-linked hHFE7A;

Figure 6 is a diagram showing the apoptosis-inducing activities of immunoliposomes prepared in Examples 4, 17, and 18 against Jurkat cells. In the diagram, the white circle with a solid line, the black circle with a dotted line, and the white circle with a dotted line represent the activities of the liposomes obtained in Examples 4, 17, and 18, respectively. The black circle with a solid line represents the activity of secondarily cross-linked hTRA-8;

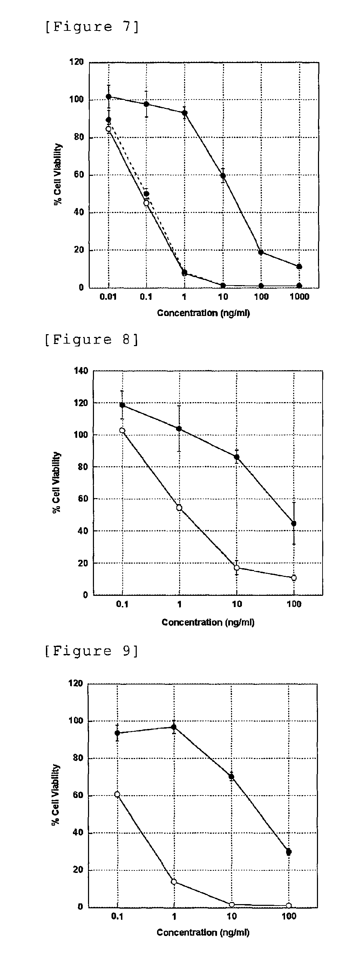

Figure 7 is a diagram showing the apoptosis-inducing activities of immunoliposomes prepared in Examples 22 and 23 against Jurkat cells. In the diagram, the white circle with a solid line and the black circle with a dotted line represent the activities of the liposomes obtained in Examples 22 and 23, respectively. The black circle with a solid line represents the activity of secondarily cross-linked hTRA-8;

Figure 8 is a diagram showing the apoptosis-inducing activity of an immunoliposome prepared in Example 24 against Jurkat cells. In the diagram, the white circle with a solid line represents the activity of the liposome obtained in Example 24. The black circle with a solid line represents the activity of secondarily cross-linked hTRA-8;

Figure 9 is a diagram showing the apoptosis-inducing activity of an immunoliposome prepared in Example 25 against Jurkat cells. In the diagram, the white circle with a solid line represents the activity of the liposome obtained in Example 25. The black circle with a solid line represents the activity of secondarily cross-linked hTRA-8;

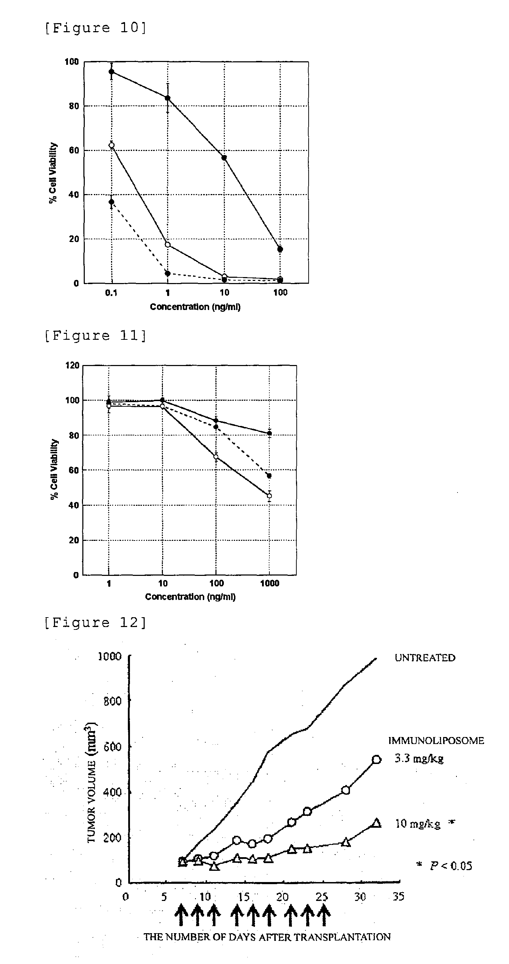

Figure 10 is a diagram showing the apoptosis-inducing activities of immunoliposomes prepared in Examples 5 and 6 against Jurkat cells. In the diagram, the white circle with a solid line and the black circle with a dotted line represent the activities of the liposomes obtained in Examples 5 and 6, respectively. The black circle with a solid line represents the activity of secondarily cross-linked hTRA-8;

Figure 11 is a diagram showing the apoptosis-inducing activities of immunoliposomes prepared in Examples 3 and 20 against synovial cells derived from particular rheumatism patients. In the diagram, the white circle with a solid line and the black circle with a dotted line represent the activities of the liposomes obtained in Examples 3 and 20, respectively. The black circle with a solid line represents the activity of secondarily cross-linked hTRA-8;

Figure 12 is a diagram showing the antitumor activity of an immunoliposome prepared in Example 5 against human colon cancer strain COLO 205-transplanted nude mice. In the diagram, the solid line without a symbol represents the tumor volume of antibody-unadministered mice. The white circle with a solid line and the white triangle with a solid line represent the tumor volumes of the mice that have received the administration of 3.3 mg/kg and 10 mg/kg antibodies, respectively;

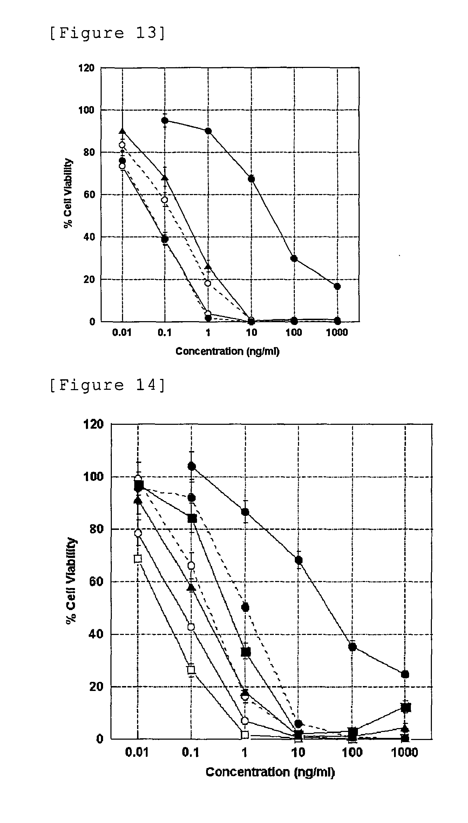

Figure 13 is a diagram showing the apoptosis-inducing activities of immunoliposomes prepared in Examples 32, 33, 34, and 35 against Jurkat cells. In the diagram, the white circle with a solid line, the white circle with a dotted line, the black circle with a dotted line, and the black triangle with a solid line represent the activities of the liposomes obtained in Examples 32, 33, 34, and 35, respectively. The black circle with a solid line represents the activity of secondarily cross-linked hTRA-8;

Figure 14 is a diagram showing the apoptosis-inducing activities of immunoliposomes prepared in Examples 44, 45, 46, 47, 48, and 49 against Jurkat cells. In the diagram, the white circle with a solid line, the white circle with a dotted line, the black square with a solid line, the white square with a solid line, the black triangle with a solid line, and the black circle with a dotted line represent the activities of the liposomes obtained in Examples 44, 45, 46, 47, 48, and 49, respectively. The black circle with a solid line represents the activity of secondarily cross-linked hTRA-8;

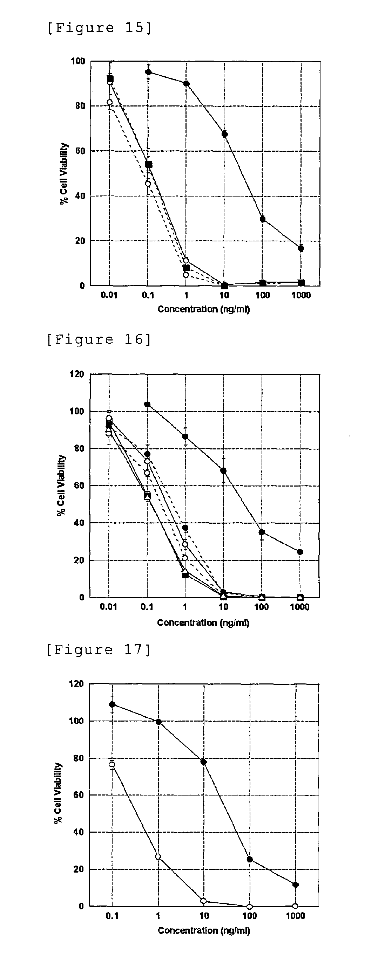

Figure 15 is a diagram showing the apoptosis-inducing activities of immunoliposomes prepared in Examples 50, 51, and 52 against Jurkat cells. In the diagram, the white circle with a solid line, the white circle with a dotted line, and the black square with a dotted line represent the activities of the liposomes obtained in Examples 50, 51, and 52, respectively. The black circle with a solid line represents the activity of secondarily cross-linked hTRA-8;

Figure 16 is a diagram showing the apoptosis-inducing activities of immunoliposomes prepared in Examples 53, 54, 55, 56, and 57 against Jurkat cells. In the diagram, the white circle with a dotted line, the black square with a solid line, the white circle with a solid line, the black circle with a dotted line, and the white triangle with a solid line represent the activities of the liposomes obtained in Examples 53, 54, 55, 56, and 57, respectively. The black circle with a solid line represents the activity of secondarily cross-linked hHFE7A;

Figure 17 is a diagram showing the apoptosis-inducing activity of an immunoliposome prepared in Example 58 against Jurkat cells. In the diagram, the white circle with a solid line represents the activity of the liposome obtained in Example 58. The black circle with a solid line represents the activity of secondarily cross-linked hTRA-8;

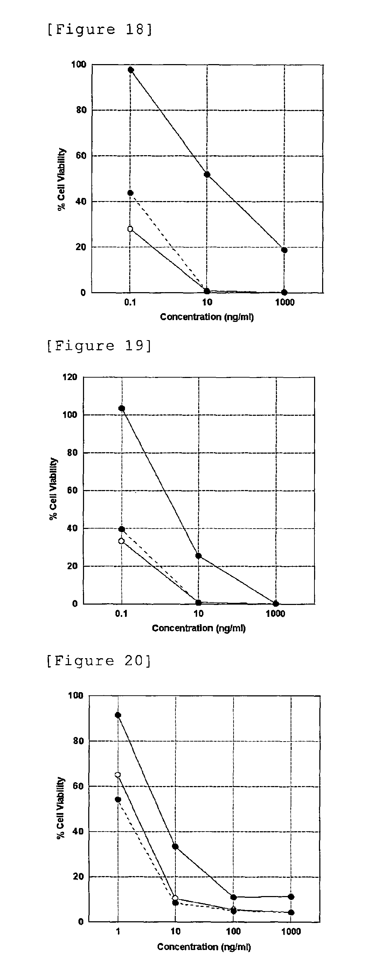

Figure 18 is a diagram showing the apoptosis-inducing activities of immunoliposomes prepared in Examples 36 and 37 against Jurkat cells. In the diagram, the white circle with a solid line and the black circle with a dotted line represent the activities of the liposomes obtained in Examples 36 and 37, respectively. The black circle with a solid line represents the activity of secondarily cross-linked MAB631;

Figure 19 is a diagram showing the apoptosis-inducing activities of immunoliposomes prepared in Examples 38 and 39 against Jurkat cells. In the diagram, the white circle with a solid line and the black circle with a dotted line represent the activities of the liposomes obtained in Examples 38 and 39, respectively. The black circle with a solid line represents the activity of secondarily cross-linked MAB6311;

Figure 20 is a diagram showing the apoptosis-inducing activities of immunoliposomes prepared in Examples 59 and 60 against Jurkat cells. In the diagram, the white circle with a solid line and the black circle with a dotted line represent the activities of the liposomes obtained in Examples 59 and 60, respectively. The black circle with a solid line represents the activity of secondarily cross-linked rsTRAIL(P);

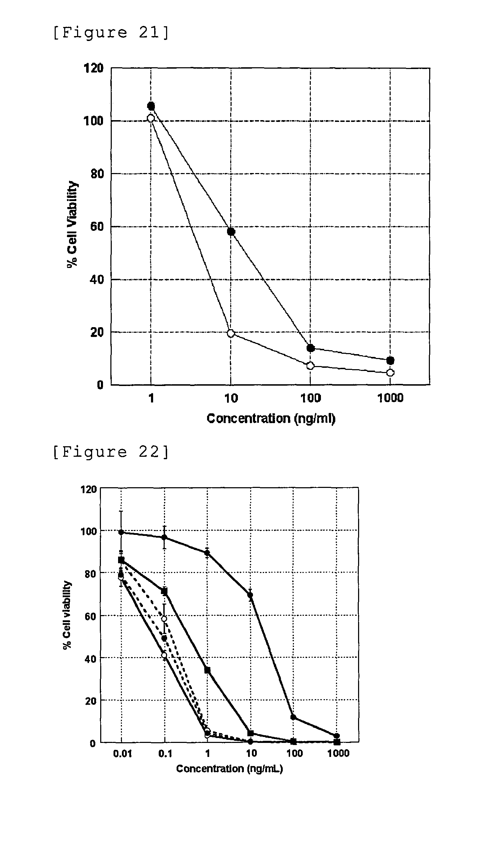

Figure 21 is a diagram showing the apoptosis-inducing activity of an immunoliposome prepared in Example 61 against Jurkat cells. In the diagram, the white circle with a solid line represents the activity of the liposome obtained in Example 61. The black circle with a solid line represents the activity of secondarily cross-linked rsTRAIL(B);

Figure 22 is a diagram showing the apoptosis-inducing activities of immunoliposomes prepared in Examples 41, 42, 43, and 69 against Jurkat cells. In the diagram, the white circle with a solid line, the white circle with a dotted line, the black circle with a dotted line, and the black square with a solid line represent the activities of the liposomes obtained in Examples 41, 42, 43, and 69, respectively. The black circle with a solid line represents the activity of secondarily cross-linked hTRA-8;

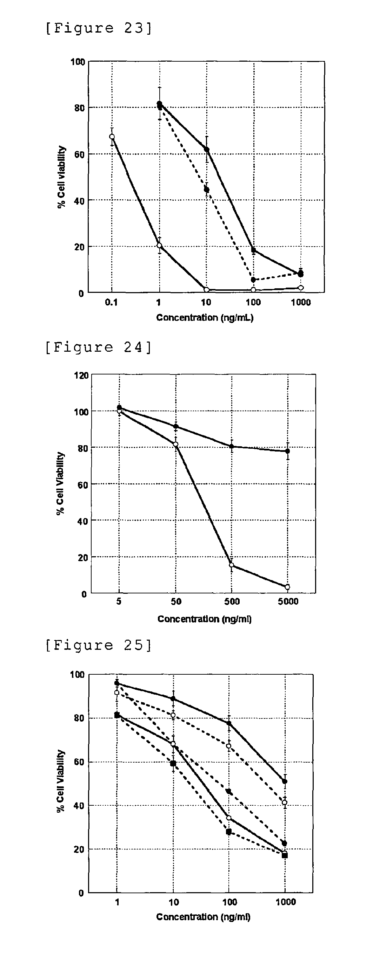

Figure 23 is a diagram showing the apoptosis-inducing activities of immunoliposomes prepared in Examples 67 and 68 against Jurkat cells. In the diagram, the black circle with a dotted line and the white circle with a solid line represent the activities of the liposomes obtained in Examples 67 and 68, respectively. The black circle with a solid line represents the activity of secondarily cross-linked hTRA-8;

Figure 24 is a diagram showing the apoptosis-inducing activity of an immunoliposome prepared in Example 40 against WR19L12a cells. In the diagram, the white circle with a solid line represents the activity of the liposome obtained in Example 40. The black circle with a solid line represents the activity of secondarily cross-linked DX2;

Figure 25 is a diagram showing the apoptosis-inducing activities of immunoliposomes prepared in Examples 62, 64, 65, and 66 against Alexander cells. In the diagram, the white circle with a dotted line, the black circle with a dotted line, the white circle with a solid line, and the black square with a dotted line represent the activities of the liposomes obtained in Examples 62, 64, 65, and 66, respectively. The black circle with a solid line represents the activity of secondarily cross-linked m2E12; and

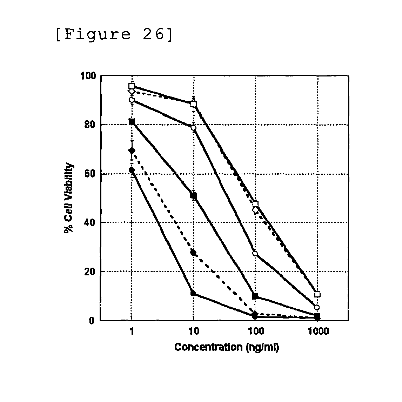

Figure 26 is a diagram showing the apoptosis-inducing activities of immunoliposomes prepared in Examples 70, 71, and 72 against Alexander cells. In the diagram, the black circle with a solid line, the black rhombus with a dotted line, and the black square with a solid line represent the activities of the liposomes obtained in Examples 70, 71, and 72, respectively. The white circle with a solid line, the white rhombus with a dotted line, and the white square with a solid line represent the activities of m2E12 and hTRA-8 mixed at a ratio of 22:78, m2E12 and hTRA-8 mixed at a ratio of 56: 44, and m2E12 and hTRA-8 mixed at a ratio of 80:20, respectively.

Best Mode for Carrying Out the Invention

In the present specification, the terms "cancer" and "tumor" are used in the same sense.

In the present specification, the term "gene" is meant to encompass not only DNA but also mRNA thereof, cDNA, and cRNA thereof.

In the present specification, the term "polynucleotide" is used in the same sense as a nucleic acid and also encompasses DNA, RNA, probes, oligonucleotides, and primers.

In the present specification, the terms "polypeptide" and "protein" are used without being differentiated therebetween.

In the present specification, the term "RNA fraction" refers to a fraction containing RNA.

In the present specification, the term "cell" also encompasses cells in individual animals and cultured cells.

In the present specification, the term "cell malignant transformation" means that cells exhibit abnormal growth, for example, lose sensitivity to contact inhibition or exhibit anchorage-independent growth. Cells exhibiting such abnormal growth are referred to as "cancer cells".

In the present specification, the term "cytotoxicity" refers to any pathologic change in cells and refers not only to direct injury but also to any structural or functional damage to cells such as DNA cleavage, dimerization of bases, chromosomal breakage, damage of mitotic apparatus, and decrease in various enzyme activities.

In the present specification, the term "cytotoxic activity" refers to an activity that causes the cytotoxicity.

In the present specification, the term "contained" used in, for example, the phrase "antibody (or polypeptide) contained in an immunoliposome" refers to a state in which the functional group of a constituent lipid of the liposome forms a covalent bond or a non-covalent bond based on physical/biological affinity, with the functional group of the polypeptide.

In the present specification, the term "death domain-containing receptor" refers to a receptor molecule that has, in the intracellular domain, an apoptotic signal transduction region called a "death domain" homologous to Drosophila suicide gene reaper (examples of the receptor molecule include, but are not limited to, Fas, TNFRI, DR3, DR4, DR5, and DR6).

In the present specification, the term "functional fragment of an antibody" means a partial antibody fragment having binding affinity for antigens and encompasses Fab, F(ab')$_2$, scFv, and the like. Moreover, the functional fragment of the antibody also encompasses Fab', which is a monovalent fragment of an antibody variable region obtained by treating F(ab')$_2$ under reductive conditions. However, the functional fragment of the antibody is not limited to these molecules as long as it is capable of binding to antigens. Moreover, these functional fragments encompass not only fragments obtained by treating a full-length molecule of the antibody protein with appropriate enzymes but also proteins produced by appropriate host cells using genetically modified antibody genes.

In the present invention, the term "Fab '" refers to a monovalent fragment of an antibody variable region obtained by treating F(ab')$_2$ under reductive conditions, as described above. This Fab' can be conjugated with a liposome by use of a thiol group in the Fab' produced under the reductive conditions. However, Fab' produced using genetically modified antibody genes or Fab comprising a cysteine residue-containing polypeptide genetically engineered at the carboxy terminus can also be conjugated with a liposome by use of their respective thiol groups and is also encompassed in the Fab' according to the present invention.

In the present specification, the term "single-chain variable fragment antibody" is used in the same sense as single-chain Fv (scFv).

In the present specification, the term "secondary antibody" refers to an antibody that specifically binds to an antibody molecule to form a cross-link between the antibody molecules.

In the present specification, the term "amphiphilic vesicle-forming lipid" encompasses a lipid that has hydrophobic and hydrophilic moieties and can further form a bilayer vesicle in itself in water, and all amphiphilic lipids that are incorporated together with other lipids into a lipid bilayer, in which the hydrophobic regions thereof are contacted with the internal hydrophobic regions of the bilayer membrane while the hydrophilic regions thereof are arranged to face the outer polar surfaces of the membrane.

In the present specification, the term "liposome" refers to a lipid structure formed by an amphiphilic vesicle-forming lipid. The liposome is typically a closed vesicle composed of a unilamellar or multilamellar lipid bilayer having an internal aqueous phase. In the present invention, the liposome refers to a lipid complex particle in a broader sense.

In the present specification, the term "immunoliposome" refers to a complex formed by a liposome and a protein.

In the present specification, the "antibody density" of the immunoliposome refers to the ratio (indicated in mol%) of the number of moles of the antibody contained in the immunoliposome to the number of moles of total constituent lipids of the immunoliposome.

In the present specification, the term "average particle size" refers to a mean of particle size distributions measured with respect to volumes or numbers. The particle size of particles is measured by, for example, electromagnetic wave scattering methods (e.g., laser diffractometry and dynamic light scattering) and light transmission methods (e.g., centrifugal sedimentation).

1. Regarding apoptosis-related gene

In the present invention, an antibody contained in the immunoliposome of interest is required to bind to a particular antigen and exhibit a cytotoxic activity via the antigen. Moreover, the antigen must be selected from those present in a manner specific for tumor cells to prevent normal cells from being killed. One example of such antigen groups can include tumor necrosis factor (hereinafter, referred to as "TNF" in the present specification)-related apoptosis-inducing ligand (hereinafter, referred to as "TRAIL" in the present specification) receptor groups. TRAIL is a member of the TNF family of proteins and encompasses Fas ligands and TNF-$\alpha$ (Wiley SR, et al., Immunity 1995 Dec; 3 (6): 673-82). These proteins are strong apoptosis-inducing factors.

Receptors for these TNF family proteins are characterized by cysteine-rich repeats in the extracellular domain. Among them, Fas, a receptor for Fas ligands, and a TNF receptor I (hereinafter, referred to as "TNFRI" in the present specification), a receptor for TNF$\alpha$, are collectively called death domain-containing receptors that have, in the intracellular domain, a region essential for apoptotic signal transduction, called a "death domain" homologous to Drosophila suicide gene reaper (Golstein, P., et al., (1995) Cell. 81, 185-186; and White, K, et al., (1994) Science 264, 677-683).

Five receptors for TRAIL have been identified. Of them, two (DR4 (TRAIL-R1) and DR5 (TRAIL-R2)) are capable of transducing apoptotic signals, and the other three (DcR1 (TRAIL-R3), DcR2 (TRAIL-R4), and osteoprotegerin (OPG)) do not transduce apoptotic signals. As in Fas and TNFRI, both DR4 and DR5 comprise a death domain in the intracellular segment and transduce apoptotic signals via a pathway containing Fas-associated death domain proteins (hereinafter, referred to as "FADD" in the present specification) and caspase 8 (Delgi-Esposti MA, et al., Immunity 1997 Dec; 7 (6): 813-20; and Chaudhary PM, et al,. Immunity 1997 Dec; 7 (6): 821-30).

For the Fas, TNFRI, DR4, or DR5 described above, an antibody that functions as an agonist binding to this molecule is known to exhibit an apoptosis-inducing ability against cells bearing the molecule on the cell surface (Journal of Cellular Physiology, 209: 1021-1028 (2006); Leukemia, Apr; 21 (4): 805-812 (2007); Blood, 99: 1666-1675 (2002); and Cellular Immunology, Jan; 153 (1): 184-193 (1994)). The pharmacological effect of the agonistic antibody is enhanced by cross-linking with secondary antibodies or effector cells (Journal of Immunology, 149: 3166-3173 (1992); and European Journal of Immunology, Oct; 23 (10): 2676-2681 (1993)). The immunoliposome comprises many antibodies bound onto the liposome membrane and can thus be interpreted as a structure that mimics the cross-linked state of antibodies. Thus, the pharmacological effect of the agonistic antibody can probably be enhanced more greatly by the immunoliposome than by the conventional cross-linking via secondary antibodies or effector cells. Moreover, the immunoliposome can artificially achieve a highly cross-linked state. It is therefore expected that the antibody alone does not necessarily have an agonistic activity. From these reasons, the antibody binding to the death domain-containing receptor can be selected as the antibody that can be contained in the immunoliposome of the present invention.

2. Antibody binding to DR5

(1) DR5 gene

The nucleotide sequence of the human DR5 (death receptor 5) gene and the amino acid sequence thereof are recorded as GI:22547118 (Accession No: NM_147187) in GenBank. In this context, the nucleotide sequence of the DR5 gene also encompasses nucleotide sequences encoding proteins which consist of an amino acid sequence derived from the DR5 amino acid sequence by the substitution, deletion, or addition of one or more amino acids and have an equivalent biological activity to that of DR5. Moreover, DR5 also encompasses proteins which consist of an amino acid sequence derived from the DR5 amino acid sequence by the substitution, deletion, or addition of one or more amino acids and have an equivalent biological activity to that of DR5.

(2) Antibody against DR5

The antibody against DR5 according to the present invention can be obtained according to standard methods by immunizing animals with DR5 or with an arbitrary polypeptide selected from the DR5 amino acid sequence and collecting and purifying antibodies produced *in vivo.*
Moreover, antibody-producing cells that produce the antibody against DR5 are fused with myeloma cells according to a method known in the art (e.g., Kohler and Milstein, Nature (1975) 256, p. 495-497; and Kennet, R. ed., Monoclonal Antibodies, p. 365-367, Plenum Press, N.Y. (1980)) to thereby establish hybridomas, from which monoclonal antibodies can also be obtained.
In this context, the DR5 used as an antigen can be obtained by causing the DR5 gene to be expressed in host cells by genetic engineering.
Specifically, vectors capable of expressing the DR5 gene are prepared and introduced into host cells, which are then caused to express the gene, and the expressed DR5 may be purified.
Moreover, artificial genes encoding a DR5 extracellular region fused with an antibody constant region are constructed, and proteins prepared therefrom in an appropriate gene expression system can also be used as immunogens.
Hereinafter, the method for obtaining the antibody against DR5 will be described specifically.

(2)-1. Preparation of antigen

Examples of the antigen for preparing the anti-DR5 antibody can include DR5, polypeptides consisting of a consecutive partial amino acid sequence of at least 6 amino acids thereof, and derivatives obtained by adding an arbitrary amino acid sequence or a carrier to these sequences.
DR5 can be purified directly, for use, from human tumor tissues or tumor cells. Moreover, DR5 can be synthesized *in vitro* or obtained by causing host cells by genetic engineering to produce the protein.
In the genetic engineering, specifically, the DR5 gene is incorporated into vectors capable of expressing DR5, and DR5 can then be synthesized in a solution containing enzymes, substrates, and energy substances necessary for transcription and translation. Alternatively, host cells of other prokaryotes or eukaryotes can be transformed therewith and caused to express DR5 to obtain the protein.
DR5 cDNA can be obtained, for example, by a so-called polymerase chain reaction (hereinafter, referred to as "PCR") method in which PCR (see Saiki, R.K., et al. Science (1988) 239, p. 487-489) is performed using DR5-expressing cDNA libraries as templates and primers specifically amplifying DR5 cDNA.
Examples of *in-vitro* polypeptide synthesis methods include, but are not limited to, Rapid Translation System (RTS) manufactured by Roche Diagnostics GmbH.

Examples of the host prokaryotic cells include *Escherichia coli* and *Bacillus subtilis.* To transform these host cells with the gene of interest, the host cells are transformed with plasmid vectors comprising a replicon, i.e., a replication origin, and a regulatory sequence derived from a species compatible with the hosts. Moreover, it is preferred that the vectors should have a sequence that can impart phenotypic character (phenotype) selectivity on the transformed cells.

The host eukaryotic cells encompass cells of vertebrates, insects, yeast, and the like. For example, monkey COS cells (Gluzman, Y. Cell (1981) 23, p. 175-182, ATCC CRL-1650), mouse fibroblasts NIH3T3 (ATCC No. CRL-1658), and dihydrofolate reductase-deficient strains (Urlaub, G. and Chasin, L.A., Proc. Natl. Acad. Sci. USA (1980) 77, p. 4126-4220) of Chinese hamster ovarian cells (CHO cells, ATCC CCL-61) are often used as the vertebrate cells, though the vertebrate cells are not limited thereto.

The transformants thus obtained can be cultured according to standard methods and are caused by the culture to intracellularly or extracellularly produce the polypeptide of interest.

A medium used for the culture can be selected appropriately according to the adopted host cells from among various media routinely used. For *Escherichia coli,* for example, an LB medium optionally supplemented with an antibiotic (e.g., ampicillin) or IPTG can be used.

The recombinant protein intracellularly or extracellularly produced by the transformants in the culture can be separated and purified by various separation procedures known in the art by use of the physical properties, chemical properties, or the like of the protein.

The procedures can be exemplified specifically by treatment with usual protein precipitants, ultrafiltration, various liquid chromatography techniques such as molecular sieve chromatography (gel filtration), adsorption chromatography, ion-exchange chromatography, affinity chromatography, and high-performance liquid chromatography (HPLC), dialysis, and combinations thereof.

Moreover, the recombinant protein to be expressed can be linked to 6 histidine residues to thereby efficiently purify the resulting protein on a nickel affinity column.

By combining these methods, the polypeptide of interest can be produced easily in large amounts with high yields and high purity.

(2)-2. Production of anti-DR5 monoclonal antibody

Examples of the antibody specifically binding to DR5 can include monoclonal antibodies specifically binding to DR5. A method for obtaining the antibodies is as described below.

For monoclonal antibody production, the following process is generally required:

(a) the step of purifying biopolymers used as antigens,
(b) the step of immunizing animals with the antigens through injection, then collecting blood from the animals, assaying the antibody titer thereof to determine the timing of splenectomy, and then preparing antibody-producing cells,
(c) the step of preparing myeloma cells (hereinafter, referred to as "myelomas"),
(d) the step of performing cell fusion between the antibody-producing cells and the myelomas,
(e) the step of selecting a hybridoma group that produces the antibody of interest,
(f) the step of dividing into single cell clones (cloning),
(g) the step of culturing the hybridomas for producing monoclonal antibodies in large amounts or raising hybridoma-transplanted animals, according to circumstances,
(h) the step of studying the bioactivities and binding specificities of the monoclonal antibodies thus produced or assaying properties of labeling reagents, etc.

Hereinafter, the method for preparing monoclonal antibodies will be described in detail in line with these steps, though the method for preparing antibodies is not limited thereto. For example, antibody-producing cells other than the splenic cells and myelomas can also be used.

(a) Purification of antigens

DR5 or a portion thereof prepared by the method as described above can be used as the antigen.

Moreover, membrane fractions prepared from DR5-expressing recombinant cells, the DR5-expressing recombinant cells themselves, fusion proteins of DR5 and another protein, and partial peptides of the protein of the present invention chemically synthesized according to a method well known by those skilled in the art can also be used as antigens.

(b) Preparation of antibody-producing cells

The antigens obtained in the step (a) are mixed with complete or incomplete Freund's adjuvants or other auxiliaries such as potassium aluminum sulfate, and experimental animals are immunized with these immunogens. Animals used in hybridoma preparation methods known in the art can be used as the experimental animals without problems. Specifically, for example, mice, rats, goats, sheep, cows, and horses can be used. However, mice or rats are preferably used as the animals to be immunized, from the viewpoint of the easy availability of myeloma cells to be fused with the extracted antibody-producing cells, etc.

Moreover, the lineages of the mice and rats actually used are not particularly limited. For example, mouse lineages such as A, AKR, BALB/c, BDP, BA, CE, C3H, 57BL, C57BR, C57L, DBA, FL, HTH, HT1, LP, NZB, NZW, RF, R III, SJL, SWR, WB, and 129 and rat lineages such as Low, Lewis, Sprague, Dawley, ACI, BN, and Fischer can be used.

These mice and rats can be obtained from, for example, experimental animal growers/distributors such as CLEA Japan, Inc. and Charles River Laboratories Japan, Inc.

Of these lineages, the mouse BALB/c lineage and the rat Low lineage are particularly preferable as the animals to be immunized, in consideration of fusion compatibility to myeloma cells described later.

Moreover, mice having a reduced biological mechanism for autoantibody removal, i.e., autoimmune disease mice, are also preferably used in consideration of antigenic homology between humans and mice.

These mice or rats are preferably 5 to 12 weeks old, more preferably 6 to 8 weeks old, at the time of immunization.

For the immunization of the animals with DR5 or the recombinants thereof, methods known in the art described in detail in, for example, Weir, D.M., Handbook of Experimental Immunology Vol. I. II. III., Blackwell Scientific Publications, Oxford (1987), and Kabat, E.A. and Mayer, M.M., Experimental Immunochemistry, Charles C Thomas Publisher Springfield, Illinois (1964) can be used.

Of these immunization methods, a method preferable in the present invention is specifically illustrated as described below.

Specifically, the membrane protein fractions used as antigens or antigen-expressing cells are first administered intradermally or intraperitoneally to the animals.

However, the combined use of both the administration routes is preferable for enhancing immunization efficiency. Immunization efficiency can be enhanced particularly by performing intradermal administration in early immunizations and performing intraperitoneal administration in later immunizations or only in the last immunization.

The administration schedule of the antigens differs depending on the type of the animals to be immunized, the individual difference thereof, etc. The antigens are generally administered at 3 to 6 doses preferably at 2-to 6-week intervals, more preferably at 3 to 4 doses at 2- to 4-week intervals.

Moreover, the dose of the antigens differs depending on the type of the animals, the individual difference thereof, etc., and is generally of the order of 0.05 to 5 mg, preferably 0.1 to 0.5 mg.

A booster is performed 1 to 6 weeks later, preferably 2 to 4 weeks later, more preferably 2 to 3 weeks later, from such antigen administration.

In this context, the dose of the antigens in the booster differs depending on the type of animal, the size thereof, etc., and is generally of the order of 0.05 to 5 mg, preferably 0.1 to 0.5 mg, more preferably 0.1 to 0.2 mg, for example, for mice.

1 to 10 days later, preferably 2 to 5 days later, more preferably 2 to 3 days later, after the booster, splenic cells or lymphocytes containing antibody-producing cells are aseptically extracted from the animals thus immunized.

In this procedure, their antibody titers are measured, and animals having a sufficiently increased antibody titer can be used as sources of antibody-producing cells to thereby enhance the efficiency of the subsequent procedures.

Examples of methods for measuring the antibody titers used here can include, but are not limited to, RIA and ELISA.

The antibody titer measurement according to the present invention can be performed by procedures as described below, for example, according to ELISA.

First, the purified or partially purified antigens are adsorbed onto the surface of a solid phase such as 96-well plates for ELISA. Furthermore, antigen-unadsorbed solid phase surface is covered with proteins unrelated to the antigens, for example, bovine serum albumin (hereinafter, referred to as "BSA"). The surfaces are washed and then contacted with serially diluted samples (e.g., mouse serum) as primary antibodies such that the antibodies in the samples are bound to the antigens.

Furthermore, enzyme-labeled antibodies against the mouse antibodies are added thereto as secondary antibodies such that the secondary antibodies are bound to the mouse antibodies. After washing, substrates for the enzyme are added thereto, and, for example, the change in absorbance caused by color development based on substrate degradation is measured to thereby calculate antibody titers.

The antibody-producing cells can be separated from these splenic cells or lymphocytes according to methods known in the art (e.g., Kohler et al., Nature (1975) 256, p. 495; Kohler et al., Eur. J. Immunol. (1977) 6, p. 511; Milstein et al., Nature (1977), 266, p. 550; and Walsh, Nature, (1977) 266, p. 495).

For example, for the splenic cells, a general method can be adopted, which comprises cutting the cells into strips, filtering them through a stainless mesh, and then separating the antibody-producing cells therefrom by floating in Eagle's minimal essential medium (MEM).

(C) Preparation of myeloma cells (hereinafter, referred to as "myelomas")

Myeloma cells used in cell fusion are not particularly limited and can be selected appropriately, for use, from cell strains known in the art. However, HGPRT (hypoxanthine-guanine phosphoribosyl transferase)-deficient strains for which selection methods have been established are preferably used in consideration of convenient hybridoma selection from fused cells.

Specific examples thereof include: mouse-derived X63-Ag8 (X63), NS1-ANS/1 (NS1), P3X63-Ag8.U1 (P3U1), X63-Ag8.653 (X63.653), SP2/0-Ag14 (SP2/0), MPC11-45.6TG1.7 (45.6TG), FO, S149/5XXO, and BU.1; rat-derived 210.RSY3.Ag.1.2.3 (Y3); and human-derived U266AR (SKO-007), GM1500.GTG-A12 (GM1500), UC729-6, LICR-LOW-HMy2 (HMy2), and 8226AR/NIP4-1 (NP41).

These HGPRT-deficient strains can be obtained from, for example, American Type Culture Collection (ATCC).

These cell strains are subcultured in an appropriate medium, for example, an 8-azaguanine medium [RPMI-1640 medium supplemented with glutamine, 2-mercaptoethanol, gentamicin, and fetal calf serum (hereinafter, referred to as "FCS") and further supplemented with 8-azaguanine], an Iscove's modified Dulbecco's medium (hereinafter, referred to as "IMDM"), or a Dulbecco's Modified Eagle Medium (hereinafter, referred to as "DMEM") and subcultured in a normal medium [e.g., an ASF104 medium (manufactured by Ajinomoto Co., Inc.) containing 10% FCS] for 3 to 4 days before cell fusion. On the day of fusion, $2 \times 10^7$ or more cells are secured.

(d) Cell fusion

Fusion between the antibody-producing cells and the myeloma cells can be performed appropriately under conditions that do not excessively reduce the cell viability, according to methods known in the art (e.g., Weir, D.M., Handbook of Experimental Immunology Vol. I. II. III., Blackwell Scientific Publications, Oxford (1987); and Kabat, E.A. and Mayer, M.M., Experimental Immunochemistry, Charles C Thomas Publisher Springfield, Illinois (1964)).

For example, a chemical method which comprises mixing the antibody-producing cells and the myeloma cells in a high-concentration polymer (e.g., polyethylene glycol) solution and a physical method using electric stimulations can be used as such methods.

Of these methods, the chemical method is specifically exemplified as described below.

Specifically, when polyethylene glycol is used as a polymer in the high-concentration polymer solution, the antibody-producing cells and the myeloma cells are mixed in a solution of polyethylene glycol having a molecular weight of 1500 to 6000, preferably 2000 to 4000, at a temperature of 30 to 40°C, preferably 35 to 38°C, for 1 to 10 minutes, preferably 5 to 8 minutes.

(e) Selection of hybridoma group

A method for selecting the hybridomas obtained by the cell fusion is not particularly limited, and a HAT (hypoxanthine-aminopterin-thymidine) selection method (Kohler et al., Nature (1975) 256, p. 495; and Milstein et al., Nature (1977) 266, p. 550) is usually used.

This method is effective for obtaining hybridomas using HGPRT-deficient myeloma cells that cannot survive in aminobuterin.

Specifically, unfused cells and the hybridomas can be cultured in a HAT medium to thereby cause only aminobuterin-resistant hybridomas to selectively remain and grow.

(f) Dividing into single cell clones (cloning)

For example, methods known in the art, such as methylcellulose, soft agarose, and limiting dilution methods can be used as methods for cloning the hybridomas (e.g., Barbara, B.M. and Stanley, M.S.: Selected Methods in Cellular Immunology, W.H. Freeman and Company, San Francisco (1980)). Of these methods, the limiting dilution method is particularly preferable.

In this method, feeders such as rat fetus-derived fibroblast strains or normal mouse splenic, thymus, or ascites cells are inoculated onto a microplate.

On the other hand, the hybridomas are diluted to 0.2 to 0.5 individuals/0.2 ml in advance in a medium. This solution containing the diluted hybridomas floating therein is added at a concentration of 0.1 ml/well, and the hybridomas can be continuously cultured for approximately 2 weeks while approximately 1/3 of the medium is replaced with a new one at regular intervals (e.g., 3-day intervals), to thereby grow hybridoma clones.

For wells having an observable antibody titer, for example, cloning by the limiting dilution method is repeated 2 to 4 times, and clones whose antibody titer is stably observed are selected as anti-DR5 monoclonal antibody-producing hybridoma strains.

(g) Preparation of monoclonal antibodies by hybridoma culture

The hybridomas thus selected can be cultured to thereby efficiently obtain monoclonal antibodies. Prior to the culture, it is preferred that hybridomas producing the monoclonal antibody of interest should be screened.

For this screening, methods known *per se* in the art can be adopted.

The antibody titer measurement according to the present invention can be performed, for example, by ELISA described in paragraph (b).

The hybridomas obtained by the method as described above can be cryopreserved in liquid nitrogen or in a freezer at -80°C or lower.

The completely cloned hybridomas can be cultured in a HT medium, which is then changed to a normal medium.

Large-scale culture is performed by rotational culture using large culture bottles or spinner culture.

A supernatant obtained in this large-scale culture can be purified according to methods well known by those skilled in the art, such as gel filtration, to obtain monoclonal antibodies specifically binding to the protein of the present invention.

Moreover, the hybridomas can be intraperitoneally injected into mice of the same lineage thereas (e.g., the BALB/c) or Nu/Nu mice and grown to obtain ascites containing the monoclonal antibody of the present invention in large amounts.

For the intraperitoneal administration, mineral oil such as 2,6,10,14-tetramethyl pentadecane (pristane) is administered beforehand (3 to 7 days before the administration) to obtain ascites in larger amounts.

For example, an immunosuppressive agent is intraperitoneally injected, in advance, to the mice of the same lineage as the hybridomas, to inactivate the T cells. 20 days later, $10^6$ to $10^7$ hybridoma clone cells are allowed to float (0.5 ml) in a serum-free medium, and this solution is intraperitoneally administered to the mice. Ascites are usually collected from the mice when abdominal distention occurs by accumulated ascites.

By this method, monoclonal antibodies are obtained with a concentration approximately 100 times higher than that in the culture solution.

The monoclonal antibodies obtained by the method can be purified by methods described in, for example, Weir, D.M.: Handbook of Experimental Immunology, Vol. I, II, III, Blackwell Scientific Publications, Oxford (1978).

Specific examples thereof include ammonium sulfate precipitation, gel filtration, ion-exchange chromatography, and affinity chromatography.

For the purification, commercially available monoclonal antibody purification kits (e.g., MAbTrap GII Kit; manufactured by Pharmacia Inc.) and the like can also be used as convenient methods.

The monoclonal antibodies thus obtained have high antigen specificity for DR5.

(h) Assay of monoclonal antibodies

The isotype and subclass of the monoclonal antibodies thus obtained can be determined as described below.

First, examples of identification methods include the Ouchterlony method, ELISA, and RIA.

The Ouchterlony method is convenient but requires a concentration procedure for a low concentration of monoclonal antibodies.

On the other hand, when the ELISA or RIA is used, the culture supernatant is directly reacted with an antigen-adsorbed solid phase, and further, antibodies compatible with various immunoglobulin isotypes and subclasses can be used as secondary antibodies to thereby identify the isotype or subclass of the monoclonal antibodies.

Moreover, commercially available kits for identification (e.g., Mouse Typer Kit; manufactured by Bio-Rad Laboratories, Inc.) and the like can also be used as more convenient methods.

Furthermore, the proteins can be quantified according to a Folin-Lowry method and a calculation method using absorbance at 280 nm [1.4 (OD280)=1 mg/ml immunoglobulin].

(3) Other antibodies

The antibody of the present invention encompasses the monoclonal antibody against DR5 as well as genetic recombinant antibodies artificially modified for the purpose of, for example, reducing xenoantigenicity against humans, for example, chimeric, humanized, and human antibodies. These antibodies can be produced according to known methods.

Examples of the chimeric antibody include an antibody having variable and constant regions derived from species different from each other and specifically include a chimeric antibody comprising mouse-derived variable regions and human-derived constant regions joined together (Proc. Natl. Acad. Sci. U.S.A., 81, 6851-6855, (1984)).

Examples of the humanized antibody can include an antibody comprising a human-derived antibody with complementarity-determining regions (CDRs) replaced with those of another species (Nature (1986) 321, p. 522-525) and an antibody comprising a human antibody with CDR sequences and some framework amino acid residues replaced with those of another species by CDR grafting (the pamphlet of WO90/07861).

Further examples of the antibody of the present invention can include an anti-human antibody. The anti-DR5 human antibody means a human antibody having only the gene sequence of a human chromosome-derived antibody. The anti-DR5 human antibody can be obtained by methods using human antibody-producing mice having a human chromosome fragment containing genes of human antibody H and L chains (e.g., Tomizuka, K. et al., Nature Genetics (1997) 16, p. 133-143; Kuroiwa, Y. et al., Nucl. Acids Res. (1998) 26, p. 3447-3448; Yoshida, H. et al., Animal Cell Technology: Basic and Applied Aspects vol. 10, p. 69-73 (Kitagawa, Y., Matsuda, T. and Iijima, S. eds.), Kluwer Academic Publishers, 1999; and Tomizuka, K. et al., Proc. Natl. Acad. Sci. USA (2000) 97, p. 722-727).

For such transgenic animals, specifically, genetic recombinant animals in which loci of endogenous immunoglobulin heavy and light chains in non-human mammals are broken and loci of human immunoglobulin heavy and light chains are introduced instead via yeast artificial chromosome (YAC) vectors or the like can be created by preparing knockout animals and transgenic animals and crossing these animals.

Moreover, eukaryotic cells are transformed with cDNA encoding each of such humanized antibody heavy and light chains, preferably vectors containing the cDNA, by gene recombination techniques, and transformed cells producing genetic recombinant human monoclonal antibodies can also be cultured to thereby obtain these antibodies from the culture supernatant.

In this context, for example, eukaryotic cells, preferably mammalian cells such as CHO cells, lymphocytes, and myelomas can be used as hosts.

Moreover, methods for obtaining phage-displayed human antibodies selected from human antibody libraries are also known (Wormstone, I.M. et al., Investigative Ophthalmology & Visual Science. (2002) 43 (7), p. 2301-2308; Carmen, S. et al., Briefings in Functional Genomics and Proteomics (2002), 1 (2), p. 189-203; and Siriwardena, D. et al., Ophthalmology (2002) 109 (3), p. 427-431).

For example, a phage display method can be used, which comprises causing human antibody variable regions to be expressed as a single-chain antibody (scFv) on phage surface and selecting phages binding to antigens (Nature Biotechnology (2005), 23, (9), p. 1105-1116).

Genes of the phages selected based on antigen binding can be analyzed to thereby determine DNA sequences encoding human antibody variable regions binding to the antigens.

When the DNA sequence of scFv binding to the antigens is clarified, expression vectors having the sequence can be prepared and introduced into appropriate hosts, followed by gene expression to obtain human antibodies (WO92/01047, WO92/20791, WO93/06213, WO93/11236, WO93/19172, WO95/01438, WO95/15388, Annu. Rev. Immunol (1994) 12, p. 433-455, and Nature Biotechnology (2005) 23 (9), p. 1105-1116).

The antibody genes can be temporarily isolated and then introduced into appropriate hosts to prepare antibodies. In such a case, appropriate hosts and expression vectors can be combined for use.

When eukaryotic cells are used as hosts, animal cells, plant cells, and eukaryotic microorganisms can be used.

Examples of the animal cells can include (1) mammalian cells, for example, monkey COS cells (Gluzman, Y. Cell (1981) 23, p. 175-182, ATCC CRL-1650), mouse fibroblasts NIH3T3 (ATCC No. CRL-1658), and dihydrofolate reductase-deficient strains (Urlaub, G. and Chasin, L.A. Proc. Natl. Acad. Sci. U.S.A. (1980) 77, p. 4126-4220) of Chinese hamster ovarian cells (CHO cells, ATCC CCL-61).

When prokaryotic cells are used, examples thereof can include *Escherichia coli* and *Bacillus subtilis.*

The antibody gene of interest is introduced into these cells by transformation, and the transformed cells are cultured *in vitro* to obtain antibodies.

The isotype of the antibody of the present invention is not limited, and examples thereof include IgG (IgG1, IgG2, IgG3, or IgG4), IgM, IgA (IgA1 or IgA2), IgD, and IgE and can preferably include IgG and IgM.

Moreover, the antibody of the present invention may be an antibody fragment having the antigen-binding site of the antibody or a modified form thereof as long as it maintains binding affinity for the antigen.

Examples of the functional fragment of the antibody include Fab, F(ab')$_2$, Fab' which is a monovalent fragment of an antibody variable region obtained by reducing F(ab')$_2$, Fv, single-chain Fv (scFv) comprising heavy and light chain Fvs linked via an appropriate linker, a diabody, a linear antibody, and a multispecific antibody formed by antibody fragments. However, the functional fragment of the antibody is not limited to these fragments as long as it maintains affinity for the antigen. These antibody fragments can be obtained by treating the full-length antibody molecule with an enzyme such as papain or pepsin. Moreover, the antibody fragments can also be obtained by producing proteins in an appropriate gene expression system using nucleic acid sequences encoding the heavy and light chains of the antibody fragments. Moreover, proteins comprising a cysteine residue-containing polypeptide genetically engineered at the carboxy terminus of the functional fragment of the antibody can also be used as the functional fragment of the antibody according to the present invention. Examples of such a functional fragment can include, but are not limited to, Fab comprising a cysteine residue-containing polypeptide added at the carboxy terminus of the heavy or light chain. The thiol group of the added cysteine residue can be used for conjugating the functional fragment of the antibody to the liposome.

Furthermore the antibody of the present invention may be a multispecific antibody having specificity for at least two different antigens. Such a molecule usually comprises two antigens bound together (i.e., a bispecific antibody). The "multispecific antibody" according to the present invention encompasses antibodies having specificity for more (e.g., three) antigens.

The multispecific antibody used as the antibody of the present invention may be a full-length antibody or a fragment of such an antibody (e.g., a F(ab')$_2$ bispecific antibody). The bispecific antibody can be prepared by binding the heavy and light chains (HL pairs) of two antibodies or can also be prepared by fusing hybridomas producing monoclonal antibodies different from each other to prepare bispecific antibody-producing fused cells (Millstein et al., Nature (1983) 305, p. 537-539).

The antibody of the present invention may be a single-chain variable fragment antibody (also referred to as scFv). The single-chain variable fragment antibody is obtained by linking the heavy and light chain variable regions of the antibody via a polypeptide linker (Pluckthun, The Pharmacology of Monoclonal Antibodies, 113 (Rosenberg and Moore ed., Springer Verlag, New York, p. 269-315 (1994); and Nature Biotechnology (2005), 23, p. 1126-1136).

Methods for preparing the single-chain variable fragment antibody are well known in the art (e.g., U.S. Patent Nos. 4,946,778, 5,260,203, 5,091,513, and 5,455,030). In this single-chain variable fragment antibody, the heavy and light chain variable regions are linked via a linker that does not form a conjugate, preferably a polypeptide linker (Huston, J.S. et al., Proc. Natl. Acad. Sci. U.S.A. (1988), 85, p. 5879-5883). The heavy and light chain variable regions in the single-chain variable fragment antibody may be derived from the same antibodies or may be derived from different antibodies. For example, an arbitrary single-chain peptide of 12 to 19 residues is used as the peptide linker for linking the variable regions.

DNA encoding the single-chain variable fragment antibody is obtained by: amplifying, as templates, the full-length sequences or partial sequences (encoding the desired amino acid sequences) of DNA encoding the heavy chain or heavy chain variable region of the antibody and DNA encoding the light chain or light chain variable region thereof, by a PCR method using primer pairs designed for both ends thereof; and subsequently further amplifying DNA encoding the peptide linker portion in combination with a primer pair designed to respectively link both ends of the linker sequence to the heavy and light chain sequences.

Moreover, once the DNA encoding the single-chain variable fragment antibody is prepared, expression vectors containing the DNA and hosts transformed with the expression vectors can be obtained according to standard methods. Moreover, by use of the hosts, the single-chain variable fragment antibody can be obtained according to standard methods.

For these antibody fragments, their genes are obtained and expressed in the same way as above, and the hosts can be allowed to produce the antibody fragments.

The antibody of the present invention may be a polyclonal antibody, which is a mixture of a plurality of anti-DR5 antibodies differing in amino acid sequences. One example of the polyclonal antibody can include a mixture of a plurality of antibodies differing in CDRs. A mixture of cells producing antibodies different from each other is cultured, and antibodies purified from the culture can be used as such polyclonal antibodies (WO2004/061104).

Antibodies obtained by binding the antibody of the present invention with various molecules such as polyethylene glycol (PEG) can also be used as the modified form of the antibody.

Furthermore, the antibody of the present invention may be a conjugate of these antibodies formed with other drugs (immunoconjugate). Examples of such an antibody can include conjugates obtained by binding these antibodies to radioactive materials or compounds having a pharmacological effect (Nature Biotechnology (2005) 23, p. 1137-1146).

The obtained antibodies can be purified until homogeneous. In the antibody separation and purification, any separation/purification method used for usual proteins can be used.

The antibodies can be separated and purified by appropriately selecting and combining, for example, using chromatography columns, filters, ultrafiltration, salting-out, dialysis, polyacrylamide gel electrophoresis for preparation, and isoelectric focusing (Strategies for Protein Purification and Characterization: A Laboratory Course Manual, Daniel R. Marshak et al. eds., Cold Spring Harbor Laboratory Press (1996); and Antibodies: A Laboratory Manual. Ed Harlow and David Lane, Cold Spring Harbor Laboratory (1988)), though the separation/purification method is not limited thereto.

Examples of chromatography include affinity chromatography, ion-exchange chromatography, hydrophobic chromatography, gel filtration, reverse-phase chromatography, and adsorption chromatography.

These chromatography techniques can be performed using liquid-phase chromatography such as HPLC or FPLC.

Examples of columns used in the affinity chromatography include protein A and protein G columns.

Examples of columns based on the protein A column include Hyper D, POROS, Sepharose F.F. (Pharmacia Inc.).

Moreover, the antibodies can also be purified by use of their affinity for antigens using an antigen-immobilized carrier.

(4) Specific examples of anti-DR5 antibody

For example, anti-DR5 antibodies described in the pamphlets of WO98/51793, WO2001/83560, WO2002/94880, WO2003/54216, WO2006/83971, and WO2007/22157, which induce the apoptosis of DR5-expressing cells may be used as a constituent of the immunoliposome of the present invention. Moreover, anti-DR5 antibodies called Lexatumumab, HGSTR2J, APOMAB, APOMAB7.3, AMG-655, and LBY135 and their variants may also be used as a constituent of the immunoliposome of the present invention. However, the anti-DR5 antibody of the present invention is not limited to these antibodies as long as it is capable of binding to the DR5 protein.

3. Antibody binding to Fas

(1) Fas gene

The nucleotide sequence of the human Fas gene and the amino acid sequence thereof are recorded as GI: 182409 (Accession No: M67454) in GenBank. In this context, the nucleotide sequence of the Fas gene also encompasses nucleotide sequences encoding proteins which consist of an amino acid sequence derived from the Fas amino acid sequence by the substitution, deletion, or addition of one or more amino acids and which have an equivalent biological activity to that of Fas. Moreover, Fas also encompasses proteins which consist of an amino acid sequence derived from the Fas amino acid sequence by the substitution, deletion, or addition of one or more amino acids and which have an equivalent biological activity to that of Fas.

(2) Antibody against Fas

The antibody binding to Fas can be obtained according to the methods described in the paragraph "2.(2) Antibody against DR5".

(3) Other antibodies

The antibodies binding to Fas can be obtained according to the methods described in the paragraph "2.(3) Other antibodies".

(4) Specific examples of anti-Fas antibody

For example, anti-Fas antibodies described in U.S. Patent No. 6,972,323, which induce the apoptosis of Fas-expressing cells, and their variants may be used as a constituent of the immunoliposome of the present invention. However, the anti-Fas antibody of the present invention is not limited to these antibodies as long as it is capable of binding to the Fas protein.

4. Antibodies against other antigens

For receptors other than DR5 and Fas, antibodies that induce, through the binding to the receptors, the apoptosis of cells expressing the receptors can be obtained according to the antibody preparation methods described above, when the receptors contain a death domain. Such antibodies can be used as a constituent of the immunoliposome of the present invention. Examples of the receptors containing a death domain can include TNFRI, DR3, DR4, and DR6. However, the receptors containing a death domain are not limited to these receptors as long as they are receptors that transduce cell apoptosis-inducing signals.

The nucleotide sequence of the human TNFRI gene and the amino acid sequence thereof are recorded as GI: 339748 (Accession No: M75866) in GenBank. In this context, the nucleotide sequence of the TNFRI gene also encompasses nucleotide sequences encoding proteins which consist of an amino acid sequence derived from the TNFRI amino acid sequence by the substitution, deletion, or addition of one or more amino acids and which have an equivalent biological activity to that of TNFRI. Moreover, TNFRI also encompasses proteins which consist of an amino acid sequence derived from the TNFRI amino acid sequence by the substitution, deletion, or addition of one or more amino acids and which have an equivalent biological activity to that of TNFRI.

The nucleotide sequence of the human DR3 (death receptor 3) gene and the amino acid sequence thereof are recorded as GI:23200020 (Accession No: NM_148965) in GenBank. In this context, the nucleotide sequence of the DR3 gene also encompasses nucleotide sequences encoding proteins which consist of an amino acid sequence derived from the DR3 amino acid sequence by the substitution, deletion, or addition of one or more amino acids and which have an equivalent biological activity to that of DR3. Moreover, DR3 also encompasses proteins which consist of an amino acid sequence derived from the DR3 amino acid sequence by the substitution, deletion, or addition of one or more amino acids and which have an equivalent biological activity to that of DR3.

The nucleotide sequence of the human DR4 (death receptor 4) gene and the amino acid sequence thereof are recorded as GI:21361085 (Accession No: NM_003844) in GenBank. In this context, the nucleotide sequence of the DR4 gene also encompasses nucleotide sequences encoding proteins which consist of an amino acid sequence derived from the DR4 amino acid sequence by the substitution, deletion, or addition of one or more amino acids and which have an equivalent biological activity to that of DR4. Moreover, DR4 also encompasses proteins which consist of an amino acid sequence derived from the DR4 amino acid sequence by the substitution, deletion, or addition of one or more amino acids and which have an equivalent biological activity to that of DR4. For example, antibodies described in the pamphlet of WO2002/097033, Mapatumumab, and their variant anti-DR4-antibodies may be used as a constituent of the immunoliposome of the present invention. However, the anti-DR4 antibody of the present invention is not limited to these antibodies as long as it is capable of binding to the DR4 protein.

The nucleotide sequence of the human DR6 (death receptor 6) gene and the amino acid sequence thereof are recorded as GI:23238206 (Accession No: NM_014452) in GenBank. In this context, the nucleotide sequence of the DR6 gene also encompasses nucleotide sequences encoding proteins which consist of an amino acid sequence derived from the DR6 amino acid sequence by the substitution, deletion, or addition of one or more amino acids and which have an equivalent biological activity to that of DR6. Moreover, DR6 also encompasses proteins which consist of an amino acid sequence derived from the DR6 amino acid sequence by the substitution, deletion, or addition of one or more amino acids and which have an equivalent biological activity to that of DR6.

5. Constituents of immunoliposome

The present invention provides an immunoliposome which comprises a protein specifically binding to any of the receptors on cell surfaces involved in apoptosis, described in the paragraphs 1 to 4, and exhibits an apoptosis-inducing activity.

The term "liposome" means a lipid structure formed by an amphiphilic vesicle-forming lipid ((D.D. Lasic, "Liposomes: From Physics to Applications", Elsevier Science Publishers, pp. 1-171 (1993))). The liposome is typically a closed vesicle composed of a unilamellar or multilamellar lipid bilayer having an internal aqueous phase. In the present invention, the liposome refers to a lipid complex particle in a broader sense. For example, the liposome of the present invention encompasses even a complex whose aqueous phase is not definitively proven. The lipid complex contains at least one type of lipid and may additionally contain a hydrophilic polymer, a polysaccharide, an amino acid, and the like. The lipid complex means a particle formed by these constituents via a covalent or non-covalent bond.

The term "immunoliposome" means a complex formed by the liposome and the protein. The protein is not limited to antibodies and also encompasses an endogenous ligand, a functional peptide of the endogenous ligand, and the like.

The immunoliposome contains an amphiphilic vesicle-forming lipid and comprises a protein (e.g., antibody or endogenous ligand) or a peptide specifically binding to the receptor involved in apoptosis induction, wherein the protein or the peptide is supported by the liposome via a covalent or non-covalent bond. The "amphiphilic vesicle-forming lipid" encompasses a lipid that has hydrophobic and hydrophilic moieties and can further form a bilayer vesicle in itself in water, and all amphiphilic lipids that are incorporated together with other lipids into a lipid bilayer, in which the hydrophobic regions thereof are contacted with the internal hydrophobic regions of the bilayer membrane while the hydrophilic regions thereof are arranged to face the outer polar surfaces of the membrane.

The "lipid bilayer" refers to a structure in which the hydrophobic regions of polar lipid molecules are associated with each other and these hydrophobic moieties face the center of the bilayer whereas the hydrophilic regions are arranged to face aqueous phases.

The immunoliposome of the present invention comprises (1) an amphiphilic vesicle-forming lipid, (2) a hydrophilic polymer, (3) a protein or peptide, and the like and may contain a therapeutic drug within the liposome. Moreover, the constituents of the immunoliposome of the present invention are not limited thereto unless they inhibit lipid complex formation. Hereinafter, each constituent of the immunoliposome will be described in detail.

(1) Amphiphilic vesicle-forming lipid

Constituent lipid components of the liposome encompass phospholipids, glycolipids, sphingolipids, sterols, glycols, saturated or unsaturated fatty acids, surfactants, and derivative lipids having a hydrophilic polymer (see the document "Liposomes: From Physics to Applications", Chapter 1. Chemistry of lipids and liposomes). These lipids can be listed as examples in (1)-1. to (1)-8. shown below.

(1)-1. Phospholipids

The phospholipids are broadly classified into glycerophospholipids and sphingophospholipids. Typical examples of the glycerophospholipids include phosphatidylcholine (PC), phosphatidylserine (PS), phosphatidylinositol (PI), phosphatidylglycerol (PG), phosphatidylethanolamine (PE), and phosphatidic acid (PA). On the other hand, typical examples of the sphingophospholipids include sphingomyelin. Examples of these phospholipids can include lipids described in (a) to (i) below.

(a) Phosphatidylcholines

Examples thereof include dipalmitoylphosphatidylcholine (DPPC), distearoylphosphatidylcholine (DSPC), dimyristoylphosphatidylcholine (DMPC), dioleoylphosphatidylcholine (DOPC), dilauroylphosphatidylcholine (DLPC), didecanoylphosphatidylcholine (DDPC), dioctanoylphosphatidylcholine (DOPC), dihexanoylphosphatidylcholine (DHPC), dibutyrylphosphatidylcholine (DBPC), dielaidoylphosphatidylcholine, dilinoleoylphosphatidylcholine, diarachidonoylphosphatidylcholine, dieicosenoylphosphatidylcholine (DEPC), diheptanoylphosphatidylcholine, dicaproylphosphatidylcholine, diheptadecanoylphosphatidylcholine, dibehenoylphosphatidylcholine, eleostearoylphosphatidylcholine, hydrogenated egg phosphatidylcholine (HEPC), hydrogenated soybean phosphatidylcholine (HSPC), 1-palmitoyl-2-arachidonoylphosphatidylcholine, 1-palmitoyl-2-oleoylphosphatidylcholine, 1-palmitoyl-2-linoleoylphosphatidylcholine, 1-palmitoyl-2-myristoylphosphatidylcholine, 1-palmitoyl-2-stearoylphosphatidylcholine, 1-stearoyl-2-palmitoylphosphatidylcholine, 1,2-dimyristoylamido-1,2-deoxyphosphatidylcholine, 1-myristoyl-2-palmitoylphosphatidylcholine, 1-myristoyl-2-stearoylphosphatidylcholine, di-O-hexadecylphosphatidylcholine, transdielaidoylphosphatidylcholine, dipalmitelaidoylphosphatidylcholine, n-octadecyl-2-methylphosphatidylcholine, n-octadecylphosphatidylcholine, 1-laurylpropanediol-3-phosphocholine, erythro-N-lignoceroylsphingophosphatidylcholine, and palmitoyl-(9-cis-octadecenoyl)-3-sn-phosphatidylcholine.

(b) Phosphatidylserines

Examples thereof include distearoylphosphatidylserine (DSPS), dimyristoylphosphatidylserine (DMPS), dilauroylphosphatidylserine (DLPS), dipalmitoylphosphatidylserine (DPPS), dioleoylphosphatidylserine (DOPS), lysophosphatidylserine, eleostearoylphosphatidylserine, and 1,2-di-(9-cis-octadecenoyl)-3-sn-phosphatidylserine.

(c) Phosphatidylinositols

Examples thereof include dipalmitoylphosphatidylinositol (DPPI), distearoylphosphatidylinositol (DSPI), and dilauroylphosphatidylinositol (DLPI).

(d) Phosphatidylglycerols

Examples thereof include dipalmitoylphosphatidylglycerol (DPPG), distearoylphosphatidylglycerol (DSPG), dioleoylphosphatidylglycerol (DOPG), dilauroylphosphatidylglycerol (DLPG), dimyristoylphosphatidylglycerol (DMPG), lysophosphatidylglycerol, hydrogenated soybean phosphatidylglycerol (HSPG), hydrogenated egg phosphatidylglycerol (HEPG), and cardiolipin (diphosphatidylglycerol).

(e) Phosphatidylethanolamines (cephalins)

Examples thereof include dipalmitoylphosphatidylethanolamine (DPPE), distearoylphosphatidylethanolamine (DSPE), dioleoylphosphatidylethanolamine (DOPE), dilauroylphosphatidylethanolamine (DLPE), dimyristoylphosphatidylethanolamine (DMPE), didecanoylphosphatidylethanolamine (DDPE), N-glutarylphosphatidylethanolamine (NGPE), lysophosphatidylethanolamine, N-(7-nitro-2,1,3-benzoxadiazol-4-yl)-1,2-dioleoyl-sn-phosphatidylethanolamine, eleostearoylphosphatidylethanolamine, N-succinyldioleoylphosphatidylethanolamine, and 1-hexadecyl-2-palmitoylglycerophosphatidylethanolamine.

(f) Phosphatidic acids

Examples thereof include dipalmitoyl phosphatidic acid (DPPA), distearoyl phosphatidic acid (DSPA), dimyristoyl phosphatidic acid (DMPA), and dioleoyl phosphatidic acid (DOPA).

(g) Sphingophospholipids

Examples thereof include sphingomyelin, dipalmitoylsphingomyelin, distearoylsphingomyelin, ceramide ciliatine, ceramide phosphorylethanolamine, and ceramide phosphorylglycerol.

(h) Polymerizable phospholipids having polymerizable residue

Examples of the polymerizable phospholipids having a polymerizable residue as unsaturated phospholipids include 1,2-bis(2,4-octadecadienoyl)-sn-glycero-3-phosphocholine, 1,2-bis(2,4-hexadecadienoyl)-sn-glycero-3-phosphocholine, 1-(octadecanoyl)-2-(2,4-octadecadienoyl)-sn-glycero-3-phosphocholine, 1-(hexadecanoyl)-2-(2,4-octadecadienoyl)-sn-glycero-3-phosphocholine, 1-(octadecanoyl)-2-(2,4-hexadecadienoyl)-sn-glycero-3-phosphocholine, 1-(hexadecanoyl)-2-(2,4-hexadecadienoyl)-sn-glycero-3-phosphocholine, 1-(2,4-octadecadienoyl)-2-(octadecanoyl)-sn-glycero-3-phosphocholine, 1-(2,4-hexadecadienoyl)-2-(hexadecanoyl)-sn-glycero-3-phosphocholine, 1,2-bis-(8,10,12-octadecatrienoyl)-sn-glycero-3-phosphocholine, 1,2-bis(12-methacryloyloxydodecanoyl)-sn-glycero-3-phosphocholine, and 1,2-bis(9-(p-vinylbenzoyl)nonanoyl)-sn-glycero-3-phosphocholine.

Moreover, examples of other polymerizable phospholipids include 1,2-bis(2,4-octadecadienoyl)-sn-glycero-3-phosphoethanolamine, 1,2-bis(2,4-hexadecadienoyl)-sn-glycero-3-phosphoethanolamine, 1-(octadecanoyl)-2-(2,4-octadecadienoyl)-sn-glycero-3-phosphoethanolamine, 1-(hexadecanoyl)-2-(2,4-octadecadienoyl)-sn-glycero-3-phosphoethanolamine, 1-(octadecanoyl)-2-(2,4-hexadecadienoyl)-sn-glycero-3-phosphoethanolamine, 1-(hexadecanoyl)-2-(2,4-hexadecadienoyl)-sn-glycero-3-phosphoethanolamine, 1-(2,4-octadecadienoyl)-2-(octadecanoyl)-sn-glycero-3-phosphoethanolamine, 1-(2,4-hexadecadienoyl)-2-(hexadecanoyl)-sn-glycero-3-phosphoethanolamine, 1,2-bis-(8,10,12-octadecatrienoyl)-sn-glycero-3-phosphoethanolamine, 1,2-bis(12-methacryloyloxydodecanoyl)-sn-glycero-3-phosphoethanolamine, and 1,2-bis(9-(p-vinylbenzoyl)nonanoyl)-sn-glycero-3-phosphoethanolamine.

Further examples of other polymerizable phospholipids include phosphoric acid derivatives such as 1,2-bis(2,4-octadecadienoyl)-sn-glycero-3-phosphoric acid, 1,2-bis(2,4-hexadecadienoyl)-sn-glycero-3-phosphoric acid, 1-(octadecanoyl)-2-(2,4-octadecadienoyl)-sn-glycero-3-phosphoric acid, 1-(hexadecanoyl)-2-(2,4-octadecadienoyl)-sn-glycero-3-phosphoric acid, 1-(octadecanoyl)-2-(2,4-hexadecadienoyl)-sn-glycero-3-phosphoric acid, 1-(hexadecanoyl)-2-(2,4-hexadecadienoyl)-sn-glycero-3-phosphoric acid, 1-(2,4-octadecadienoyl)-2-(octadecanoyl)-sn-glycero-3-phosphoric acid, 1-(2,4-hexadecadienoyl)-2-(hexadecanoyl)-sn-glycero-3-phosphoric acid, 1,2-bis-(8,10,12-octadecatrienoyl)-sn-glycero-3-phosphoric acid, 1,2-bis(12-methacryloyloxydodecanoyl)-sn-glycero-3-phosphoric acid, and 1,2-bis(9-(p-vinylbenzoyl)nonanoyl)-sn-glycero-3-phosphoric acid, and salts thereof.

In this context, the polymerizable phospholipids may contain a non-polymerizable fatty acid residue. Examples of the non-polymerizable fatty acid residue include linear or branched alkyl groups having 2 to 24 carbon atoms, acyl groups, non-polymerizable alkenyl groups, and non-polymerizable alkenoyl groups.

(i) Other phospholipids

Examples thereof include phosphatidylthreonine, dicetyl phosphate, lysophospholipid, and egg or soybean lecithin, a mixture of a plurality of lipids which is composed mainly of phosphatidylcholine and comprises phosphatidylethanolamine, sphingomyelin, cholesterol, and the like.

(1)-2. Glycolipids

The glycolipids are broadly classified into glyceroglycolipids and sphingoglycolipids. Examples thereof can include lipids descried in (a) to (c) below.

(a) Glyceroglycolipids

Examples thereof include diglycosyl diglyceride, glycosyl diglyceride, digalactosyl diglyceride, galactosyl diglyceride, sulfoxyribosyl diglyceride, (1,3)-D-mannosyl-(1,3)diglyceride, digalactosyl glyceride, digalactosyl dilauroyl glyceride, digalactosyl dimyristoyl glyceride, digalactosyl dipalmitoyl glyceride, digalactosyl distearoyl glyceride, galactosyl glyceride, galactosyl dilauroyl glyceride, galactosyl dimyristoyl glyceride, galactosyl dipalmitoyl glyceride, galactosyl distearoyl glyceride, and digalactosyl diacyl glycerol.

(b) Sphingoglycolipids

Examples thereof include ceramide (cerebroside), galactosylceramide, lactosylceramide, digalactosylceramide, ganglioside $GM_1$, ganglioside $GM_2$, ganglioside $GM_3$, sulfatide, ceramide oligohexoside, and globoside.

(c) Other glycolipids

Examples thereof include ceramide oligohexoside, palmityl glycoside, stearyl glycoside, myristyl glycoside, alkyl glycoside, aminophenyl glycoside, cholesteryl maltoside, cholesteryl glycoside, 3-cholesteryl-6'-(glycosylthio)hexyl ether glycolipid, and glucamides.

(1)-3. Sterols

The most typical example of the sterols includes cholesterol. The cholesterol is known to contribute to the membrane rigidity and stability of a lipid bilayer structure. Examples of the sterols other than the cholesterol include cholesterol succinic acid, dihydrocholesterol, lanosterol, dihydrolanosterol, desmosterol, stigmasterol, sitosterol, campesterol, brassicasterol, zymosterol, ergosterol, campesterol, fucosterol, 22-ketosterol, 20-hydroxysterol, 7-hydroxycholesterol, 19-hydroxycholesterol, 22-hydroxycholesterol, 25-hydroxycholesterol, 7-dehydrocholesterol, $5\alpha$-cholest-7-en-3$\beta$-ol, epicholesterol, dehydroergosterol, cholesterol sulfate, cholesterol hemisuccinate, cholesterol phthalate, cholesterol phosphate, cholesterol valerate, 3$\beta$N-(N',N'-dimethylaminoethane)-carbamoyl cholesterol, cholesterol acetate, cholesteryl oleate, cholesteryl linoleate, cholesteryl myristate, cholesteryl palmitate, cholesteryl arachidate, coprostanol, cholesterol ester, cholesteryl phosphocholine, and 3,6,9-trioxaoctan-1-ol-cholesteryl-3e-ol.

(1)-4. Neutral lipids

Examples of the neutral lipids include diglyceride (e.g., diolein and dipalmitolein) and mixed caprylin-caprin diglyceride, triacylglycerol (e.g., triolein, tripalmitolein, trimyristolein, trilaurin, tricaprin, tricaprylin, and tricaproin), squalene, tocopherol, and cholesterol.

(1)-5. Saturated or unsaturated fatty acids

Examples of the saturated and unsaturated fatty acids that may be used include saturated or unsaturated fatty acids having 5 to 30 carbon atoms, such as caprylic acid, pelargonic acid, capric acid, undecylenic acid, lauric acid, tridecylenic acid, myristic acid, pentadecylenic acid, palmitic acid, margaric acid, stearic acid, nonadecylenic acid, arachidic acid, dodecenoic acid, tetradecenoic acid, oleic acid, linoleic acid, linolenic acid, eicosenoic acid, erucic acid, and docosapentaenoic acid.

(1)-6. Charged lipids

Examples thereof can include lipids described in (a) to (b) below.

(a) Anionic lipids

The anionic lipids refer to lipids that are negatively charged at physiological pH. Examples thereof include: acidic phospholipids such as phosphatidylglycerol, phosphatidic acid, phosphatidylserine, phosphatidylinositol, and cardiolipin; fatty acids such as oleic acid, palmitic acid, stearic acid, myristic acid, linoleic acid, and linolenic acid; gangliosides such as ganglioside $GM_1$, ganglioside $GM_2$, and ganglioside $GM_3$; acidic lipids such as dicetyl phosphate; and acidic amino acid surfactants such as N-acyl-L-glutamic acid.

(b) Cationic lipids

The cationic lipids refer to lipids that are positively charged at physiological pH. Examples thereof include N,N-distearyl-N,N-dimethyl ammonium bromide (DDAB), cetyltrimethylammonium bromide (CTAB), N-($\alpha$-trimethylammonioacetyl)-didodecyl-D-glutamate chloride (TMAG), DL-1,2-dioleoyl-3-dimethylaminopropyl-$\beta$-hydroxyethyl ammonium (DORI), N-[1-(2,3-dioleyloxy)propyl]-N,N-dimethyl-N-hydroxyethyl ammonium bromide (DORIE), N-(1,2-dimyristyloxyprop)-3-yl)-N,N-dimethyl-N-hydroxyethyl ammonium bromide (DMRIE), (1,2-dioleyloxypropyl)-N,N,N-trimethyl ammonium chloride (DOTMA), diheptadecylamidoglycyl spermidine, N,N-dioctadecylamidoglycyl spermine, N,N-dioctadecylamidoglycine, 1,2-dioleyl-3-succinyl-sn-glycerocholine ester (DOSC), 1,2-dioleyl-sn-glycero-3-succinyl-2-hydroxyethyl disulfide ornithine (DOGSDSO), cetylpyridinium chloride (CPyC), 1,2-dioleyl-sn-glycero-3-ethylphosphocholine (DOEPC), N-(1-(2,3-dioleyloxy)propyl)-N-(2-(sperminecarboxamido)ethyl)-N,N-dimethyl ammonium trifluoroacetate (DOSPA), N-(1-(2,3-dioleoyloxy)propyl)-N,N,N-trimethyl ammonium chloride (DOTAP), N,N-dioleyl-N,N-dimethyl ammonium chloride (DODAC), 1,2-dioleoyl-sn-3-phosphoethanolamine (DOPE), 1,2-dioleoyl-3-(4'-trimethylammonio)butanoyl-sn-glycerol (DOBT), 1,3-dioleoyloxy-2-(6-carboxyspermyl)-propylamide (DOSPER), lipopolyamine (e.g., dipalmitoyl phosphatidylethanolamidospermine (DPPES)), O-alkylphosphatidylcholine, O-alkylphosphatidylethanolamine, amides and phosphatidylethanolamines of lysine, arginine, or ornithine, 3β-(N-(N',N'-dimethylaminoethane)carbamoyl) cholesterol (DC-Chol), 3-β-[N-(N',N',N'-trimethylaminoethane)carbamoyl]cholesterol (TC-Chol), bis-guanidium-spermidine-cholesterol (BGSC), bis-guanidium-tren-cholesterol (BGTC), cholesteryl (4'-trimethylammonio) butanoate (ChOTB), 3β-N-(polyethyleneimine)-carbamoyl cholesterol, cholesteryl-3β-carboxyl-amido-ethylenetrimethylammonium iodide, 1-dimethylamino-3-trimethylammonio-DL-2-propyl-cholesteryl carboxylate iodide, cholesteryl-3β-carboxyamidoethyleneamine, cholesteryl-3β-oxysuccinamido-ethylenetrimethylammonium iodide, 1-dimethylamino-3-trimethylammonio-DL-2-propyl-cholesteryl-3β-oxysuccinate iodide, 2-(2-trimethylammonio)-ethylmethylaminoethyl-cholesteryl-3β-oxysuccinate iodide, cholesteryl hemisuccinic acid ester, long-chain amine, long-chain pyridinium compounds, and quaternary ammonium compounds.

(1)-7. Surfactants

The surfactants are broadly classified into (a) cationic surfactants, (b) anionic surfactants, and (c) amphoteric surfactants, which have an ionic hydrophilic moiety, and (d) non-ionic surfactants, which have a non-ionic hydrophilic moiety.

(a) Cationic surfactants

Examples of the cationic surfactants include alkylamine salts, acylamine salts, quaternary ammonium salts, and amine derivatives. Specific examples thereof include benzalkonium chloride, acylaminoethyldiethylamine salts, N-alkylpolyalkylpolyamine salts, fatty acid polyethylene polyamide, cetyltrimethylammonium bromide, dodecyltrimethylammonium bromide, alkylpolyoxyethyleneamine, N-alkylaminopropylamine, and fatty acid triethanolamine ester.

(b) Anionic surfactants

Examples of the anionic surfactants include acylsarcosine, sodium alkylsulfate, sodium alkylbenzenesulfonate, sodium alkylsulfuric acid ester, sodium alkyl ether sulfate, sodium alpha-olefin sulfonate, sodium alpha-sulfofatty acid ester, and fatty acid sodium or potassium having 7 to 22 carbon atoms. Specific examples thereof include sodium dodecyl sulfate, sodium lauryl sulfate, sodium cholate, sodium deoxycholate, and sodium taurodeoxycholate.

(c) Amphoteric surfactants

Examples of the amphoteric surfactants include alkylamino fatty acid sodium, alkyl betaine, and alkylamine oxide. Specific examples thereof include 3-[(3-cholamidopropyl)dimethylammonio]-1-propanesulfonic acid and N-tetradecyl-N,N-dimethyl-3-ammonio-1-propanesulfonic acid.

(d) Non-ionic surfactants

Examples of the non-ionic surfactants include polyoxyethylene alkyl ether, polyoxyethylene alkylphenyl ether, polyoxyethylene sorbitan fatty acid ester, sorbitan fatty acid ester, sucrose fatty acid ester, glycerin fatty acid ester, fatty acid alkanolamide, block polymer-based non-ionic surfactants, alkylamine-based non-ionic surfactants, and alkylamide-based non-ionic surfactants. Specific examples thereof include polyoxyethylene sorbitan monooleate, polysorbate 80, polyoxyethylene polyoxypropylene glycol, Pluronic F68, sorbitan monolaurate, sorbitan monooleate, polyoxyethylene hydrogenated castor oil 60, and polyoxyethylene lauryl alcohol.

(1)-8.

A "lipid derivative of a hydrophilic polymer" (described in detail in the paragraph "6.(1) Binding mode of lipid with hydrophilic polymer" comprising any of the lipids bound with a hydrophilic polymer can also be used as a constituent of the immunoliposome of the present invention.
The amphiphilic vesicle-forming lipids shown above are usually mixed appropriately to prepare a liposome. Examples of typical lipid composition can include a lipid composition that is composed mainly of phospholipid or glycolipid and further contains sterol at a content of 20 to 50 mol% with respect to the number of moles of total lipids. Preferable examples thereof can include a lipid composition that is composed mainly of phosphatidylcholines and further contains cholesterol at a content of 20 to 50 mol% with respect to the number of moles of total lipids.

(2) Hydrophilic polymer

Liposomes have the property of being easily captured by the reticulo-endothelial system (RES) in the liver, spleen, lung, or the like, when administered to blood circulation. This property is advantageous if the target organ is the liver, lung, or the like. However, the property is a large disadvantage in targeting a site other than RES for the purpose of achieving systemic effects. The presence of the hydrophilic polymer provides the property of improving blood retention and circumventing the RES uptake of the liposome. Moreover, the presence of the hydrophilic polymer also provides dispersion stability during liposome storage.

Examples of the type of the hydrophilic polymer used in the immunoliposome of the present invention include polyvinyl pyrrolidone (PVP), polyalkylene oxide, polyalkylene glycol (e.g., polyethylene glycol (PEG), polypropylene glycol, polytetramethylene glycol, and polyhexamethylene glycol), polyglycerin, polyacrylic acid, polyacrylamide, polyethyleneimine, polyglycidol, ganglioside, dextran, Ficoll, polyvinyl alcohol, polyvinyl methyl ether, polymethyloxazoline, polyethyloxazoline, polyhydroxypropyloxazoline, polyhydroxypropylmethacrylamide, polymethacrylamide, polydimethylacrylamide, poly(hydroxypropyl methacrylate), poly(hydroxyethyl acrylate), hydroxymethylcellulose, hydroxyethylcellulose, hydroxypropylcellulose, polyaspartamide, polyphosphazene, poly(hydroxyalkylcarboxylic acid), ethylene glycol-propylene glycol copolymers, styrene-maleic anhydride copolymers, divinyl ether-maleic anhydride alternating copolymers, cyclodextrin, hyaluronic acid, cerebroside sulfate, chondroitin sulfate, polysaccharides (e.g., amylose, amylopectin, pectin, chitosan, mannan, pullulan, carrageenan, and methylcellulose), polypeptides, and synthetic polyamino acids (e.g., poly-L-lysine).

The hydrophilic polymer comprises one or more repeating unit structures. The number of the unit structures is not limited, and the hydrophilic polymer may be linear or branched. Preferable examples thereof can include hydrophilic polymers having a molecular weight of 500 to 20000. More preferable examples thereof can include hydrophilic polymers having a molecular weight of 2000 to 5000. Moreover, these polymers can be used as homopolymers or block or random copolymers. The hydrophilic polymer of the present invention also encompasses, for example, polylactic acid/polyglycolic acid copolymers consisting of polylactic acid and polyglycolic acid.

The hydrophilic polymer may be a derivative obtained by introducing, into the polymer, a substituent such as an alkyl, alkoxy, hydroxyl, carbonyl, alkoxycarbonyl, cyano, amino, thiol, maleimide, vinylsulfone, or, hydrazide group.

For the purpose of improving blood retention and circumventing the RES uptake of the liposome, preferable examples of the content of the hydrophilic polymer or the lipid derivative of the hydrophilic polymer in the liposome can include a content of 0.1 to 10 mol% with respect to the number of moles of total lipids. More preferably, it is a content of 1 to 6 mol% with respect to the number of moles of total lipids.

(3) Protein

The antibodies described in the paragraphs "2. Antibody binding to DR5", "3. Antibody binding to Fas", and "4. Antibodies binding to other antigens" can be used as a constituent of the immunoliposome of the present invention. Moreover, a liposome bound with a natural ligand for the receptor having an apoptosis-inducing ability described in the present invention is also included in the scope of the present invention. Furthermore, an artificial peptide specifically binding to the death domain-containing receptor can also be used as a constituent of the immunoliposome of the present invention. Examples of such an artificial peptide can include functional peptides binding to Fas (Yoshimori et al., Apoptosis 10 (2), 323-329 (2005)). Moreover, two or more molecules binding to the same antigen can be selected from the group of molecules described above and allowed to coexist on the same immunoliposome to thereby improve the ability to bind to the particular antigen. Furthermore, two or more molecules binding to different antigens can also be selected from the group of molecules described above and allowed to coexist on the same immunoliposome to thereby achieve the effects on a plurality of antigens.

(4) Internal aqueous phase

When the liposome has an internal aqueous phase, an aqueous solution used in the aqueous phase is not particularly limited unless it inhibits liposome formation. An aqueous sodium chloride solution, a buffer solution (e.g., a phosphate, acetate, or HEPES buffer solution), or a monosaccharide or disaccharide solution (e.g., aqueous glucose or trehalose solution) can usually be used.

(5) Additives

A functionality-imparting agent may further be added to the constituents of the immunoliposome. Examples of such a functionality-imparting agent include membrane stabilizers, hydrophilicity modifiers for membrane surface, curvature regulators, antioxidants, charge-imparting agents, and cryoprotectant agents. Specific examples thereof include stabilizers such as sugar, glycolipid, glycerin, and polyethylene glycol, and antioxidants such as tocopherol and ascorbic acid. Cholesterols may be used as membrane stabilizers, hydrophilicity modifiers for membrane surface, or curvature regulators for the liposome. Tocopherols may be used as antioxidants.

Moreover, when these additives are added to the immunoliposome, the types and contents thereof are not particularly limited and appropriately selected in consideration of the physical properties of the immunoliposome.

(6) Encapsulated drug

Moreover, the immunoliposome of the present invention can encapsulate a hydrophilic drug in the internal aqueous phase and a hydrophobic drug in the membrane. The encapsulated drugs are not particularly limited unless they inhibit liposome formation. Examples thereof include drugs having a cytotoxic activity, such as antitumor agents, antirheumatic agents, antiviral agents, and antimicrobial agents. Examples of the antitumor agents include bleomycin, carboplatin, chlorambucil, cisplatin, colchicine, cyclophosphamide, daunorubicin, dactinomycin, diethylstilbestrol, doxorubicin, etoposide, 5-fluorouracil, floxuridine, melphalan, gemcitabine, imatinib, irinotecan, methotrexate, mitomycin, 6-mercaptopurine, paclitaxel, sorafenib, sunitinib, teniposide, 6-thioguanine, vincristine, and vinblastine. Further examples of anticancer compounds and therapeutic agents are found in The Merck Manual of Diagnosis and Therapy, 15th edition, Berkow et al. ed., 1987, and Rahway, N.J. and Sladek et al., Metabolism and Action of Anti-Cancer Drugs, 1987, Powis et al. ed., Taylor and Francis, New York, N.Y.

Examples of therapeutic agents for autoimmune disease and anti-inflammatory agents can include nonsteroidal anti-inflammatory agents such as diclofenac, loxoprofen sodium, celecoxib, etodolac, meloxicam, rofecoxib, piroxicam, indomethacin, ibuprofen, and naproxen, and disease modifying antirheumatic drugs such as methotrexate, chloroquine, hydrochloroquine, cyclosporine, penicillamine, sulfasalazine, azathioprine, and leflunomide.

As described above, various drugs can be encapsulated in the internal aqueous phase of the immunoliposome such that they act. On the other hand, these drugs can be allowed to act on the target cells by the combined use with the immunoliposome, with them unencapsulated in the immunoliposome.

6. Binding mode of immunoliposome components

The immunoliposome of the present invention comprises an amphiphilic vesicle-forming lipid, a hydrophilic polymer, a protein, a peptide, a sugar, and so on, wherein the lipid, the protein, the peptide, and the hydrophilic polymer form a covalent or non-covalent bond. The immunoliposome of the present invention may have a form in which the functional molecule such as the protein or peptide is directly bound to the liposome surface or bound to the liposome via the hydrophilic polymer. In the former case, the hydrophilic polymer may also be directly bound to the liposome such that it is contained in the liposome.

(1) Binding mode of lipid with hydrophilic polymer

The lipid derivative of the hydrophilic polymer comprises the amphiphilic vesicle-forming lipid described in the paragraph 5.(1) and the hydrophilic polymer described in the paragraph 5.(2). The combination therebetween is not particularly limited and can be selected appropriately according to the purpose. The lipid and the hydrophilic polymer are linked through a covalent bond formed, either directly or via a linker, between the functional group of the lipid (including a functional group artificially introduced in the lipid) and the functional group of the hydrophilic polymer (including a functional group artificially introduced in the hydrophilic polymer).

Examples of the lipid derivative of the hydrophilic polymer include, but not limited to, the followings: polyethylene glycol-modified lipids, polyethyleneimine derivatives, polyvinyl alcohol derivatives, polyacrylic acid derivatives, polyacrylamide derivatives, dextran derivatives, polyglycerin derivatives, chitosan derivatives, polyvinyl pyrrolidone derivatives, polyaspartic acid amide derivatives, poly-L-lysine derivatives, mannan derivatives, and pullulan derivatives.

Examples of the "polyethylene glycol (PEG)-modified lipids" can include polyethylene glycol (PEG)-modified phosphatidylethanolamines described in the document ((D.D. Lasic, "Liposomes: From Physics to Applications", Elsevier Science Publishers, pp. 1-171 (1993)), "Chapter 11. Liposomes as a drug delivery system") and more specifically include N-monomethoxy poly(ethylene glycol)succinyl phosphatidylethanolamines, N-monomethoxy poly(ethylene glycol)carbonyl phosphatidylethanolamines, N-monomethoxy poly(ethylene glycol)ethylene phosphatidylethanolamines, N-monomethoxy poly(ethylene glycol)carbonyl ethylcarbonyl phosphatidylethanolamines, N-monomethoxy poly(ethylene glycol)carbonyl propylcarbonyl phosphatidylethanolamines, N-monomethoxy polyethylene glycol(2-chloro-1,3,5-triazine-4,6-diyl)succinyl phosphatidylethanolamines, di-$C_{12-24}$ acyl-glycero-phosphatidylethanolamine-N-PEG, di-$C_{12-24}$ acylglycerol-mono-PEG ether, mono-$C_{12-24}$ acylglycerol-di-PEG ether, polyethylene glycol alkyl ether, N-(2,3-dimyristyloxypropyl)amide polyethylene glycol methyl ether, N-(2,3-dimyristyloxypropyl)carbamate polyethylene glycol methyl ether, N-(2,3-dimyristyloxypropyl)succinamide polyethylene glycol methyl ether, sphingosine-1-(succinylmethoxy polyethylene glycols, sphingosine-1-(succinyl(methoxy polyethylene glycol))s, polyethylene glycol sorbitan fatty acid ester, polyethylene glycol fatty acid ester (e.g., polyethylene glycol monostearate, polyethylene glycol monooleate, polyethylene glycol dilaurate, polyethylene glycol distearate, polyethylene glycol dioleate, and diethylene glycol stearate), polyethylene glycol castor oil, pyrrolidone carboxylic acid PEG-40 hydrogenated castor oil isostearate, monomethoxy poly(ethylene glycol)-3,5-dipentadecyloxybenzyl carbamate, 4-(N'-(methoxy poly(ethylene glycol)carbonyl)2-aminoethyl)N,N-distearoylbenzamide, DSPE-PEG (products of NOF CORPORATION; SUNBRIGHT DSPE-020C, DSPE-050C, DSPE-020G, DSPE-050G, DSPE-020CN, and DSPE-050CN), DSPE-Multi-arm PEG (product of NOF CORPORATION; SUNBRIGHT DSPE-PTE020), DSPE-Comb-shaped PEG (product of NOF CORPORATION; SUNBRIGHT DSPE-AM0530K), Diacylglycerol-PEG (products of NOF CORPORATION; SUNBRIGHT GS-020 and GS-050), Cholesterol-PEG (products of NOF CORPORATION; SUNBRIGHT CS-010, CS-020, and CS-050), PEG-Ceramide (product of Avanti POLAR LIPID, INC.), mPEG-DTB-DSPE (DTB: dithiobenzyl; the pamphlet of WO2005/051351), and PEG-DAA (DAA: dialkyloxypropyl; the pamphlet of WO2005/026372).

Other examples of the lipid derivative of the hydrophilic polymer can specifically include polyoxyethylene castor oil/hydrogenated castor oil (e.g., polyoxyethylene POE (3) castor oil and POE (5) hydrogenated castor oil), N-[omega-methoxy poly(oxyethylene)-alpha-oxycarbonyl]-phosphatidylethanolamines, O-($C_{10-18}$ alkanoyl or alkenoyl)pullulan, N-($C_{10-18}$ alkanoyl or alkenoyl)polyacrylamide, and DSPE-Polyglycerine (product of NOF CORPORATION; SUNBRIGHT DSPE-PGBG).

Examples of methods for binding the hydrophilic polymer to the lipid include (a) a method which comprises binding the hydrophilic polymer to a liposome dispersion formed in advance, and (b) a method which comprises preparing a lipid derivative of the hydrophilic polymer and incorporating it into the liposome.

In the method (a) which comprises binding the hydrophilic polymer to a liposome dispersion formed in advance, for example, tresyl chloride-activated PEG is used and added under high-pH conditions to a liposome containing a lipid having an amino group (e.g., phosphatidylethanolamine) to thereby obtain a hydrophilic polymer-modified liposome (Senior et al., Biochim. Biophys. Acta. 1062: 77-82 (1991)). Moreover, it is well known by those skilled in the art that many other bonds can be used.

In the method (b) which comprises preparing a lipid derivative of the hydrophilic polymer and incorporating it to the liposome, the hydrophilic polymer can be used in the form of a lipid-bound derivative to thereby insert the hydrophobic site thereof into the lipid layer of the liposome. This hydrophilic polymer derivative is added to a liposome dispersion formed in advance, and the mixture is heated; or otherwise, the hydrophilic polymer derivative is added during the mixing of the constituent lipids of the liposome. As a result, a hydrophilic polymer-modified liposome can be obtained. The production of the lipid derivatized by the hydrophilic polymer is described in, for example, U.S. Patent No. 5,395,619, and it can be produced according to methods known in the art. For example, for forming a covalent bond between the reactive functional group of phospholipid and PEG, methods known in the art are used, such as a method using cyanuric chloride (Blume et al., Biochim. Biophys. Acta. 1029: 91-97 (1990) and U.S. Patent No. 5,213,804), a method using carbodiimide (Proc. Intern. Symp. Control. Rel. Bioact. Mater., 17: 77-78 (1990); Proc. Natl. Acad. Sci. USA, 88: 11460-11464 (1991); Biochim. Biophys. Acta., 1066: 29-36 (1991); Biochim. Biophys. Acta., 1105: 193-200 (1992); and Period. Biol., 93: 349-352 (1991)), a method using an acid anhydride, and a method using glutaraldehyde.

(2) Binding mode of lipid with protein

Examples of methods for immobilizing the protein or peptide or the like onto the liposome include methods shown in (i) to (x) below. The binding of the constituent lipids of the liposome with the protein is formed by a covalent bond between the functional group of the lipid (including a functional group artificially introduced into the lipid) and the functional group of the protein (including a functional group artificially introduced into the protein) or by a non-covalent bond based on physical/biological affinity. Examples of combinations of the functional groups forming the covalent bond include amino/carboxyl groups, amino group/N-hydroxysuccinimide ester, amino/aldehyde groups, amino/tresyl groups, amino/nitrophenylcarbonyl groups, amino/acetal groups, amino/isothiocyanate groups, amino/acyl halide groups, amino/benzotriazole carbonate groups, hydrazide/aldehyde groups, thiol/maleimide groups, thiol/vinylsulfone groups, and thiol/thiol groups. Hydrophobic bonds or specific bonds such as avidin/biotin or polyhistidine tag/nickel bonds can be used for the non-covalent bond.

(i) A method which comprises: introducing a hydrophobic group into the protein such that the protein has affinity for the lipid layer of the liposome placed in a hydrophobic atmosphere; and incorporating the resulting protein into a liposome prepared separately (Biochimica et Biophysica Acta, 812, 116 (1985)).

(ii) A method which comprises causing the antibody or the endogenous ligand to be expressed as a fusion protein with a cell membrane protein and incorporating this membrane protein portion into a liposome prepared separately (Mol Med. 7 (10) 723, (2001)).

(iii) A method which comprises, when the liposome contains glycolipid as a constituent lipid component, oxidizing this sugar with an oxidizing agent to form an aldehyde group, which is in turn reacted with the amino group of the protein for Schiff base formation to thereby bind the lipid with the protein (Biochimica et Biophysica Acta. 640, 66 (1981)).

(iv) A method which comprises mixing a lipid having an amino group-reactive functional group during liposome preparation and reacting this functional group with the Lys residue ε-amino group or N-terminal α-amino group of the protein to incorporate the protein to the liposome (J. Immunol Methods. 1983, 65 (3): 295-306).

Examples of the reactive functional group of the lipid necessary for forming a covalent bond between the amino group of the protein and the lipid include N-hydroxysuccinimide ester (NHS ester), aldehyde, tresyl, nitrophenylcarbonyl, acetal, carboxyl, isothiocyanate, acyl halide, and benzotriazole carbonate groups.

(v) A method which comprises mixing a lipid having a thiol group-reactive functional group during liposome preparation and reacting this functional group with a protein having a thiol group (Journal of Immunological Method, 75, 351 (1984); and Biochemical and Biophysical Research Communications, 117, 399 (1983)).

The thiol group is added to the protein by methods known in the art, for example, methods using compounds such as N-succinimidyl-3-(2-pyridyldithio)propionate (SPDP) (Carlsson, J. et al., Biochem. J. 173, 723, (1978)), N-succinimidyl-S-acetylthioacetate (SATA), N-succinimidyl-S-acetylthiopropionate (SATP), iminothiolane (Traut, R.R. et al., Biochemistry 12, 3266 (1973)), and mercaptoalkylimidate. In addition to these methods, the endogenous dithiol group of the protein is reduced to a thiol group, which can in turn be used in the binding. Particularly, when the protein is an antibody, dithiol groups in the hinge region of the antibody (full-length molecule or fragment) and in the linkage between the heavy and light chains thereof can be used. For the binding of the antibody with the lipid, the latter method, which uses the endogenous thiol group, is more preferable in terms of maintaining the protein activity. For example, an IgG antibody is digested with an enzyme such as pepsin to F(ab')$_2$, and this fragment is further reduced with dithiothreitol or the like to obtain a thiol group formed in Fab', which can in turn be used in the binding (Martin, F.J. et al., Biochemistry 20, 4229 (1981)). When IgM is used, the J chain of IgM is reduced under mild conditions according to the method of Miller et al. (J. Biol. Chem 257, 286 (1965)) to obtain a thiol group in the Fc portion, which can in turn be used in the binding.

Examples of the functional group of the lipid necessary for forming a covalent bond between the thiol group of the protein and the lipid include maleimide, vinylsulfone, and thiol groups. Examples of lipids containing a maleimide group include N-(3-(2-pyridyldithio)propionyl)phosphatidylethanolamine, N-(m-maleimidobenzoyl)dipalmitoylphosphatidylethanolamine, and N-(4-p-maleimidophenyl)butyrylphosphatidylethanolamine (MPB-PE). Examples of a maleimidating reagent include N-(ε-maleimidocaproyloxy)succinimide as well as N-succinimidyl 4-(p-maleimidophenyl)butyrate (SMPB), N-succinimidyl 4-(p-maleimidophenyl)propionate, and N-(γ-maleimidobutyryloxy)succinimide generally used for preparing maleimide derivatives of amino groups.

Moreover, the amino group of the protein may be substituted with a maleimide group using the maleimidating reagent. Such a protein forms a covalent bond with a lipid having a thiol group, for example, a (pyridyldithio)propionate (PDP)-modified lipid, through reaction.

(vi) A method which comprises mixing a lipid having an aldehyde group-reactive functional group during liposome preparation and binding a sugar chain-derived aldehyde group thereto.

Eukaryotic proteins are usually modified with a sugar chain by post-translational modification. Particularly, in antibodies, their Fc regions have a sugar chain. An aldehyde group formed by oxidizing these sugar chains forms a covalent bond with hydrazide through reaction. Therefore, by modifying a lipid with hydrazide, the protein or peptide can be immobilized via the sugar chain on the lipid (Biochimica et Biophysica Acta 1420 153-167 (1999)).

(vii) A method which comprises binding the respective functional groups of the liposome and the protein using a cross-linking agent, condensing agent, or the like.

The amino group of the lipid can be bound with the amino group of the protein to thereby immobilize the protein onto the liposome surface (Biochemical and Biophysical Research Communications, 89, 1114 (1979); and Liposome Technology, 155 (1983)). Examples of the lipid having an amino group include phosphatidylethanolamine, phosphatidylserine, and phosphatidylthreonine. The amino group of the protein is provided by a lysine residue ε-amino group and an N-terminal α-amino group. For immobilizing the protein onto the liposome comprising the lipid having an amino group, methods known in the art can be adopted, such as a method which comprises directly cross-linking the amino group of the lipid to the amino group of the protein using glutaraldehyde, and a method which comprises chemically binding the amino group of the lipid to the amino group of the protein using a reactive reagent. Examples of a divalent cross-linking agent include glutaraldehyde as well as disuccinimidyl suberate (DSS), dialdehyde (e.g., phthalaldehyde and terephthalaldehyde), dimethyl pimelimidate (DMP), and diisothiocyanostilbene disulfonic acid sodium (DIDS). Examples of the reactive reagent include N-hydroxysuccinimidyl 3-(2-pyridildithio)propionate, m-maleimidobenzoyl-N-hydroxysuccinimide ester, dithiobis(succinimidyl propionate), bis(sulfosuccinimidyl) suberate, and disuccinimidyl suberate.

The amino group of the lipid and the thiol group of the protein or the thiol group of the lipid and the amino group of the protein can be bound with each other to thereby immobilize the protein onto the liposome surface. Examples of a divalent cross-linking agent reactive for amino and thiol groups include N-succinimidyl 3-(2-pyridyldithio)propionate (SPDP), N-succinimidyl 4-(p-maleimidophenyl)butyrate (SMPB), N-succinimidyl 4-(p-maleimidophenyl)acetate (SMPA), N-succinimidyl bromoacetate, N-succinimidyl 4-(p-maleimidophenyl)propionate (SMPP), N-($\gamma$-maleimidobutyryloxy)succinimide (GMBS), and N-($\varepsilon$-maleimidocaproyloxy)succinimide (EMCS).

The carboxyl group of the lipid and the amino group of the protein or the amino group of the lipid and the carboxyl group of the protein can be bound with each other using a condensing agent to thereby immobilize the protein onto the liposome surface (Biochemistry, Vol. 31, 2850-2855 (1992)).

The thiol group of the lipid can be bound with the thiol group of the protein to thereby immobilize the protein onto the liposome surface. For example, a cross-linking agent such as bismaleimidohexane is used.

(viii) A method which comprises binding the protein with the lipid by use of the specific affinity of biotin for streptavidin or avidin.

A biotin-modified protein can be bound through a non-covalent bond to a streptavidin- or avidin-modified lipid (Antisense and Nucleic Acid Drug Development 12: 311-325 (2002)). Moreover, streptavidin or avidin form a tetramer and therefore permit binding of four biotin molecules at the maximum. Using this principle, a biotin-modified protein can be bound through a non-covalent bond to a biotin-modified lipid via an avidin or streptavidin linker (Biochimica et Biophysica Acta 1239 133-144 (1995)).

(ix) A method which comprises binding the protein with the lipid by use of the affinity of a histidine tag for nickel ions.

The protein or peptide can be immobilized onto the liposome by use of the affinity of a polyhistidine tag (His-Tag) for nickel ions ($Ni^{2+}$) (Molecular Pharmaceutics 3, 5, 525-530). A chimeric protein or peptide fused with a His-Tag by a genetic engineering approach can be expressed and purified by methods known in the art. Moreover, the lipid can be terminally modified with NTA (nitriloacetic acid), IDA (iminodiacetic acid), or the like having a metal ($Ni^{2+}$) chelating site to prepare a $Ni^{2+}$-immobilized liposome. The His-Tag is specifically bound to the $Ni^{2+}$ of the liposome such that the His-Tag-fused protein or peptide is immobilized on the liposome.

(x) A method which comprises binding the full-length antibody molecule to the liposome via a protein having affinity for an antibody Fc domain or a protein domain thereof.

The full-length antibody molecule can be supported by the liposome via a protein A or G having affinity for an antibody Fc domain or a domain protein thereof involved in Fc domain binding. In this case, the protein A or G can be bound to the liposome lipid by any of the methods (i) to (ix) (BMC Immunology 2006, 7: 24).

In the methods (i) to (x), the amount of the protein immobilized on the liposome can be changed arbitrarily by adjusting the ratio of the protein to the lipid involved in immobilization.

(3) Binding mode of hydrophilic polymer with protein

Examples of methods for immobilizing the protein or peptide or the like on the hydrophilic polymer include methods shown in (i) to (vii) below. Examples for methods binding the hydrophilic polymer or the lipid derivative of the hydrophilic polymer obtained by the method with the protein include a method which comprises forming a covalent or non-covalent bond through reaction between the functional group of the hydrophilic polymer (including a functional group artificially introduced in the hydrophilic polymer) and the functional group of the protein (including a functional group artificially introduced in the protein). Examples of combinations of the functional groups forming the covalent bond include amino/carboxyl groups, amino group/N-hydroxysuccinimide ester, amino/aldehyde groups, amino/tresyl groups, amino/nitrophenylcarbonyl groups, amino/acetal groups, amino/isothiocyanate groups, amino/acyl halide groups, amino/benzotriazole carbonate groups, hydrazide/aldehyde groups, thiol/maleimide groups, thiol/vinylsulfone groups, and thiol/thiol groups. Specific bonds such as avidin/biotin or polyhistidine tag/nickel bonds can be used for the non-covalent bond. The protein may be bound to any end or non-terminal site of the main or side chain of the hydrophilic polymer. Moreover, a plurality of protein molecules may be bound per molecule of the hydrophilic polymer.

(i) A method which comprises reacting a hydrophilic polymer having an amino group-reactive functional group with the Lys residue $\varepsilon$-amino group or N-terminal $\alpha$-amino group of the protein or peptide (Int J Oncol. 2003 23 (4): 1159-65).

The amino group of the protein is provided by a Lys residue $\varepsilon$-amino group and an N-terminal $\alpha$-amino group. Examples of the reactive functional group of the hydrophilic polymer derivative necessary for binding the amino group of the protein with the lipid include N-hydroxysuccinimide ester (NHS ester), aldehyde, tresyl, nitrophenylcarbonyl, acetal, carboxyl, isothiocyanate, acyl halide, and benzotriazole carbonate groups.

(ii) A method which comprises binding a hydrophilic polymer having a thiol group-reactive functional group to the thiol group of a protein or peptide having it (Dmitri Kirpotin et al., Biochemistry, 1997, 36, 66-75).

The thiol group is added to the protein by methods known in the art, for example, methods using compounds such as N-succinimidyl-3-(2-pyridyldithio)propionate (SPDP) (Carlsson, J. et al., Biochem. J. 173, 723, (1978)), N-succinimidyl-S-acetylthioacetate (SATA), N-succinimidyl-S-acetylthiopropionate (SATP), iminothiolane (Traut, R.R. et al., Biochemistry 12, 3266 (1973)), and mercaptoalkylimidate. In addition to these methods, the endogenous dithiol group of the protein is reduced with TCEP, DTT, mercaptoethanol, cysteine, cysteamine, or the like to a thiol group, which can in turn be used in the binding. Particularly, when the protein is an antibody, dithiol groups in the hinge region of the antibody (full-length molecule or fragment) and in the linkage between the heavy and light chains thereof can be used. For the binding of the antibody with the hydrophilic polymer, the latter method, which uses the endogenous thiol group, is more preferable in terms of maintaining the activity. For example, an IgG antibody is digested with an enzyme such as pepsin to F(ab')$_2$, and this fragment is further reduced with dithiothreitol or the like to obtain a thiol group formed in Fab', which can in turn be used in the binding (Martin, F.J. et al., Biochemistry 20, 4229 (1981)). When IgM is used, the J chain of IgM is reduced under mild conditions according to the method of Miller et al. (J. Biol. Chem 257, 286 (1965)) to obtain a thiol group in the Fc portion, which can in turn be used in the binding.

Examples of the functional group necessary for binding to the thiol group of the protein include maleimide, vinylsulfone, and thiol groups. Examples of such a hydrophilic polymer derivative include PEG-Maleimide [products of NOF CORPORATION; SUNBRIGHT MA SERIES (Maleimide-PEGs)], PEG-vinylsulfone (Bo B. Lundberg et al., Int. J. Pharm, 205, 101-108 (2000)), methoxy(hydrazide)polyethylene glycol, and bis(hydrazide)polyethylene glycol. Examples of a maleimidating reagent include N-(ε-maleimidocaproyloxy)succinimide as well as N-succinimidyl 4-(p-maleimidophenyl)butyrate (SMPB), N-succinimidyl 4-(p-maleimidophenyl)propionate, and N-(γ-maleimidobutyryloxy)succinimide generally used for preparing maleimide derivatives of amino groups.

Moreover, the amino group of the protein may be substituted with a maleimide group using the maleimidating reagent. Such a protein forms a covalent bond with a hydrophilic polymer having a thiol group, for example, a (pyridyldithio) propionate (PDP)-modified hydrophilic polymer, through reaction.

(iii) A method which comprises binding a hydrophilic polymer having an aldehyde group-reactive functional group to the sugar chain-derived aldehyde group of the protein.

Eukaryotic proteins are usually modified with a sugar chain by post-translational modification. Particularly, in antibodies, their Fc regions have a sugar chain. An aldehyde group formed by oxidizing these sugar chains forms a covalent bond with hydrazide through reaction. Therefore, by modifying a hydrophilic polymer with hydrazide, the protein or peptide can be immobilized via the sugar chain on the hydrophilic polymer (Biochimica et Biophysica Acta 1420 153-167 (1999)).

(iv) A method which comprises binding the respective functional groups of the hydrophilic polymer and the protein using a cross-linking agent, condensing agent, or the like.

The amino group of the hydrophilic polymer can be bound with the amino group of the protein to thereby immobilize the protein onto the hydrophilic polymer. Examples of the hydrophilic polymer derivative having an amino group include PEG-NH$_2$ [products of NOF CORPORATION; SUNBRIGHT PA SERIES (Amino-PEGs)]. The amino group of the protein is provided by a lysine residue ε-amino group and an N-terminal α-amino group. For immobilizing the protein onto the hydrophilic polymer having an amino group, methods known in the art can be adopted, such as a method which comprises directly cross-linking the amino group of the hydrophilic polymer to the amino group of the protein using glutaraldehyde, and a method which comprises chemically binding the amino group of the hydrophilic polymer to the amino group of the protein using a reactive reagent. Examples of a divalent cross-linking agent include glutaraldehyde as well as disuccinimidyl suberate (DSS), dialdehyde (e.g., phthalaldehyde and terephthalaldehyde), dimethyl pimelimidate (DMP), and diisothiocyanostilbene disulfonic acid sodium (DIDS). Examples of the reactive reagent include N-hydroxysuccinimidyl 3-(2-pyridildithio)propionate, m-maleimidobenzoyl-N-hydroxysuccinimide ester, dithiobis(succinimidyl propionate), bis(sulfosuccinimidyl) suberate, and disuccinimidyl suberate.

The amino group of the hydrophilic polymer and the thiol group of the protein or the thiol group of the hydrophilic polymer and the amino group of the protein can be bound with each other to thereby immobilize the protein onto the hydrophilic polymer. Examples of a divalent cross-linking agent reactive for amino and thiol groups include N-succinimidyl 3-(2-pyridyldithio)propionate (SPDP), N-succinimidyl 4-(p-maleimidophenyl)butyrate (SMPB), N-succinimidyl 4-(p-maleimidophenyl)acetate (SMPA), N-succinimidyl bromoacetate, N-succinimidyl 4-(p-maleimidophenyl)propionate (SMPP), N-(γ-maleimidobutyryloxy)succinimide (GMBS), and N-(ε-maleimidocaproyloxy)succinimide (EMCS).

The carboxyl group of the hydrophilic polymer and the amino group of the protein or the amino group of the hydrophilic polymer and the carboxyl group of the protein can be bound with each other using a condensing agent to thereby immobilize the protein onto the hydrophilic polymer (Maruyama K. et al., Biochimica et Biophysica, 1234, 74-80 (1995)).

The thiol group of the hydrophilic polymer can be bound with the thiol group of the protein to thereby immobilize the protein onto the hydrophilic polymer. For example, a cross-linking agent such as bismaleimidohexane is used.

(v) A method which comprises binding the protein with the hydrophilic polymer by use of the specific affinity of biotin for streptavidin or avidin.

A biotin-modified protein can be bound through a non-covalent bond to a streptavidin- or avidin-modified hydrophilic polymer (Antisense and Nucleic Acid Drug Development 12: 311-325 (2002)). Moreover, streptavidin or avidin form a tetramer and therefore permit binding of four biotin molecules at the maximum. Using this principle, a biotin-modified protein can be bound through a non-covalent bond to a biotin-modified hydrophilic polymer via an avidin or streptavidin linker (Biochimica et Biophysica Acta 1239 133-144 (1995)).

(vi) A method which comprises binding the protein with the hydrophilic polymer by use of the affinity of a histidine tag for nickel ions.

The protein or peptide can be immobilized onto the liposome by use of the affinity of a polyhistidine tag (His-Tag) for nickel ions ($Ni^{2+}$) (Molecular Pharmaceutics 3, 5, 525-530). A chimeric protein or peptide fused with a His-Tag by a genetic engineering approach can be expressed and purified by methods known in the art. Moreover, the hydrophilic polymer can be modified, either terminally or at the side chain, with NTA (nitriloacetic acid), IDA (iminodiacetic acid), or the like having a metal ($Ni^{2+}$) chelating site to prepare a $Ni^{2+}$-immobilized liposome. The His-Tag is specifically bound to the $Ni^{2+}$ of the liposome such that the His-Tag-fused protein or peptide is immobilized on the hydrophilic polymer.

(vii) A method which comprises binding the full-length antibody molecule to the hydrophilic polymer via a protein having affinity for an antibody Fc domain or a protein domain thereof.

The full-length antibody molecule can be supported by the hydrophilic polymer via a protein A or G having affinity for an antibody Fc domain or a domain protein thereof involved in Fc domain binding. In this case, the protein A or G can be bound to the hydrophilic polymer by any of the methods (i) to (vi) (BMC Immunology 2006, 7: 24) .

In the methods (i) to (vii), the amount of the protein immobilized on the liposome via the hydrophilic polymer can be changed arbitrarily by adjusting the content of the hydrophilic polymer reactive for the protein.

7. Form of immunoliposome

The liposome is typically a closed vesicle composed of a unilamellar or multilamellar lipid bilayer having an internal aqueous phase (D.D. Lasic, "Liposomes: From Physics to Applications", Elsevier Science Publishers, pp. 1-171 (1993)). In the present invention, the liposome refers to a lipid complex particle in a broader sense. The form of the liposome having a typical closed vesicle structure may be any of a multilamellar vesicle (MLV), a small unilamellar vesicle (SUV), and a large unilamellar vesicle (LUV). The structure of the lipid complex is not specifically defined. The particle size of the immunoliposome according to the present invention differs depending on the size adjustment method. The liposome size that can be produced is approximately 20 nm as a lower limit (D.D. Lasic, "Liposomes: From Physics to Applications", Elsevier Science Publishers, pp. 1-171 (1993)). On the other hand, the upper limit of the liposome size needs to be 7 $\mu$m or smaller, which permits intravascular administration. The particle size of the immunoliposome used in the present invention is not particularly limited and is usually of the order of 0.03 to 5 $\mu$m. For systemic administration by injection, the particle size is preferably 400 nm or smaller in consideration of the blood retention of the liposome and the accumulation thereof at disease tissues such as tumors or inflammatory sites. The constituent lipid components of the liposome and the composition ratio thereof can be selected in consideration of the physical properties of a liposome dispersion, such as pH, osmotic pressure, zeta potential, phase transition temperature, in-vivo blood retention, and stability.

8. Methods for preparing and purifying immunoliposome

(1) Method for preparing immunoliposome

The method for producing the liposome of the present invention is not particularly limited, and any of methods (D.D. Lasic, "Liposomes: From Physics to Applications", Elsevier Science Publishers, pp. 1-171 (1993)) available by those skilled in the art can be applied thereto. The liposome can be produced using the lipid by, for example, thin-film, reverse phase evaporation, ethanol injection, ether injection, dehydration-rehydration, surfactant dialysis, hydration, freeze-thaw, calcium-induced small liposome vesicle fusion, mechanochemical, hexane-span 80 dialysis, and organic solvent spherule evaporation methods. The particle size of the liposome can be adjusted by methods such as ultrasonic irradiation, post-freeze-thaw ultrasonic irradiation, extrusion, French press, and homogenization methods. Moreover, conversion from multilamellar to unilamellar liposomes or from unilamellar to multilamellar liposomes can be performed according to methods known in the art.

Moreover, a drug can be supported within the liposome according to methods known in the art. Examples of the methods generally include encapsulation, remote loading (pH gradient), counterion concentration gradient, freeze-thaw, surfactant removal, electroporation, supercritical carbon dioxide, and film loading methods (G. Gregoriadis, "Liposome Technology Liposome Preparation and Related Techniques", 2nd edition, Vol. I-III, CRC Press). The encapsulation form of the drug is not particularly limited as long as the form has a structure in which the drug is supported by the liposome. Examples thereof include a form in which the drug is enclosed in the closed space of a lipid vesicle, a form in which the drug is enclosed between lipid layers, and a form in which the drug is incorporated within a lipid layer. Alternatively, a form provided by combinations thereof may be used.

The method for preparing the immunoliposome is broadly classified into (A) a method which comprises preparing a liposome comprising a lipid or a hydrophilic polymer having a reactive functional group and reacting a protein therewith and (B) a method which comprises reacting a protein with a hydrophilic polymer having a reactive functional group and fusing the reaction product to a separately prepared liposome having no reactive functional group.

(A)

In the method for preparing the liposome, a liposome is prepared by an arbitrary method using a lipid having a reactive functional group or a lipid derivative of a hydrophilic polymer having a reactive functional group, as some constituent lipid component of the liposome. The obtained liposome is reacted with a protein as described in the paragraph 6.(2) or 6.(3) to form a covalent or non-covalent bond between the lipid molecule or the hydrophilic polymer of the liposome and the protein.

(B)

It is known that a lipid-hydrophilic polymer-protein complex is fused in a mixed dispersion with a liposome (which is not required to have a reactive functional group) under temperature conditions around or higher than the phase transition temperature of the lipid molecule, i.e., reconstitution of an amphiphilic vesicle-forming lipid occurs, to obtain an immunoliposome comprising the hydrophilic polymer-protein portion located in the aqueous phase and the lipid portion located in the lipid phase (Paul S. Uster et al., FEBS letters 386, 1996, 243-246; and T. Ishida et al., FEBS letters 460, 1999, 129-133). In this context, appropriate conditions for fusing the liposome with the lipid-hydrophilic polymer-protein complex are selected in consideration of the physical properties of the lipid, etc.

(2) Method for purifying immunoliposome

The purification of the immunoliposome means separation of liposome-unbound, unreacted proteins or peptides or the like produced during the process of the method for preparing the immunoliposome in the paragraph 8.(1), or separation of liposome-unfused hydrophilic polymer-proteins during the process of the method for preparing the immunoliposome in the paragraph 8.(1). Examples of the purification of the immunoliposome include separation by chromatography, ultrafiltration, dialysis, and ultracentrifugation.

(3) Method for measuring antibody density of immunoliposome

The "antibody density" of the immunoliposome is defined as the ratio (indicated in mol%) of the number of moles of the antibody contained in the immunoliposome to the number of moles of total constituent lipids of the immunoliposome. In this context, the total lipids mean lipids included in the amphiphilic vesicle-forming lipids listed in the paragraph 5-(1) and all lipids constituting the liposome.

Examples of methods for quantifying the lipid include a method using a radioisotope (Maehama T, et al., Anal Biochem. 279 (2): 248, 2000), a method using high-performance liquid chromatography (Serunian L.A. et al., Methods Enzymol. 1991; 198: 78), a method using gas chromatography (Hoving EB, et al., J Chromatogr B Biomed Appl. 1995, 15; 671 (1-2): 341), a method using mass spectrometry (Wenk M.R. et al., Nat Biotechnol. 2003, 21 (7): 813), absorption photometry, chemical quantification, and enzymatic quantification. Particularly, for phospholipids, examples of quantification methods include: a Bartlett method (Bartlett GR. et al., J Biol Chem. 1959, 234 (3): 469) and a Stewart method (John Charles, et al., Anal Biochem. 1980, 1; 104 (1): 10); for lipids containing choline, such as phosphatidylcholines, enzymatic quantification using choline oxidase (Takayama M, et al., Clin Chim Acta. 1977, 15; 79 (1): 93); and for cholesterols, enzymatic quantification using cholesterol oxidase (Allain CC, et al., Clin Chem. 1974, 20 (4): 470). Polyethylene glycol can be quantified by differential refractometry ("Comprehensive Polymer Science", 1st Edition, 1989), a picric acid method (Int. J. Pharm. 203, 255, 2000), or the like. The total lipid level is calculated by measuring the total lipid level or the lipid level of a particular lipid component by these methods and determining the constituent ratio (theoretical value) of the lipid to the total lipid level, with the assumption that the constituent ratio of the constituent lipids of the liposome does not vary during the immunoliposome preparation process.

The protein can be quantified by quantification methods known in the art, for example, CBQCA (You WW, et al., Anal Biochem. 15; 244 (2): 277), ultraviolet absorption, Biuret, Bradford, Kjeldahl, and Lowry methods.

When the immunoliposome contains a full-length antibody molecule, the antibody density can be selected within the range of 0.000014 mol% to 0.23 mol%, preferably 0.002 mol% to 0.14 mol%. When the immunoliposome contains antibody F(ab')$_2$, the antibody density can be selected within the range of 0.000014 mol% to 0.23 mol%, preferably 0.006 mol% to 0.11 mol%. When the immunoliposome contains Fab', the antibody density can be selected within the range of 0.000014 mol% to 0.94 mol%, preferably 0.005 mol% to 0.56 mol%. When the immunoliposome contains a single-chain variable fragment antibody, the antibody density can be selected within the range of 0.000014 mol% to 1.8 mol%, preferably 0.005 mol% to 0.56 mol%.

In this context, the minimal antibody density (0.000014 mol%) of the immunoliposome means that two molecules of each antibody (fragment) are bound per immunoliposome of 1000 nm in particle size. The maximum antibody density (full-length antibody: 0.23 mol%, F(ab')$_2$: 0.23 mol%, Fab': 0.94 mol%, scFv: 1.8 mol%) means that the antibody is present in the most dense state on the immunoliposome. Specifically, when the area occupied by the antibody on the immunoliposome is assumed as the area of a circle with the major axis of each antibody molecule as a diameter, the presence of the antibody, the number of which is a value obtained by dividing the surface area of the immunoliposome by the circle area of the antibody molecule, is defined as the most dense arrangement of the antibody (Allen et al. Biochimica et Biophysica Acta (1995)). The major axis of the antibody molecule was set to full-length antibody: 14.2 nm, F(ab')$_2$: 14.2 nm, Fab': 7 nm, and scFv: 5 nm. For the major axis of the full-length antibody, F(ab')$_2$, or Fab', values described in Raghupathy et al., The Journal of Biological Chemistry (1971) were adopted. The major axis of scFv is calculated from the crystal structure of scFv described in Hoedemaeker et al., J. Biol. Chem (1997), with reference to Raghupathy et al., The Journal of Biological Chemistry (1971), and 5 nm was adopted.

9. Pharmaceutical agent comprising immunoliposome

The immunoliposome obtained by the methods described in the paragraph "8. Methods for preparing and purifying immunoliposome" induces the apoptosis of cancer cells and inhibits the growth of the cancer cells, via the biological activity of an apoptosis-related receptor such as DR5 or Fas, i.e., via the receptor, *in vivo*. Therefore, the immunoliposome can be used as a pharmaceutical agent, particularly, a therapeutic agent for cancer.

The in-vitro antitumor activity of the immunoliposome can be measured based on a cell growth inhibitory activity against cells overexpressing the apoptosis-related receptor.

For example, a DR5-overexpressing cancer cell line is cultured, and varying concentrations of the immunoliposome are added to the culture system. The inhibitory activity against focus formation, colony formation, and spheroid growth can be measured.

The *in-vivo* therapeutic effect of the immunoliposome on cancer using experimental animals can be measured by administering the immunoliposome to, for example, DR5-overexpressing tumor cell line-transplanted nude mice, and measuring the change in the cancer cells.

Examples of cancer types can include lung cancer, prostatic cancer, liver cancer, ovarian cancer, colon cancer, breast cancer, pancreatic cancer, and blood cell cancer (leukemia, lymphoma, etc.). The cancer cells to be treated are not limited thereto as long as they express the death domain-containing receptor.

It is also known that antibodies against Fas and DR5 induce the apoptosis of inflammatory cells (J. Clin. Invest. 1996, 98 (2), 271-278; and Int. Immunol. 1996, 8 (10), 1595-1602). Thus, the immunoliposome of the present invention can also be used as a therapeutic agent for autoimmune disease or inflammatory disease. Examples of the autoimmune disease or inflammatory disease include systemic lupus erythematosus, Hashimoto's disease, articular rheumatism, graft-versus-host disease, Sjogren syndrome, pernicious anemia, Addison's disease, scleroderma, Goodpasture syndrome, Crohn disease, autoimmune hemolytic anemia, impotentia generandi, myasthenia gravis, multiple sclerosis, Basedow disease, thrombocytopenic purpura, insulin-dependent diabetes mellitus, allergy, asthma, atopy, arteriosclerosis, myocarditis, myocardosis, glomerulonephritis, aplastic anemia, and organ transplant rejection.

The present invention also provides a pharmaceutical composition comprising a therapeutically effective amount of the immunoliposome and a pharmaceutically acceptable diluent, carrier, solubilizing agent, emulsifying agent, preservative, and/or adjuvant.

It is preferred that the substances pharmaceutically used that are acceptable in the pharmaceutical composition of the present invention should be nontoxic, at the dose or administration concentration used, to individuals that receive the pharmaceutical composition.

The pharmaceutical composition of the present invention can contain a pharmaceutical substance for changing, maintaining, or retaining pH, osmotic pressure, viscosity, transparency, color, isotonicity, sterility, stability, the rate of dissolution, the rate of sustained release, absorptivity, or permeability.

Examples of the pharmaceutical substance can include, but not limited to, the following: amino acids such as glycine, alanine, glutamine, asparagine, arginine, and lysine; antimicrobial agents; antioxidants such as ascorbic acid, sodium sulfate, and sodium bisulfite; buffers such as phosphate, citrate, and borate buffers, hydrogen carbonate, and Tris-HCl solutions; fillers such as mannitol and glycine; chelating agents such as ethylenediaminetetraacetic acid (EDTA); complexing agents such as caffeine, polyvinyl pyrrolidine, β-cyclodextrin, and hydroxypropyl-β-cyclodextrin; extenders such as glucose, mannose, and dextrin; monosaccharides, disaccharides, glucose, mannose, and other hydrocarbons such as dextrin; coloring agents; flavoring agents; emulsifying agents; hydrophilic polymers such as polyvinyl pyrrolidine; low-molecular-weight polypeptides; salt-forming counterions; antiseptics such as benzalkonium chloride, benzoic acid, salicylic acid, thimerosal, phenethyl alcohol, methylparaben, propylparaben, chlorhexidine, sorbic acid, and hydrogen peroxide; solvents such as glycerin, propylene glycol, and polyethylene glycol; sugar alcohols such as mannitol and sorbitol; suspending agents; surfactants such as PEG, sorbitan ester, polysorbates such as polysorbate 20 and polysorbate 80, Triton, tromethamine, lecithin, and cholesterol; stability enhancers such as sucrose and sorbitol; elasticity enhancers such as sodium chloride, potassium chloride, mannitol, and sorbitol; delivery vehicles; diluents; excipients; and/or pharmaceutical adjuvants.

The amounts of these pharmaceutical substances added are preferably 0.01 to 100 times, particularly, 0.1 to 10 times higher than the weight of the immunoliposome.

The preferable composition of the pharmaceutical composition in a preparation can be determined appropriately by those skilled in the art according to applicable disease, an applicable administration route, etc.

The excipients or carriers in the pharmaceutical composition may be liquid or solid.

The appropriate excipients or carriers may be injectable water, saline, cerebrospinal fluids, or other substances usually used in parenteral administration.

Neutral saline or serum albumin-containing saline can also be used as a carrier.

The pharmaceutical composition can also contain a Tris buffer (pH 7.0 to 8.5) or an acetate buffer (pH 4.0 to 5.5) as well as sorbitol or other compounds.

The pharmaceutical composition of the present invention is prepared in a freeze-dried or liquid form as an appropriate drug having the selected composition and necessary purity.

The pharmaceutical composition comprising the immunoliposome can also be prepared in a freeze-dried form using an appropriate excipient such as sucrose.

The pharmaceutical composition of the present invention can be prepared for parenteral administration or can also be prepared for gastrointestinal absorption through an oral route.

The composition and concentration of the preparation can be determined depending on an administration method. When the antibody contained in the pharmaceutical composition of the present invention has higher affinity for the antigen, i.e., higher affinity (lower Kd value) for the antigen with respect to a dissociation constant (Kd value), the pharmaceutical composition can exert its pharmacological effect at a lower dose in humans. Based on this result, the dose of the pharmaceutical composition of the present invention in humans can also be determined.

The dose of the immunoliposome in humans may be approximately 0.1 to 100 mg/kg in terms of the amount of the antibody administered once per 1 to 30 days.

Examples of dosage forms of the pharmaceutical composition of the present invention include injections including drip, suppositories, nasal agents, sublingual agents, and transdermally absorbable agents.

Hereinafter, the present invention will be described specifically with reference to the Examples. However, the present invention is not intended to be limited to these Examples. In the Examples below, procedures related to genetic engineering were performed according to methods described in "Molecular Cloning", (Sambrook, J., Fritsch, E.F., and Maniatis, T., published by Cold Spring Harbor Laboratory Press, 1989) or according to instructions included in commercially available reagents or kits used, unless otherwise specified.

The concentration of a protein conjugated to an immunoliposome was quantified using CBQCA Protein Quantitation Kit (Molecular Probes) according to the instructions included therein. The phospholipid concentration of a liposome was quantified using Phospholipid C Test Wako (Wako Pure Chemical Industries, Ltd.) according to the instructions included therein.

The particle size of an immunoliposome was measured using a particle size measurement apparatus (Nicomp Particle Sizer Model 370, Nicomp Particle Sizing Systems).

Reference Example 1

Preparation of hTRA-8 F(ab')$_2$

The concentration of an anti-human DR5 antibody hTRA-8 was adjusted to 10 mg/ml with an acetate buffer (20 mM sodium acetate, pH 4.5). In this context, the hTRA-8 is an antibody obtained by humanizing a mouse anti-human DR5 antibody TRA-8 (Nature Med. 2001, 7 (8), 954-60) and has the amino acid sequence of SEQ ID NO: 1 described in the sequence listing as the heavy chain amino acid sequence and the amino acid sequence of SEQ ID NO: 2 described in the sequence listing as the light chain amino acid sequence. An amino acid sequence consisting of amino acid residues 1 to 118 of the amino acid sequence of SEQ ID NO: 1 described in the sequence listing corresponds to the heavy chain variable region of hTRA-8, and an amino acid sequence consisting of amino acid residues 1 to 107 of the amino acid sequence of SEQ ID NO: 2 described in the sequence listing corresponds to the light chain variable region of hTRA-8.

To 1 ml of the present antibody solution, 125 μl of Immobilized pepsin (Pierce Biotechnology, Inc.) was added, and the mixture was then incubated at 37°C for 8.5 hr to degrade the present antibody to F(ab')$_2$ fragments. The reaction solution was spinned down, and the supernatant was filtered to remove the Immobilized pepsin. Peptide fragments and undigested full-length hTRA-8 were removed by ion-exchange chromatography (AKTA explorer 10S (GE HEALTHCARE INC.);

column (GE HEALTHCARE INC., Resource S 6 ml); solution A (50 mM citrate buffer, pH 4.0), solution B (50 mM citrate buffer, 1 M NaCl, pH 4.0); Gradient (solution B: 15→40%, 50 CV, linear gradient); 4°C; 6 ml/min; detection wavelength: UV 280 nm) to collect F(ab')$_2$ fractions (57-111 ml elution fractions). The citrate buffer was replaced with a HEPES buffer (20 mM HEPES, 150 mM NaCl, pH 7.4) by ultrafiltration procedures using Labscale TFF system (MILLIPORE INC.) and a polyethersulfone membrane (MILLIPORE INC., Pellicon XL, Biomax 50 (molecular cutoff: 50,000)).

Reference Example 2

Preparation of hHFE7A F(ab')$_2$

The concentration of an anti-human Fas antibody hHFE7A was adjusted to 10 mg/ml with an acetate buffer (20 mM sodium acetate, pH 4.5). In this context, the hHFE7A is an antibody obtained by humanizing a mouse anti-human Fas antibody HFE7A (Int. Immunol. 2000, 12 (4), 555-62) and has the amino acid sequence of SEQ ID NO: 3 described in the sequence listing as the heavy chain amino acid sequence and the amino acid sequence of SEQ ID NO: 4 described in the sequence listing as the light chain amino acid sequence. An amino acid sequence consisting of amino acid residues 1 to 139 of the amino acid sequence of SEQ ID NO: 3 described in the sequence listing corresponds to the heavy chain variable region of hHFE7A, and an amino acid sequence consisting of amino acid residues 1 to 131 of the amino acid sequence of SEQ ID NO: 4 described in the sequence listing corresponds to the light chain variable region of hHFE7A. To 1 ml of the present antibody solution, 125 μl of Immobilized pepsin (Pierce Biotechnology, Inc.) was added, and the mixture was then incubated at 37°C for 8.5 hr to degrade the present antibody to F(ab')$_2$ fragments. The reaction solution was spinned down, and the supernatant was filtered to remove the Immobilized pepsin. Peptide fragments and undigested full-length hHFE7A were removed by ion-exchange chromatography (AKTA explorer 10S (GE HEALTHCARE INC.); column (GE HEALTHCARE INC., Resource S 6 ml); solution A (50 mM citrate buffer, pH 4.0), solution B (50 mM citrate buffer, 1 M NaCl, pH 4.0); Gradient (solution B: 15→40%, 50 CV, linear gradient); 4°C; 6 ml/min; detection wavelength: UV 280 nm) to collect F(ab')$_2$ fractions (40-65 ml fractions). The citrate buffer was replaced with a HEPES buffer (20 mM HEPES, 150 mM NaCl, pH 7.4) by ultrafiltration procedures using Labscale TFF system (MILLIPORE INC.) and a polyethersulfone membrane (MILLIPORE INC., Pellicon XL, Biomax 50 (molecular cutoff: 50,000)).

Reference Example 3

Preparation of hTRA-8 scFv

(1) Preparation of hTRA-8 scFv expression vector

DNA encoding the first half of hTRA-8 scFv was amplified by PCR using vectors TRA-8-H pHA15-1 encoding a hTRA-8 H chain region as templates for VH and two oligonucleotide primers.
Likewise, DNA encoding the latter half of hTRA-8 scFv was amplified by PCR using vectors TRA-8-L LM-1L encoding a hTRA-8 L chain region as templates for VL and two oligonucleotide primers.
In this procedure, primers were designed to add a flexible peptide linker (GGGGS)x3 between VH and VL and a sequence HHHHHHGGAC (sequence containing a six-histidine residue tag and a Cys residue) to the C terminus. Then, to these DNA fragments, 5' NdeI (TAKARA BIO INC.) and 3' XhoI (TAKARA BIO INC.) sites were inserted, and the resulting fragments were ligated by PCR to prepare hTRA-8 scFv-encoding DNA.
The prepared DNA was digested with restriction enzymes NdeI and XhoI and then inserted into the restriction sites NdeI/XhoI present in the multicloning site of pET22b(+) (Novagen) to construct pET22b(+)hTRA-8 scFv expression vectors.

(2) hTRA-8 scFv expression

The pET22b(+)hTRA-8 scFv expression vectors obtained in the paragraph (1) were introduced into an Escherichia coli strain OrigamiB (DE3) (Novagen) according to a method known in the art, and the bacterial cells were applied to an LB plate containing 100 μg/ml ampicillin (Sigma-Aldrich, Inc.), 25 μg/ml kanamycin (WAKO Pure Chemical Industries, Ltd.), and 34 μg/ml chloramphenicol (WAKO Pure Chemical Industries, Ltd.) to obtain transformants. Next, colonies of these transformants were grown overnight at 37°C with shaking in 20 ml of an LB medium (Invitrogen Corp.) until nearly saturated, and these colonies were inoculated onto 2 L of a fresh LB medium. The bacteria were grown by aerobic culture at 37°C in a 2-1 shaking flask until OD 600 nm=0.8. The temperature was dropped to 16°C, and protein expression was then induced using 0.5 mM isopropyl-β-D-thiogalactopyranoside (IPTG) (WAKO Pure Chemical Industries, Ltd.). The bacterial culture was continued at 16°C for 18 hours under aerobic culture.

(3) Protein purification

The cells were centrifuged at 6000 g for 20 minutes to form pellets. Then, the pellets were resuspended in a buffer containing 20 mM Tris-HCl (WAKO Pure Chemical Industries, Ltd.) (pH 8.0), 100 mM NaCl (MANAC Incorporated), 4 mM CHAPS (Sigma-Aldrich, Inc.), 10% glycerol (WAKO Pure Chemical Industries, Ltd.) (v/v), and Protease Inhibitor Cocktail Complete mini (EDTA free) (Roche Diagnostics GmbH), and the resuspension was stored at -80°C. The resuspension was thawed with running water and ultrasonically homogenized on ice, and cell debris was removed by centrifugation at 24,000 g at 4°C for 20 minutes.

The centrifugation supernatant was passed through a 0.45 $\mu$l filter, followed by purification with a His tag affinity column. Specifically, after sample application to a column His-Trap HP 1 ml (GE Healthcare Inc.) in AKTA Prime system (GE Healthcare Inc.), the column was washed with 5 column volumes of 20 mM Tris-HCl (pH 7.5), 500 mM NaCl, 40 mM imidazole (CALBIOCHEM), 0.6% CHAPS, and 1 mM DTT (Sigma-Aldrich, Inc.) at a flow rate of 1 ml/min. Next, gradient elution was performed using 20 mM Tris-HCl (pH 7.5), 500 mM NaCl, 0.6% CHAPS, 1 mM DTT, and 500 mM imidazole.

Each fraction eluted from His-Trap HP was analyzed by SDS-PAGE, and fractions containing scFv were further subjected to purification by gel filtration chromatography. Specifically, the sample was added to a column Superdex 75 pg HiLoad 16/60 (GE Healthcare Inc.) using 50 mM Tris-HCl (pH 7.5), 500 mM NaCl, 10% glycerol, 10 mM CHAPS, and 1 mM DTT. Each fraction eluted from Superdex 75 pg HiLoad 16/60 was separated and analyzed by SDS-PAGE, and scFv fractions were collected.

Example 1

(1) Preparation of hTRA-8 Fab'

The F(ab')$_2$ solution (antibody concentration: 2.5 mg/ml, HEPES buffer) prepared in Reference Example 1 was incubated at room temperature for 90 minutes in the presence of 10 mM L-cysteine (Wako Pure Chemical Industries, Ltd.) for reduction to Fab'. The L-cysteine was removed by gel filtration purification (column: GE HEALTHCARE INC., PD-10 Desalting column; HEPES buffer) to obtain a hTRA-8 Fab' fragment.

(2) Preparation of liposome

22 mg, 7.73 mg, and 1.26 mg (molar ratio 100:66:1) of L-$\alpha$-dipalmitoylphosphatidylcholine (hereinafter, referred to as DPPC; NOF CORPORATION, COATSOME MC-6060), cholesterol (Sigma-Aldrich, Inc.), and poly(ethylene glycol) succinyl distearoylphosphatidylethanolamine having a maleimide group at the end of polyethylene glycol of approximately 3400 in molecular weight (hereinafter, referred to as DSPE-PEG3400-Mal; NOF CORPORATION, SUNBRIGHT DSPE-034MA) were weighed, respectively, in an eggplant-shaped flask, to which 3 ml of chloroform (KANTO CHEMICAL CO., INC.) was added for dissolution. Next, the chloroform was distilled off under reduced pressure to thereby form a thin layer of lipids on the interior wall of the flask. To this thin layer of lipids, 3 ml of a HEPES buffer (20 mM HEPES, 150 mM NaCl, pH 7.4) was added for suspension to obtain a crude liposome (DPPC concentration: 10 mM) dispersion. Subsequently, this liposome dispersion was repeatedly extruded from a polycarbonate membrane (Avanti POLAR LIPID, INC.) having a pore size of 100 nm using an extruder (The Mini-Extruder, Avanti POLAR LIPID, INC.) to produce liposomes with an appropriate particle size.

(3) Binding reaction of antibody with liposome

The hTRA-8 Fab' (3.6 mg/ml, 15.3 $\mu$l) and the liposome dispersion (10 mM DPPC, 500 $\mu$l) were mixed at a ratio of hTRA-8 Fab':DSPE-PEG3400-Mal=1:50 (molar ratio) to react the thiol group of the antibody with the terminal maleimide group of the PEG chain on the liposome. Antibody-unbound maleimide groups were inactivated by the addition of 5 $\mu$l of 100 mM mercaptoethanol (Wako Pure Chemical Industries, Ltd.) which was 10 equivalents with respect to DSPE-PEG3400-Mal and subsequent reaction at room temperature for 30 minutes. Unreacted hTRA-8 Fab' was removed by gel filtration chromatography (AKTA explorer 10S (GE HEALTHCARE INC.); column (GE HEALTHCARE INC., HiLoad Superdex 200 16/60 prep grade); HEPES buffer (pH 7.4); 4°C; 2 ml/min; detection wavelength: UV 280 nm) to separate immunoliposome fractions (36-48 ml fractions). Ultrafiltration concentration was performed using Amicon Ultra (MILLIPORE INC., molecular cutoff: 50,000) to obtain the present immunoliposome in which the cysteine residue on the antibody was bound with the end of PEG on the liposome (liposome composition: No. 1 of Table 1, antibody concentration: 33.5 $\mu$g/ml, phospholipid concentration: 6.3 mM, antibody density: 0.0058 mol%, average particle size: 73.0$\pm$35.9 nm (HEPES buffer)).

Example 2

A hTRA-8 Fab' fragment was prepared just before use in the same way as in paragraph (1) of Example 1. Furthermore, a liposome dispersion was prepared just before use in the same way as in paragraph (2) of Example 1.

The antibody was bound to the liposome as follows: the hTRA-8 Fab' (3.6 mg/ml, 152 $\mu$l) and the liposome dispersion (10 mM DPPC, 500 $\mu$l) were mixed at a ratio of hTRA-8 Fab':DSPE-PEG3400-Mal=1:5 (molar ratio) to react the thiol group of the antibody with the terminal maleimide group of the PEG chain on the liposome. Antibody-unbound maleimide groups were inactivated by the addition of 5 $\mu$l of 100 mM mercaptoethanol (Wako Pure Chemical Industries, Ltd.) which was 10 equivalents with respect to DSPE-PEG3400-Mal and subsequent reaction at room temperature for 30 minutes. Unreacted hTRA-8 Fab' was removed by gel filtration chromatography (AKTA explorer 10S (GE HEALTHCARE INC.); column (GE HEALTHCARE INC., HiLoad Superdex 200 16/60 prep grade); HEPES buffer (pH 7.4); 4°C; 2 ml/min; detection wavelength: UV 280 nm) to separate immunoliposome fractions (36-48 ml fractions). Ultrafiltration concentration was performed using Amicon Ultra (MILLIPORE INC., molecular cutoff: 50,000) to obtain the present immunoliposome in which the cysteine residue on the antibody was bound with the end of PEG on the liposome (liposome composition: No. 2 of Table 1, antibody concentration: 278.5 $\mu$g/ml, phospholipid concentration: 6.1 mM, antibody density: 0.050 mol%, average particle size: 86.6$\pm$39.9 nm (HEPES buffer)).

Example 3

A hTRA-8 Fab' fragment was prepared just before use in the same way as in paragraph (1) of Example 1. Furthermore, a liposome dispersion was prepared just before use in the same way as in paragraph (2) of Example 1.

The antibody was bound to the liposome by the following steps: the hTRA-8 Fab' (3.6 mg/ml, 244 $\mu$l) and the liposome dispersion (10 mM DPPC, 320 $\mu$l) were mixed at a ratio of hTRA-8 Fab':DSPE-PEG3400-Mal=1:2 (molar ratio) to react the thiol group of the antibody with the terminal maleimide group of the PEG chain on the liposome. Antibody-unbound maleimide groups were inactivated by the addition of 3.2 $\mu$l of 100 mM mercaptoethanol (Wako Pure Chemical Industries, Ltd.) which was 10 equivalents with respect to DSPE-PEG3400-Mal and subsequent reaction at room temperature for 30 minutes. Unreacted hTRA-8 Fab' was removed by gel filtration chromatography (AKTA explorer 10S (GE HEALTHCARE INC.); column (GE HEALTHCARE INC., HiLoad Superdex 200 16/60 prep grade); HEPES buffer (pH 7.4); 4°C; 2 ml/min; detection wavelength: UV 280 nm) to separate immunoliposome fractions (36-48 ml fractions). Ultrafiltration concentration was performed using Amicon Ultra (MILLIPORE INC., molecular cutoff: 50,000) to obtain the present immunoliposome in which the cysteine residue on the antibody was bound with the end of PEG on the liposome (liposome composition: No. 3 of Table 1, antibody concentration: 413.7 $\mu$g/ml, phospholipid concentration: 4.38 mM, antibody density: 0.102 mol%, average particle size: 84.4$\pm$42.2 nm (HEPES buffer)).

Example 4

(1) Preparation of hTRA-8 Fab'

A hTRA-8 Fab' fragment was prepared just before use in the same way as in paragraph (1) of Example 1.

(2) Preparation of liposome

11 mg, 3.9 mg, and 3.15 mg (molar ratio 100:66:5) of L-$\alpha$-dipalmitoylphosphatidylcholine (hereinafter, referred to as DPPC; NOF CORPORATION, COATSOME MC-6060), cholesterol (Sigma-Aldrich, Inc.), and poly(ethylene glycol) succinyl distearoylphosphatidylethanolamine having a maleimide group at the end of polyethylene glycol of approximately 3400 in molecular weight (hereinafter, referred to as DSPE-PEG3400-Mal; NOF CORPORATION, SUNBRIGHT DSPE-034MA) were weighed, respectively, in an eggplant-shaped flask, to which 1.5 ml of chloroform (KANTO CHEMICAL CO., INC.) was added for dissolution. Next, the chloroform was distilled off under reduced pressure to thereby form a thin layer of lipids on the interior wall of the flask. To this thin layer of lipids, 1.5 ml of a HEPES buffer (20 mM HEPES, 150 mM NaCl, pH 7.4) was added for suspension to obtain a crude liposome (DPPC concentration: 10 mM) dispersion. Subsequently, this liposome dispersion was repeatedly extruded from a polycarbonate membrane (Avanti POLAR LIPID, INC.) having a pore size of 100 nm using an extruder (The Mini-Extruder, Avanti POLAR LIPID, INC.) to produce liposomes with an appropriate particle size.

(3) Binding reaction of antibody with liposome

The antibody was bound to the liposome by the following steps: the hTRA-8 Fab' (4.64 mg/ml, 237 $\mu$l) and the liposome dispersion (10 mM DPPC, 200 $\mu$l) were mixed at a ratio of hTRA-8 Fab':DSPE-PEG3400-Mal=1:1 (molar ratio) to react the thiol group of the antibody with the terminal maleimide group of the PEG chain on the liposome. Antibody-unbound maleimide groups were inactivated by the addition of 2 μl of 100 mM mercaptoethanol (Wako Pure Chemical Industries, Ltd.) which was 10 equivalents with respect to DSPE-PEG3400-Mal and subsequent reaction at room temperature for 30 minutes. Unreacted hTRA-8 Fab' was removed by gel filtration chromatography (AKTA explorer 10S (GE HEALTHCARE INC.); column (GE HEALTHCARE INC., HiLoad Superdex 200 16/60 prep grade); HEPES buffer (pH 7.4); 4°C; 2 ml/min; detection wavelength: UV 280 nm) to separate immunoliposome fractions (36-48 ml fractions). Ultrafiltration concentration was performed using Amicon Ultra (MILLIPORE INC., molecular cutoff: 50,000) to obtain the present immunoliposome in which the cysteine residue on the antibody was bound with the end of PEG on the liposome (liposome composition: No. 4 of Table 1, antibody concentration: 394.5 μg/ml, phospholipid concentration: 1.91 mM, antibody density: 0.224 mol%, average particle size: 83.7±40.8 nm (HEPES buffer)).

Example 5

(1) Preparation of hTRA-8 Fab'

The F(ab')$_2$ solution (antibody concentration: 5 mg/ml, HEPES buffer) prepared in Reference Example 1 was incubated at room temperature for 30 minutes in the presence of 40 mM (±)-dithiothreitol (hereinafter, referred to as DTT; Wako Pure Chemical Industries, Ltd.) for reduction to Fab'. The DTT was removed by gel filtration purification (column: GE HEALTHCARE INC., PD-10 Desalting column; HEPES buffer) to obtain a hTRA-8 Fab' fragment.

(2) Preparation of liposome

90 ml of ethanol (Wako Pure Chemical Industries, Ltd.) was added, for dissolution, to 1762 mg, 618.6 mg, and 2481.4 mg (molar ratio 100:66:1) of L-α-dipalmitoylphosphatidylcholine (hereinafter, referred to as DPPC; NOF CORPORATION, COATSOME MC-6060), cholesterol (Sigma-Aldrich, Inc.), and poly(ethylene glycol)succinyl distearoylphosphatidylethanolamine having a maleimide group at the end of polyethylene glycol of approximately 3400 in molecular weight(hereinafter, referred to as DSPE-PEG3400-Mal; NOF CORPORATION, SUNBRIGHT DSPE-034MA), respectively. This lipid solution was added dropwise to 900 ml of a HEPES buffer (20 mM HEPES, 150 mM NaCl, pH 7.4) to obtain a crude liposome dispersion. Subsequently, the concentration of the liposome dispersion was adjusted to 10 mM DPPC by ultrafiltration (LabScale TFF System (MILLIPORE INC.)) through a polyethersulfone membrane (Pellicon XL 50, Biomax 300, molecular cutoff: 300,000, (MILLIPORE INC.)). The liposome dispersion was repeatedly extruded from a polycarbonate membrane (Nucleopore Track-Etch Membrane, Whatman INC.) having a pore size of 50 nm using a continuous, high-pressure homogenizer (EmulsiFlex-C5, AVESTIN, INC.) to produce liposomes with an appropriate particle size.

(3) Binding reaction of antibody with liposome

The hTRA-8 Fab' (4.86 mg/ml, 5.7 ml) and the liposome dispersion (10 mM DPPC, 132 ml) were mixed at a ratio of hTRA-8 Fab':DSPE-PEG3400-Mal=1:25 (molar ratio) to react the thiol group of the antibody with the terminal maleimide group of the PEG chain on the liposome. Antibody-unbound maleimide groups were inactivated by the addition of 1.32 ml of 100 mM mercaptoethanol (Wako Pure Chemical Industries, Ltd.) which was 10 equivalents with respect to DSPE-PEG3400-Mal and subsequent reaction at room temperature for 30 minutes. Unreacted hTRA-8 Fab' was removed by ultrafiltration (LabScale TFF System (MILLIPORE INC.) through a polyethersulfone membrane (Pellicon XL 50, Biomax 300, molecular cutoff: 300,000, (MILLIPORE INC.)) to obtain the present immunoliposome in which the cysteine residue on the antibody was bound with the end of PEG on the liposome (liposome composition: No. 5 of Table 1, antibody concentration: 1011.0 μg/ml, phospholipid concentration: 27.63 mM, antibody density: 0.040 mol%, average particle size: 53.7±22.4 nm (HEPES buffer)) .

Example 6

A hTRA-8 Fab' fragment was prepared just before use in the same way as in paragraph (1) of Example 5. Furthermore, a liposome dispersion was prepared just before use in the same way as in paragraph (2) of Example 5.
The binding reaction of the antibody with the liposome was performed by the following steps: the hTRA-8 Fab' (4.86 mg/ml, 9.05 ml) and the liposome dispersion (10 mM DPPC, 49 ml) were mixed at a ratio of hTRA-8 Fab':DSPE-PEG3400-Mal=1:5 (molar ratio) to react the thiol group of the antibody with the terminal maleimide group of the PEG chain on the liposome. Antibody-unbound maleimide groups were inactivated by the addition of 490 μl of 100 mM mercaptoethanol (Wako Pure Chemical Industries, Ltd.) which was 10 equivalents with respect to DSPE-PEG3400-Mal and subsequent reaction at room temperature for 30 minutes. Unreacted hTRA-8 Fab' was removed by ultrafiltration (LabScale TFF System (MILLIPORE INC.) through a polyethersulfone membrane (Pellicon XL 50, Biomax 300, molecular cutoff: 300,000, (MILLIPORE INC.)) to obtain the present immunoliposome in which the cysteine residue on the antibody was bound with the end of PEG on the liposome (liposome composition: No. 6 of Table 1, antibody concentration: 1363.3 μg/ml, phospholipid concentration: 8.85 mM, antibody density: 0.167 mol%, average particle size: 50.3±23.6 nm (HEPES buffer)).

Example 7

A hTRA-8 Fab' fragment was prepared just before use in the same way as in the paragraph (1) of Example 1. Furthermore, a liposome dispersion was prepared just before use in the same way as in the paragraph (2) of Example 1. The antibody was bound to the liposome by the following steps: the hTRA-8 Fab' (5 mg/ml, 66 μl) and the liposome dispersion (10 mM DPPC, 300 μl) were mixed at a ratio of hTRA-8 Fab':DSPE-PEG3400-Mal=1:5 (molar ratio) to react the thiol group of the antibody with the terminal maleimide group of the PEG chain on the liposome. Antibody-unbound maleimide groups were inactivated by the addition of 3 μl of 100 mM mercaptoethanol (Wako Pure Chemical Industries, Ltd.) which was 10 equivalents with respect to DSPE-PEG3400-Mal and subsequent reaction at room temperature for 30 minutes. Unreacted hTRA-8 Fab' was removed by gel filtration chromatography (AKTA explorer 10S (GE HEALTHCARE INC.); column (GE HEALTHCARE INC., HiLoad Superdex 200 16/60 prep grade); HEPES buffer (pH 7.4); 4°C; 2 ml/min; detection wavelength: UV 280 nm) to separate immunoliposome fractions (36-48 ml fractions). Ultrafiltration concentration was performed using Amicon Ultra (MILLIPORE INC., molecular cutoff: 50,000) to obtain the present immunoliposome in which the cysteine residue on the antibody was bound with the end of PEG on the liposome (liposome composition: No. 7 of Table 1, antibody concentration: 96.8 μg/ml, phospholipid concentration: 1.64 mM, antibody density: 0.064 mol%, average particle size: 101.1±44.2 nm (HEPES buffer)).

Example 8

A hTRA-8 Fab' fragment was prepared just before use in the same way as in paragraph (1) of Example 1. Furthermore, a liposome dispersion was prepared just before use in the same way as in paragraph (2) of Example 1.
The antibody was bound to the liposome by the following steps: the hTRA-8 Fab' (5 mg/ml, 16.5 μl) and the liposome dispersion (10 mM DPPC, 300 μl) were mixed at a ratio of hTRA-8 Fab':DSPE-PEG3400-Mal=1:20 (molar ratio) to react the thiol group of the antibody with the terminal maleimide group of the PEG chain on the liposome. Antibody-unbound maleimide groups were inactivated by the addition of 3 μl of 100 mM mercaptoethanol (Wako Pure Chemical Industries, Ltd.) which was 10 equivalents with respect to DSPE-PEG3400-Mal and subsequent reaction at room temperature for 30 minutes. Unreacted hTRA-8 Fab' was removed by gel filtration chromatography (AKTA explorer 10S (GE HEALTHCARE INC.); column (GE HEALTHCARE INC., HiLoad Superdex 200 16/60 prep grade); HEPES buffer (pH 7.4); 4°C; 2 ml/min; detection wavelength: UV 280 nm) to separate immunoliposome fractions (36-48 ml fractions). Ultrafiltration concentration was performed using Amicon Ultra (MILLIPORE INC., molecular cutoff: 50,000) to obtain the present immunoliposome in which the cysteine residue on the antibody was bound with the end of PEG on the liposome (liposome composition: No. 8 of Table 1, antibody concentration: 70.7 μg/ml, phospholipid concentration: 1.39 mM, antibody density: 0.055 mol%, average particle size: 83.3±40.3 nm (HEPES buffer)).

Example 9

(1) Preparation of hTRA-8 Fab'

A hTRA-8 Fab' fragment was prepared just before use in the same way as in paragraph (1) of Example 1.

(2) Preparation of liposome

24 mg, 7.73 mg, and 1.26 mg (molar ratio 100:66:1) of egg yolk lecithin (hereinafter, referred to as eggPC; Q.P. Corporation, PC-98N), cholesterol (Sigma-Aldrich, Inc.), and poly(ethylene glycol)succinyl distearoylphosphatidylethanolamine having a maleimide group at the end of polyethylene glycol of approximately 3400 in molecular weight (hereinafter, referred to as DSPE-PEG3400-Mal; NOF CORPORATION, SUNBRIGHT DSPE-034MA) were weighed, respectively, in an eggplant-shaped flask, to which 3 ml of chloroform (KANTO CHEMICAL CO., INC.) was added for dissolution. Next, the chloroform was distilled off under reduced pressure to thereby form a thin layer of lipids on the interior wall of the flask. To this thin layer of lipids, 3 ml of a HEPES buffer was added to obtain a crude liposome (eggPC concentration: 10 mM) dispersion. Subsequently, this liposome dispersion was repeatedly extruded from a polycarbonate membrane (Avanti POLAR LIPID, INC.) having a pore size of 100 nm using an extruder (The Mini-Extruder, Avanti POLAR LIPID, INC.) to produce liposomes with an appropriate particle size.

(3) Binding of antibody to liposome

The hTRA-8 Fab' (5 mg/ml, 66 μl) and the liposome dispersion (10 mM eggPC, 300 μl) were mixed at a ratio of hTRA-8 Fab':DSPE-PEG3400-Mal=1:5 (molar ratio) to react the thiol group of the antibody with the terminal maleimide group of the PEG chain on the liposome. Antibody-unbound maleimide groups were inactivated by the addition of 3 μl of 100 mM mercaptoethanol (Wako Pure Chemical Industries, Ltd.) which was 10 equivalents with respect to DSPE-PEG3400-Mal and subsequent reaction at room temperature for 30 minutes. Unreacted hTRA-8 Fab' was removed by gel filtration chromatography (AKTA explorer 10S (GE HEALTHCARE INC.); column (GE HEALTHCARE INC., HiLoad Superdex 200 16/60 prep grade); HEPES buffer (pH 7.4); 4°C; 2 ml/min; detection wavelength: UV 280 nm) to separate immunoliposome fractions (36-48 ml fractions). Ultrafiltration concentration was performed using Amicon Ultra (MILLIPORE INC., molecular cutoff: 50,000) to obtain the present immunoliposome in which the cysteine residue on the antibody was bound with the end of PEG on the liposome (liposome composition: No. 9 of Table 1, antibody concentration: 138.8 μg/ml, phospholipid concentration: 2.32 mM, antibody density: 0.065 mol%, average particle size: $90.2 \pm 13.3$ nm (HEPES buffer)).

Example 10

A hTRA-8 Fab' fragment was prepared just before use in the same way as in paragraph (1) of Example 1. Furthermore, a liposome dispersion was prepared just before use in the same way as in paragraph (2) of Example 9.

The antibody was bound to the liposome by the following steps: the hTRA-8 Fab' (5 mg/ml, 16.5 μl) and the liposome dispersion (10 mM eggPC, 300 μl) were mixed at a ratio of hTRA-8 Fab':DSPE-PEG3400-Mal=1:20 (molar ratio) to react the thiol group of the antibody with the terminal maleimide group of the PEG chain on the liposome. Antibody-unbound maleimide groups were inactivated by the addition of 3 μl of 100 mM mercaptoethanol (Wako Pure Chemical Industries, Ltd.) which was 10 equivalents with respect to DSPE-PEG3400-Mal and subsequent reaction at room temperature for 30 minutes. Unreacted hTRA-8 Fab' was removed by gel filtration chromatography (AKTA explorer 10S (GE HEALTHCARE INC.); column (GE HEALTHCARE INC., HiLoad Superdex 200 16/60 prep grade); HEPES buffer (pH 7.4); 4°C; 2 ml/min; detection wavelength: UV 280 nm) to separate immunoliposome fractions (36-48 ml fractions). Ultrafiltration concentration was performed using Amicon Ultra (MILLIPORE INC., molecular cutoff: 50,000) to obtain the present immunoliposome in which the cysteine residue on the antibody was bound with the end of PEG on the liposome (liposome composition: No. 10 of Table 1, antibody concentration: 90.9 μg/ml, phospholipid concentration: 2.98 mM, antibody density: 0.033 mol%, average particle size: $83.1 \pm 13.0$ nm (HEPES buffer)).

Example 11

(1) Preparation of hTRA-8 Fab'

A hTRA-8 Fab' fragment was prepared just before use in the same way as in paragraph (1) of Example 1.

(2) Preparation of liposome

20.3 mg, 7.73 mg, and 1.26 mg (molar ratio 100:66:1) of L-α-dimyristoylphosphatidylcholine (hereinafter, referred to as DMPC; NOF CORPORATION, COATSOME MC-4040), cholesterol (Sigma-Aldrich, Inc.), and poly(ethylene glycol)succinyl distearoylphosphatidylethanolamine having a maleimide group at the end of polyethylene glycol of approximately 3400 in molecular weight (hereinafter, referred to as DSPE-PEG3400-Mal; NOF CORPORATION, SUNBRIGHT DSPE-034MA) were weighed, respectively, in an eggplant-shaped flask, to which 3 ml of chloroform (KANTO CHEMICAL CO., INC.) was added for dissolution. Next, the chloroform was distilled off under reduced pressure to thereby form a thin layer of lipids on the interior wall of the flask. To this thin layer of lipids, 3 ml of a HEPES buffer was added to obtain a crude liposome (DMPC concentration: 10 mM) dispersion. Subsequently, this liposome dispersion was repeatedly extruded from a polycarbonate membrane (Avanti POLAR LIPID, INC.) having a pore size of 100 nm using an extruder (The Mini-Extruder, Avanti POLAR LIPID, INC.) to produce liposomes with an appropriate particle size.

(3) Binding of antibody to liposome

The hTRA-8 Fab' (5 mg/ml, 66 μl) and the liposome dispersion (10 mM DMPC, 300 μl) were mixed at a ratio of hTRA-8 Fab':DSPE-PEG3400-Mal=1:5 (molar ratio) to react the thiol group of the antibody with the terminal maleimide group of the PEG chain on the liposome. Antibody-unbound maleimide groups were inactivated by the addition of 3 μl of 100 mM mercaptoethanol (Wako Pure Chemical Industries, Ltd.) which was 10 equivalents with respect to DSPE-PEG3400-Mal and subsequent reaction at room temperature for 30 minutes. Unreacted hTRA-8 Fab' was removed by gel filtration chromatography (AKTA explorer 10S (GE HEALTHCARE INC.); column (GE HEALTHCARE INC., HiLoad Superdex 200 16/60 prep grade); HEPES buffer (pH 7.4); 4°C; 2 ml/min; detection wavelength: UV 280 nm) to separate immunoliposome fractions (36-48 ml fractions). Ultrafiltration concentration was performed using Amicon Ultra (MILLIPORE INC., molecular cutoff: 50,000) to obtain the present immunoliposome in which the cysteine residue on the antibody was bound with the end of PEG on the liposome (liposome composition: No. 11 of Table 1, antibody concentration: 148.5 μg/ml, phospholipid concentration: 2.47 mM, antibody density: 0.065 mol%, average particle size: 117.9±47.1 nm (HEPES buffer)).

Example 12

A hTRA-8 Fab' fragment was prepared just before use in the same way as in paragraph (1) of Example 1. Furthermore, a liposome dispersion was prepared just before use in the same way as in paragraph (2) of Example 11.
The antibody was bound to the liposome by the following steps: the hTRA-8 Fab' (5 mg/ml, 16.5 μl) and the liposome dispersion (10 mM DMPC, 300 μl) were mixed at a ratio of hTRA-8 Fab':DSPE-PEG3400-Mal=1:20 (molar ratio) to react the thiol group of the antibody with the terminal maleimide group of the PEG chain on the liposome. Antibody-unbound maleimide groups were inactivated by the addition of 3 μl of 100 mM mercaptoethanol (Wako Pure Chemical Industries, Ltd.) which was 10 equivalents with respect to DSPE-PEG3400-Mal and subsequent reaction at room temperature for 30 minutes. Unreacted hTRA-8 Fab' was removed by gel filtration chromatography (AKTA explorer 10S (GE HEALTHCARE INC.); column (GE HEALTHCARE INC., HiLoad Superdex 200 16/60 prep grade); HEPES buffer (pH 7.4); 4°C; 2 ml/min; detection wavelength: UV 280 nm) to separate immunoliposome fractions (36-48 ml fractions). Ultrafiltration concentration was performed using Amicon Ultra (MILLIPORE INC., molecular cutoff: 50,000) to obtain the present immunoliposome in which the cysteine residue on the antibody was bound with the end of PEG on the liposome (liposome composition: No. 12 of Table 1, antibody concentration: 76.8 μg/ml, phospholipid concentration: 2.94 mM, antibody density: 0.028 mol%, average particle size: 119.3±45.0 nm (HEPES buffer)).

Example 13

(1) Preparation of hTRA-8 Fab'

A hTRA-8 Fab' fragment was prepared just before use in the same way as in paragraph (1) of Example 1.

(2) Preparation of liposome

23.6 mg, 7.73 mg, and 1.26 mg (molar ratio 100:66:1) of L-α-dioleoylphosphatidylcholine (hereinafter, referred to as DOPC; NOF CORPORATION, COATSOME MC-8181), cholesterol (Sigma-Aldrich, Inc.), and poly(ethylene glycol) succinyl distearoylphosphatidylethanolamine having a maleimide group at the end of polyethylene glycol of approximately 3400 in molecular weight(hereinafter, referred to as DSPE-PEG3400-Mal; NOF CORPORATION, SUNBRIGHT DSPE-034MA) were weighed, respectively, in an eggplant-shaped flask, to which 3 ml of chloroform (KANTO CHEMICAL CO., INC.) was added for dissolution. Next, the chloroform was distilled off under reduced pressure to thereby form a thin layer of lipids on the interior wall of the flask. To this thin layer of lipids, 3 ml of a HEPES buffer was added to obtain a crude liposome (DOPC concentration: 10 mM) dispersion. Subsequently, this liposome dispersion was repeatedly extruded from a polycarbonate membrane (Avanti POLAR LIPID, INC.) having a pore size of 100 nm using an extruder (The Mini-Extruder, Avanti POLAR LIPID, INC.) to produce liposomes with an appropriate particle size.

(3) Binding of antibody to liposome

The hTRA-8 Fab' (5 mg/ml, 66 μl) and the liposome dispersion (10 mM DOPC, 300 μl) were mixed at a ratio of hTRA-8 Fab':DSPE-PEG3400-Mal=1:5 (molar ratio) to react the thiol group of the antibody with the terminal maleimide group of the PEG chain on the liposome. Antibody-unbound maleimide groups were inactivated by the addition of 3 μl of 100 mM mercaptoethanol (Wako Pure Chemical Industries, Ltd.) which was 10 equivalents with respect to DSPE-PEG3400-Mal and subsequent reaction at room temperature for 30 minutes. Unreacted hTRA-8 Fab' was removed by gel filtration chromatography (AKTA explorer 10S (GE HEALTHCARE INC.); column (GE HEALTHCARE INC., HiLoad Superdex 200 16/60 prep grade); HEPES buffer (pH 7.4); 4°C; 2 ml/min; detection wavelength: UV 280 nm) to separate immunoliposome fractions (36-48 ml fractions). Ultrafiltration concentration was performed using Amicon Ultra (MILLIPORE INC., molecular cutoff: 50,000) to obtain the present immunoliposome in which the cysteine residue on the antibody was bound with the end of PEG on the liposome (liposome composition: No. 13 of Table 1, antibody concentration: 136.3 μg/ml, phospholipid concentration: 2.30 mM, antibody density: 0.064 mol% (HEPES buffer)).

Example 14

A hTRA-8 Fab' fragment was prepared just before use in the same way as in paragraph (1) of Example 1. Furthermore, a liposome dispersion was prepared just before use in the same way as in paragraph (2) of Example 13.

The antibody was bound to the liposome by the following steps: the hTRA-8 Fab' (5 mg/ml, 16.5 $\mu$l) and the liposome dispersion (10 mM DOPC, 300 $\mu$l) were mixed at a ratio of hTRA-8 Fab':DSPE-PEG3400-Mal=1:20 (molar ratio) to react the thiol group of the antibody with the terminal maleimide group of the PEG chain on the liposome. Antibody-unbound maleimide groups were inactivated by the addition of 3 $\mu$l of 100 mM mercaptoethanol (Wako Pure Chemical Industries, Ltd.) which was 10 equivalents with respect to DSPE-PEG3400-Mal and subsequent reaction at room temperature for 30 minutes. Unreacted hTRA-8 Fab' was removed by gel filtration chromatography (AKTA explorer 10S (GE HEALTHCARE INC.); column (GE HEALTHCARE INC., HiLoad Superdex 200 16/60 prep grade); HEPES buffer (pH 7.4); 4°C; 2 ml/min; detection wavelength: UV 280 nm) to separate immunoliposome fractions (36-48 ml fractions). Ultrafiltration concentration was performed using Amicon Ultra (MILLIPORE INC., molecular cutoff: 50,000) to obtain the present immunoliposome in which the cysteine residue on the antibody was bound with the end of PEG on the liposome (liposome composition: No. 14 of Table 1, antibody concentration: 72.9 $\mu$g/ml, phospholipid concentration: 2.13 mM, antibody density: 0.037 mol% (HEPES buffer)).

Example 15

(1) Preparation of hTRA-8 Fab'

A hTRA-8 Fab' fragment was prepared just before use in the same way as in Example 1.

(2) Preparation of liposome

23.7 mg, 7.73 mg, and 1.26 mg (molar ratio 100:66:1) of L-$\alpha$-distearoylphosphatidylcholine (hereinafter, referred to as DSPC; NOF CORPORATION, COATSOME MC-8080), cholesterol (Sigma-Aldrich, Inc.), and poly(ethylene glycol) succinyl distearoylphosphatidylethanolamine having a maleimide group at the end of polyethylene glycol of approximately 3400 in molecular weight (hereinafter, referred to as DSPE-PEG3400-Mal; NOF CORPORATION, SUNBRIGHT DSPE-034MA) were weighed, respectively, in an eggplant-shaped flask, to which 3 ml of chloroform (KANTO CHEMICAL CO., INC.) was added for dissolution. Next, the chloroform was distilled off under reduced pressure to thereby form a thin layer of lipids on the interior wall of the flask. To this thin layer of lipids, 3 ml of a HEPES buffer was added to obtain a crude liposome (DSPC concentration: 10 mM) dispersion. Subsequently, this liposome dispersion was repeatedly extruded from a polycarbonate membrane (Avanti POLAR LIPID, INC.) having a pore size of 100 nm using an extruder (The Mini-Extruder, Avanti POLAR LIPID, INC.) to produce liposomes with an appropriate particle size.

(3) Binding of antibody to liposome

The hTRA-8 Fab' (5 mg/ml, 165 $\mu$l) and the liposome dispersion (10 mM DSPC, 300 $\mu$l) were mixed at a ratio of hTRA-8 Fab':DSPE-PEG3400-Mal=1:2 (molar ratio) to react the thiol group of the antibody with the terminal maleimide group of the PEG chain on the liposome. Antibody-unbound maleimide groups were inactivated by the addition of 3 $\mu$l of 100 mM mercaptoethanol (Wako Pure Chemical Industries, Ltd.) which was 10 equivalents with respect to DSPE-PEG3400-Mal and subsequent reaction at room temperature for 30 minutes. Unreacted hTRA-8 Fab' was removed by gel filtration chromatography (AKTA explorer 10S (GE HEALTHCARE INC.); column (GE HEALTHCARE INC., HiLoad Superdex 200 16/60 prep grade); HEPES buffer (pH 7.4); 4°C; 2 ml/min; detection wavelength: UV 280 nm) to separate immunoliposome fractions (36-48 ml fractions). Ultrafiltration concentration was performed using Amicon Ultra (MILLIPORE INC., molecular cutoff: 50,000) to obtain the present immunoliposome in which the cysteine residue on the antibody was bound with the end of PEG on the liposome (liposome composition: No. 15 of Table 1, antibody concentration: 178.9 $\mu$g/ml, phospholipid concentration: 1.97 mM, antibody density: 0.099 mol% (HEPES buffer)).

Example 16

A hTRA-8 Fab' fragment was prepared just before use in the same way as in paragraph (1) of Example 1. Furthermore, a liposome dispersion was prepared just before use in the same way as in paragraph (2) of Example 15.

The antibody was bound to the liposome by the following steps: the hTRA-8 Fab' (5 mg/ml, 66 $\mu$l) and the liposome dispersion (10 mM DSPC, 300 $\mu$l) were mixed at a ratio of hTRA-8 Fab':DSPE-PEG3400-Mal=1:5 (molar ratio) to react the thiol group of the antibody with the terminal maleimide group of the PEG chain on the liposome. Antibody-unbound maleimide groups were inactivated by the addition of 3 $\mu$l of 100 mM mercaptoethanol (Wako Pure Chemical Industries, Ltd.) which was 10 equivalents with respect to DSPE-PEG3400-Mal and subsequent reaction at room temperature for 30 minutes. Unreacted hTRA-8 Fab' was removed by gel filtration chromatography (AKTA explorer 10S (GE HEALTHCARE INC.); column (GE HEALTHCARE INC., HiLoad Superdex 200 16/60 prep grade); HEPES buffer (pH 7.4); 4°C; 2 ml/min; detection wavelength: UV 280 nm) to separate immunoliposome fractions (36-48 ml fractions). Ultrafiltration concentration was performed using Amicon Ultra (MILLIPORE INC., molecular cutoff: 50,000) to obtain the present immunoliposome in which the cysteine residue on the antibody was bound with the end of PEG on the liposome (liposome composition: No. 16 of Table 1, antibody concentration: 60.4 μg/ml, phospholipid concentration: 1.53 mM, antibody density: 0.043 mol% (HEPES buffer)).

Example 17

(1) Preparation of hTRA-8 Fab'

A hTRA-8 Fab' fragment was prepared just before use in the same way as in paragraph (1) of Example 1.

(2) Preparation of liposome

11 mg, 3.9 mg, and 0.69 mg (molar ratio 100:66:5) of L-α-dipalmitoylphosphatidylcholine (hereinafter, referred to as DPPC; NOF CORPORATION, COATSOME MC-6060), cholesterol (Sigma-Aldrich, Inc.), and distearoylphosphatidylethanolamine having a maleimide group (hereinafter, referred to as DSPE-Mal; NOF CORPORATION, COATSOME FE-8080MA3) were weighed, respectively, in an eggplant-shaped flask, to which 1.5 ml of chloroform (KANTO CHEMICAL CO., INC.) was added for dissolution. Next, the chloroform was distilled off under reduced pressure to thereby form a thin layer of lipids on the interior wall of the flask. To this thin layer of lipids, 1.5 ml of a HEPES buffer was added to obtain a crude liposome (DPPC concentration: 10 mM) dispersion. Subsequently, this liposome dispersion was repeatedly extruded from a polycarbonate membrane (Avanti POLAR LIPID, INC.) having a pore size of 100 nm using an extruder (The Mini-Extruder, Avanti POLAR LIPID, INC.) to produce liposomes with an appropriate particle size.

(3) Binding of antibody to liposome

The hTRA-8 Fab' (4.64 mg/ml, 237 μl) and the liposome dispersion (10 mM DPPC, 200 μl) were mixed at a ratio of hTRA-8 Fab':DSPE-Mal=1:1 (molar ratio) to react the thiol group of the antibody with the maleimide group on the liposome. Antibody-unbound maleimide groups were inactivated by the addition of 2 μl of 100 mM mercaptoethanol (Wako Pure Chemical Industries, Ltd.) which was 10 equivalents with respect to DSPE-Mal and subsequent reaction at room temperature for 30 minutes. Unreacted hTRA-8 Fab' was removed by gel filtration chromatography (AKTA explorer 10S (GE HEALTHCARE INC.); column (GE HEALTHCARE INC., HiLoad Superdex 200 16/60 prep grade); HEPES buffer (pH 7.4); 4°C; 2 ml/min; detection wavelength: UV 280 nm) to separate immunoliposome fractions (36-48 ml fractions). Ultrafiltration concentration was performed using Amicon Ultra (MILLIPORE INC., molecular cutoff: 50,000) to obtain the present immunoliposome in which the cysteine residue on the antibody was bound with the DSPE on the liposome (liposome composition: No. 17 of Table 1, antibody concentration: 124.0 μg/ml, phospholipid concentration: 1.34 mM, antibody density: 0.100 mol% (HEPES buffer)).

Example 18

(1) Preparation of hTRA-8 Fab'

A hTRA-8 Fab' fragment was prepared just before use in the same way as in paragraph (1) of Example 1.

(2) Preparation of DSPE-PEG3400-(hTRA-8 Fab') complex

DSPE-PEG3400-Mal (16.8 mg/ml, 100 μl) which was 20 equivalents with respect to the hTRA-8 Fab' (5 mg/ml, 220 μl) was added thereto, followed by reaction at 37°C for 1 hr. Antibody-unbound maleimide groups were inactivated by the addition of 40 μl of 100 mM mercaptoethanol (Wako Pure Chemical Industries, Ltd.) which was 10 equivalents with respect to DSPE-PEG3400-Mal and subsequent reaction at room temperature for 30 minutes. Unreacted hTRA-8 Fab' was removed by gel filtration chromatography (AKTA explorer 10S (GE HEALTHCARE INC.); column (GE HEALTHCARE INC., HiLoad Superdex 200 16/60 prep grade); HEPES buffer (pH 7.4); 4°C; 2 ml/min; detection wavelength: UV 280 nm) to separate DSPE-PEG3400-(hTRA-8 Fab') complex fractions (39-54 ml fractions). Ultrafiltration concentration was performed using Amicon Ultra (MILLIPORE INC., molecular cutoff: 50,000) to obtain a DSPE-PEG3400-(hTRA-8 Fab') complex. The amount of hTRA-8 Fab' per DSPE was 1428.6 μg/μmol of DSPE.

(3) Preparation of liposome

To prepare liposomes, 22.05 mg and 7.68 mg (molar ratio 3:2) of L-α-dipalmitoylphosphatidylcholine (hereinafter, referred to as DPPC; NOF CORPORATION, COATSOME MC-6060) and cholesterol (Sigma-Aldrich, Inc.) were weighed, respectively, in an eggplant-shaped flask, to which 3 ml of chloroform (KANTO CHEMICAL CO., INC.) was added for dissolution. Next, the chloroform was distilled off under reduced pressure to thereby form a thin layer of lipids on the interior wall of the flask. To this thin layer of lipids, 3 ml of a HEPES buffer was added to obtain a crude liposome (DPPC concentration: 10 mM) dispersion. Subsequently, this liposome dispersion was repeatedly extruded from a polycarbonate membrane (Avanti POLAR LIPID, INC.) having a pore size of 100 nm using an extruder (The Mini-Extruder, Avanti POLAR LIPID, INC.) to produce liposomes with an appropriate particle size.

(4) Fusion of liposome with DSPE-PEG3400-(hTRA-8 Fab') complex

The DSPE-PEG3400-(hTRA-8 Fab') complex (antibody concentration: 489.9 μg/ml, 408 μl) and the liposome dispersion (10 mM DPPC, 200 μl) were mixed and incubated at 60°C for 3 hr. Liposome-unfused DSPE-PEG3400-(hTRA-8 Fab') complexes were removed by gel filtration chromatography (column (GE HEALTHCARE INC., 10×300 mm, Sephacryl S-500 HR); HEPES buffer (pH 7.4); 4°C; 2 ml/min) to separate immunoliposome fractions (36-48 ml fractions). Ultrafiltration concentration was performed using Amicon Ultra (MILLIPORE INC., molecular cutoff: 50,000) to obtain the present immunoliposome in which the cysteine residue on the antibody was bound with the end of PEG on the liposome (liposome composition: No. 18 of Table 1, antibody concentration: 73.7 μg/ml, phospholipid concentration: 2.27 mM, antibody density: 0.035 mol% (HEPES buffer)).

Example 19

(1) Reduction of disulfide bond in hTRA-8 Fullbody (Fullbody means a full-length antibody; the same holds true for the description below)

hTRA-8 (antibody concentration: 2.5 mg/ml) was incubated at room temperature for 90 min in the presence of 30 mM L-cysteine (Wako Pure Chemical Industries, Ltd.) for the reduction of a disulfide bond in the hTRA-8 Fullbody. The L-cysteine was removed by gel filtration purification (column: GE HEALTHCARE INC., PD-10 Desalting column; HEPES buffer) to obtain hTRA-8 Fullbody having the reduced disulfide bond.

(2) Preparation of liposome

A liposome dispersion was prepared just before use in the same way as in paragraph (2) of Example 1.

(3) Binding reaction of antibody with liposome

The hTRA-8 Fullbody (5 mg/ml, 450 μl) and the liposome dispersion (10 mM DPPC, 300 μl) were mixed at a ratio of hTRA-8 Fullbody:DSPE-PEG3400-Mal=1:2 (molar ratio) to react the thiol group of the antibody with the terminal maleimide group of the PEG chain on the liposome. Antibody-unbound maleimide groups were inactivated by the addition of 3 μl of 100 mM mercaptoethanol (Wako Pure Chemical Industries, Ltd.) which was 10 equivalents with respect to DSPE-PEG3400-Mal and subsequent reaction at room temperature for 30 minutes. Unreacted hTRA-8 Fullbody was removed by gel filtration chromatography (AKTA explorer 10S (GE HEALTHCARE INC.); column (GE HEALTHCARE INC., HiLoad Superdex 200 16/60 prep grade); HEPES buffer (pH 7.4); 4°C; 2 ml/min; detection wavelength: UV 280 nm) to separate immunoliposome fractions (36-48 ml fractions). Ultrafiltration concentration was performed using Amicon Ultra (MILLIPORE INC., molecular cutoff: 50,000) to obtain the present immunoliposome in which the cysteine residue on the antibody was bound with the end of PEG on the liposome (liposome composition: No. 19 of Table 1, antibody concentration: 23.9 μg/ml, phospholipid concentration: 3.25 mM, antibody density: 0.0029 mol% (HEPES buffer)).

Example 20

A disulfide bond in hTRA-8 Fullbody was reduced in the same way as in paragraph (1) of Example 19. Furthermore, a liposome dispersion was prepared just before use in the same way as in the paragraph (2) of Example 1.

The binding reaction of the antibody with the liposome was performed by the following steps: the hTRA-8 Fullbody (5 mg/ml, 180 μl) and the liposome dispersion (10 mM DPPC, 300 μl) were mixed at a ratio of hTRA-8 Fullbody: DSPE-PEG3400-Mal=1:5 (molar ratio) to react the thiol group of the antibody with the terminal maleimide group of the PEG chain on the liposome. Antibody-unbound maleimide groups were inactivated by the addition of 3 μl of 100 mM

mercaptoethanol (Wako Pure Chemical Industries, Ltd.) which was 10 equivalents with respect to DSPE-PEG3400-Mal and subsequent reaction at room temperature for 30 minutes. Unreacted hTRA-8 Fullbody was removed by gel filtration chromatography (AKTA explorer 10S (GE HEALTHCARE INC.); column (GE HEALTHCARE INC., HiLoad Superdex 200 16/60 prep grade); HEPES buffer (pH 7.4); 4°C; 2 ml/min; detection wavelength: UV 280 nm) to separate immunoliposome fractions (36-48 ml fractions). Ultrafiltration concentration was performed using Amicon Ultra (MILLIPORE INC., molecular cutoff: 50,000) to obtain the present immunoliposome in which the cysteine residue on the antibody was bound with the end of PEG on the liposome (liposome composition: No. 20 of Table 1, antibody concentration: 65.4 $\mu$g/ml, phospholipid concentration: 3.07 mM, antibody density: 0.0085 mol% (HEPES buffer)).

Example 21

A disulfide bond in hTRA-8 Fullbody was reduced in the same way as in paragraph (1) of Example 19. Furthermore, a liposome dispersion was prepared just before use in the same way as in paragraph (2) of Example 1.
The binding reaction of the antibody with the liposome was performed by the following steps: the hTRA-8 Fullbody (5 mg/ml, 45 $\mu$l) and the liposome dispersion (10 mM DPPC, 300 $\mu$l) were mixed at a ratio of hTRA-8 Fullbody: DSPE-PEG3400-Mal=1:20 (molar ratio) to react the thiol group of the antibody with the terminal maleimide group of the PEG chain on the liposome. Antibody-unbound maleimide groups were inactivated by the addition of 3 $\mu$l of 100 mM mercaptoethanol (Wako Pure Chemical Industries, Ltd.) which was 10 equivalents with respect to DSPE-PEG3400-Mal and subsequent reaction at room temperature for 30 minutes. Unreacted hTRA-8 Fullbody was removed by gel filtration chromatography (AKTA explorer 10S (GE HEALTHCARE INC.); column (GE HEALTHCARE INC., HiLoad Superdex 200 16/60 prep grade); HEPES buffer (pH 7.4); 4°C; 2 ml/min; detection wavelength: UV 280 nm) to separate immunoliposome fractions (36-48 ml fractions). Ultrafiltration concentration was performed using Amicon Ultra (MILLIPORE INC., molecular cutoff: 50,000) to obtain the present immunoliposome in which the cysteine residue on the antibody was bound with the end of PEG on the liposome (liposome composition: No. 21 of Table 1, antibody concentration: 41.2 $\mu$g/ml, phospholipid concentration: 3.11 mM, antibody density: 0.0053 mol% (HEPES buffer)).

Example 22

(1) Thiolation of hTRA-8 Fullbody

4 mM Traut's Reagent (2-Iminothiolane·HCl, Pierce Biotechnology, Inc.) was added to hTRA-8 Fullbody (antibody concentration: 6 mg/ml, 20 mM HEPES, 150 mM NaCl, 2 mM EDTA, pH 8.0) at a molar ratio of hTRA-8:Traut's Reagent=1:4, followed by reaction at room temperature for 90 min. Then, the Traut's Reagent was removed by gel filtration chromatography (column: GE HEALTHCARE INC., NAP-5 Desalting column; HEPES buffer) to thiolate the amino groups of some lysine residues in the hTRA-8 Fullbody.

(2) Preparation of liposome

A liposome dispersion was prepared just before use in the same way as in paragraph (2) of Example 1.

(3) Binding reaction of antibody with liposome

The hTRA-8 Fullbody (2 mg/ml, 75 $\mu$l) and the liposome dispersion (10 mM DPPC, 150 $\mu$l) were mixed at a ratio of hTRA-8 Fullbody:DSPE-PEG3400-Mal=1:15 (molar ratio) to react the thiol group of the antibody with the terminal maleimide group of the PEG chain on the liposome. Antibody-unbound maleimide groups were inactivated by the addition of 1.5 $\mu$l of 100 mM mercaptoethanol (Wako Pure Chemical Industries, Ltd.) which was 10 equivalents with respect to DSPE-PEG3400-Mal and subsequent reaction at room temperature for 30 minutes. Unreacted hTRA-8 Fullbody was removed by gel filtration chromatography (AKTA explorer 10S (GE HEALTHCARE INC.); column (GE HEALTHCARE INC., HiLoad Superdex 200 16/60 prep grade); HEPES buffer (pH 7.4); 4°C; 2 ml/min; detection wavelength: UV 280 nm) to separate immunoliposome fractions (36-48 ml fractions). Ultrafiltration concentration was performed using Amicon Ultra (MILLIPORE INC., molecular cutoff: 50,000) to obtain the present immunoliposome in which the lysine residue on the antibody was bound with the end of PEG on the liposome (liposome composition: No. 22 of Table 1, antibody concentration: 70.3 $\mu$g/ml, phospholipid concentration: 1.17 mM, antibody density: 0.024 mol% (HEPES buffer)).

Example 23

The amino groups of some lysine residues in hTRA-8 Fullbody were thiolated in the same way as in paragraph (1) of Example 22. Furthermore, a liposome dispersion was prepared just before use in the same way as in paragraph (2) of Example 1.

The binding reaction of the antibody with the liposome was performed by the following steps:

The hTRA-8 Fullbody (5 mg/ml, 300 μl) and the liposome dispersion (10 mM DPPC, 150 μl) were mixed at a ratio of hTRA-8 Fullbody:DSPE-PEG3400-Mal=1:1.5 (molar ratio) to react the thiol group of the antibody with the terminal maleimide group of the PEG chain on the liposome. Antibody-unbound maleimide groups were inactivated by the addition of 1.5 μl of 100 mM mercaptoethanol (Wako Pure Chemical Industries, Ltd.) which was 10 equivalents with respect to DSPE-PEG3400-Mal and subsequent reaction at room temperature for 30 minutes. Unreacted hTRA-8 Fullbody was removed by gel filtration chromatography (AKTA explorer 10S (GE HEALTHCARE INC.); column (GE HEALTHCARE INC., HiLoad Superdex 200 16/60 prep grade); HEPES buffer (pH 7.4); 4°C; 2 ml/min; detection wavelength: UV 280 nm) to separate immunoliposome fractions (36-48 ml fractions). Ultrafiltration concentration was performed using Amicon Ultra (MILLIPORE INC., molecular cutoff: 50,000) to obtain the present immunoliposome in which the lysine residue on the antibody was bound with the end of PEG on the liposome (liposome composition: No. 23 of Table 1, antibody concentration: 184.4 μg/ml, phospholipid concentration: 1.07 mM, antibody density: 0.0685 mol% (HEPES buffer)).

Example 24

The amino groups of some lysine residues in hTRA-8 Fullbody were thiolated in the same way as in paragraph (1) of Example 22. Furthermore, a liposome dispersion was prepared just before use in the same way as in paragraph (2) of Example 5.

The binding reaction of the antibody with the liposome was performed by the following steps: the hTRA-8 Fullbody (5 mg/ml, 1.1 ml) and the liposome dispersion (10 mM DPPC, 16.5 ml) were mixed at a ratio of hTRA-8 Fullbody: DSPE-PEG3400-Mal=1:45 (molar ratio) to react the thiol group of the antibody with the terminal maleimide group of the PEG chain on the liposome. Antibody-unbound maleimide groups were inactivated by the addition of 165 μl of 100 mM mercaptoethanol (Wako Pure Chemical Industries, Ltd.) which was 10 equivalents with respect to DSPE-PEG3400-Mal and subsequent reaction at room temperature for 30 minutes. Unreacted hTRA-8 Fullbody was removed by gel filtration chromatography (AKTA explorer 10S (GE HEALTHCARE INC.); column (GE HEALTHCARE INC., HiLoad Superdex 200 16/60 prep grade); HEPES buffer (pH 7.4); 4°C; 2 ml/min; detection wavelength: UV 280 nm) to separate immunoliposome fractions (36-48 ml fractions). Ultrafiltration concentration was performed using Amicon Ultra (MILLIPORE INC., molecular cutoff: 50,000) to obtain the present immunoliposome in which the lysine residue on the antibody was bound with the end of PEG on the liposome (liposome composition: No. 24 of Table 1, antibody concentration: 166.5 μg/ml, phospholipid concentration: 5.25 mM, antibody density: 0.0126 mol% (HEPES buffer)).

Example 25

The amino groups of some lysine residues in hTRA-8 Fullbody were thiolated in the same way as in paragraph (1) of Example 22. Furthermore, a liposome dispersion was prepared just before use in the same way as in paragraph (2) of Example 5.

The binding reaction of the antibody with the liposome was performed by the following steps: the hTRA-8 Fullbody (5 mg/ml, 4 ml) and the liposome dispersion (10 mM DPPC, 1995 μl) were mixed at a ratio of hTRA-8 Fullbody: DSPE-PEG3400-Mal=1:1.5 (molar ratio) to react the thiol group of the antibody with the terminal maleimide group of the PEG chain on the liposome.

Antibody-unbound maleimide groups were inactivated by the addition of 19.95 μl of 100 mM mercaptoethanol (Wako Pure Chemical Industries, Ltd.) which was 10 equivalents with respect to DSPE-PEG3400-Mal and subsequent reaction at room temperature for 30 minutes. Unreacted hTRA-8 Fullbody was removed by gel filtration chromatography (AKTA explorer 10S (GE HEALTHCARE INC.); column (GE HEALTHCARE INC., HiLoad Superdex 200 16/60 prep grade); HEPES buffer (pH 7.4); 4°C; 2 ml/min; detection wavelength: UV 280 nm) to separate immunoliposome fractions (36-48 ml fractions). Ultrafiltration concentration was performed using Amicon Ultra (MILLIPORE INC., molecular cutoff: 50,000) to obtain the present immunoliposome in which the lysine residue on the antibody was bound with the end of PEG on the liposome (liposome composition: No. 25 of Table 1, antibody concentration: 314.5 μg/ml, phospholipid concentration: 0.96 mM, antibody density: 0.131 mol% (HEPES buffer)).

Example 26

(1) Preparation of hHFE7A Fab'

The F(ab')$_2$ solution (antibody concentration: 2.5 mg/ml, HEPES buffer) prepared in Reference Example 2 was incubated at room temperature for 90 minutes in the presence of 10 mM L-cysteine (Wako Pure Chemical Industries, Ltd.) for reduction to Fab'. The L-cysteine was removed by gel filtration purification (column: GE HEALTHCARE INC., PD-10 Desalting column; HEPES buffer) to obtain a hHFE7A Fab' fragment.

(2) Preparation of liposome

A liposome dispersion was prepared just before use in the same way as in paragraph (2) of Example 1.

(3) Binding reaction of antibody with liposome

The hHFE7A Fab' (1.5 mg/ml, 33 μl) and the liposome dispersion (10 mM DPPC, 180 μl) were mixed at a ratio of hHFE7A Fab':DSPE-PEG3400-Mal=1:20 (molar ratio) to react the thiol group of the antibody with the terminal maleimide group of the PEG chain on the liposome.
Antibody-unbound maleimide groups were inactivated by the addition of 1.8 μl of 100 mM mercaptoethanol (Wako Pure Chemical Industries, Ltd.) which was 10 equivalents with respect to DSPE-PEG3400-Mal and subsequent reaction at room temperature for 30 minutes. Unreacted hHFE7A Fab' was removed by gel filtration chromatography (AKTA explorer 10S (GE HEALTHCARE INC.); column (GE HEALTHCARE INC., HiLoad Superdex 200 16/60 prep grade); HEPES buffer (pH 7.4); 4°C; 2 ml/min; detection wavelength: UV 280 nm) to separate immunoliposome fractions (36-48 ml fractions). Ultrafiltration concentration was performed using Amicon Ultra (MILLIPORE INC., molecular cutoff: 50,000) to obtain the present immunoliposome in which the cysteine residue on the antibody was bound with the end of PEG on the liposome (liposome composition: No. 26 of Table 1, antibody concentration: 47.9 μg/ml, phospholipid concentration: 2.03 mM, antibody density: 0.026 mol% (HEPES buffer)).

Example 27

A hHFE7A Fab' fragment was prepared just before use in the same way as in paragraph (1) of Example 26. Furthermore, a liposome dispersion was prepared just before use in the same way as in paragraph (2) of Example 1.
The binding reaction of the antibody with the liposome was performed by the following steps: the hHFE7A Fab' (1.5 mg/ml, 132 μl) and the liposome dispersion (10 mM DPPC, 180 μl) were mixed at a ratio of hHFE7A Fab':DSPE-PEG3400-Mal=1:5 (molar ratio) to react the thiol group of the antibody with the terminal maleimide group of the PEG chain on the liposome. Antibody-unbound maleimide groups were inactivated by the addition of 1.8μ l of 100 mM mercaptoethanol (Wako Pure Chemical Industries, Ltd.) which was 10 equivalents with respect to DSPE-PEG3400-Mal and subsequent reaction at room temperature for 30 minutes. Unreacted hHFE7A Fab' was removed by gel filtration chromatography (AKTA explorer 10S (GE HEALTHCARE INC.); column (GE HEALTHCARE INC., HiLoad Superdex 200 16/60 prep grade); HEPES buffer (pH 7.4); 4°C; 2 ml/min; detection wavelength: UV 280 nm) to separate immunoliposome fractions (36-48 ml fractions). Ultrafiltration concentration was performed using Amicon Ultra (MILLIPORE INC., molecular cutoff: 50,000) to obtain the present immunoliposome in which the cysteine residue on the antibody was bound with the end of PEG on the liposome (liposome composition: No. 27 of Table 1, antibody concentration: 115.0 μg/ml, phospholipid concentration: 1.54 mM, antibody density: 0.081 mol% (HEPES buffer)).

Example 28

A hHFE7A Fab' fragment was prepared just before use in the same way as in paragraph (1) of Example 26. Furthermore, a liposome dispersion was prepared just before use in the same way as in paragraph (2) of Example 1.
The binding reaction of the antibody with the liposome was performed by the following steps: the hHFE7A Fab' (1.5 mg/ml, 330 μl) and the liposome dispersion (10 mM DPPC, 180 μl) were mixed at a ratio of hHFE7A Fab': DSPE-PEG3400-Mal=1:2 (molar ratio) to react the thiol group of the antibody with the terminal maleimide group of the PEG chain on the liposome. Antibody-unbound maleimide groups were inactivated by the addition of 1.8 μl of 100 mM mercaptoethanol (Wako Pure Chemical Industries, Ltd.) which was 10 equivalents with respect to DSPE-PEG3400-Mal and subsequent reaction at room temperature for 30 minutes. Unreacted hHFE7A Fab' was removed by gel filtration chromatography (AKTA explorer 10S (GE HEALTHCARE INC.); column (GE HEALTHCARE INC., HiLoad Superdex 200 16/60 prep grade); HEPES buffer (pH 7.4); 4°C; 2 ml/min; detection wavelength: UV 280 nm) to separate immunoliposome fractions (36-48 ml fractions). Ultrafiltration concentration was performed using Amicon Ultra (MILLIPORE INC., molecular cutoff: 50,000) to obtain the present immunoliposome in which the cysteine residue on the antibody was bound with the end of PEG on the liposome (liposome composition: No. 28 of Table 1, antibody concentration: 151.6 μg/ml, phospholipid concentration: 1.60 mM, antibody density: 0.103 mol% (HEPES buffer)).

Example 29

(1) Thiolation of hHFE7A Fullbody

4 mM Traut's Reagent (2-Iminothiolane·HCl, Pierce Biotechnology, Inc.) was added to hHFE7A Fullbody (antibody concentration: 6 mg/ml, 20 mM HEPES, 150 mM NaCl, 2 mM EDTA, pH 8.0) at a molar ratio of hHFE7A:Traut's Reagent=1:4, followed by reaction at room temperature for 90 min. Then, unreacted Traut's Reagent was removed by gel filtration chromatography (column: GE HEALTHCARE INC., NAP-5 Desalting column; HEPES buffer) to thiolate the amino groups of some lysine residues in the hHFE7A Fullbody.

(2) Preparation of liposome

A liposome dispersion was prepared just before use in the same way as in paragraph (2) of Example 1.

(3) Binding reaction of antibody with liposome

The hHFE7A Fullbody (2 mg/ml, 75 μl) and the liposome dispersion (10 mM DPPC, 150 μl) were mixed at a ratio of hHFE7A Fullbody:DSPE-PEG3400-Mal=1:15 (molar ratio) to react the thiol group of the antibody with the terminal maleimide group of the PEG chain on the liposome. Antibody-unbound maleimide groups were inactivated by the addition of 1.5 μl of 100 mM mercaptoethanol (Wako Pure Chemical Industries, Ltd.) which was 10 equivalents with respect to DSPE-PEG3400-Mal and subsequent reaction at room temperature for 30 minutes. Unreacted hHFE7A Fullbody was removed by gel filtration chromatography (AKTA explorer 10S (GE HEALTHCARE INC.); column (GE HEALTHCARE INC., HiLoad Superdex 200 16/60 prep grade); HEPES buffer (pH 7.4); 4°C; 2 ml/min; detection wavelength: UV 280 nm) to separate immunoliposome fractions (36-48 ml fractions). Ultrafiltration concentration was performed using Amicon Ultra (MILLIPORE INC., molecular cutoff: 50,000) to obtain the present immunoliposome in which the lysine residue on the antibody was bound with the end of PEG on the liposome (liposome composition: No. 29 of Table 1, antibody concentration: 48.1 μg/ml, phospholipid concentration: 1.06 mM, antibody density: 0.018 mol% (HEPES buffer)).

Example 30

(1) Thiolation of hHFE7A Fullbody

4 mM Traut's Reagent (2-Iminothiolane·HCl, Pierce Biotechnology, Inc.) was added to hHFE7A Fullbody (antibody concentration: 6 mg/ml, 20 mM HEPES, 150 mM NaCl, 2 mM EDTA, pH 8.0) at a molar ratio of hHFE7A:Traut's Reagent=1:16, followed by reaction at room temperature for 90 min. Then, unreacted Traut's Reagent was removed by gel filtration chromatography (column: GE HEALTHCARE INC., NAP-5 Desalting column; HEPES buffer) to thiolate the amino groups of some lysine residues in the hHFE7A Fullbody.

(2) Preparation of liposome

A liposome dispersion was prepared just before use in the same way as in paragraph (2) of Example 1.

(3) Binding reaction of antibody with liposome

The hHFE7A Fullbody (2 mg/ml, 75 μl) and the liposome dispersion (10 mM DPPC, 150 μl) were mixed at a ratio of hHFE7A Fullbody:DSPE-PEG3400-Mal=1:15 (molar ratio) to react the thiol group of the antibody with the terminal maleimide group of the PEG chain on the liposome. Antibody-unbound maleimide groups were inactivated by the addition of 1.5 μl of 100 mM mercaptoethanol (Wako Pure Chemical Industries, Ltd.) which was 10 equivalents with respect to DSPE-PEG3400-Mal and subsequent reaction at room temperature for 30 minutes. Unreacted hHFE7A Fullbody was removed by gel filtration chromatography (AKTA explorer 10S (GE HEALTHCARE INC.); column (GE HEALTHCARE INC., HiLoad Superdex 200 16/60 prep grade); HEPES buffer (pH 7.4); 4°C; 2 ml/min; detection wavelength: UV 280 nm) to separate immunoliposome fractions (36-48 ml fractions). Ultrafiltration concentration was performed using Amicon Ultra (MILLIPORE INC., molecular cutoff: 50,000) to obtain the present immunoliposome in which the lysine residue on the antibody was bound with the end of PEG on the liposome (liposome composition: No. 30 of Table 1, antibody concentration: 157.6 μg/ml, phospholipid concentration: 1.30 mM, antibody density: 0.048 mol% (HEPES buffer)).

Example 31

The amino groups of some lysine residues in hHFE7A Fullbody were thiolated in the same way as in paragraph (1) of Example 29. Furthermore, a liposome dispersion was prepared just before use in the same way as in paragraph (2) of Example 1.

The binding reaction of the antibody with the liposome was performed by the following steps: the hHFE7A Fullbody (5 mg/ml, 300 μl) and the liposome dispersion (10 mM DPPC, 150 μl) were mixed at a ratio of hHFE7A Fullbody: DSPE-PEG3400-Mal=1:1.5 (molar ratio) to react the thiol group of the antibody with the terminal maleimide group of the PEG chain on the liposome. Antibody-unbound maleimide groups were inactivated by the addition of 1.5 μl of 100 mM mercaptoethanol (Wako Pure Chemical Industries, Ltd.) which was 10 equivalents with respect to DSPE-PEG3400-Mal and subsequent reaction at room temperature for 30 minutes. Unreacted hHFE7A Fullbody was removed by gel filtration chromatography (AKTA explorer 10S (GE HEALTHCARE INC.); column (GE HEALTHCARE INC., HiLoad Superdex 200 16/60 prep grade); HEPES buffer (pH 7.4); 4°C; 2 ml/min; detection wavelength: UV 280 nm) to separate immunoliposome fractions (36-48 ml fractions). Ultrafiltration concentration was performed using Amicon Ultra (MILLIPORE INC., molecular cutoff: 50,000) to obtain the present immunoliposome in which the lysine residue on the antibody was bound with the end of PEG on the liposome (liposome composition: No. 31 of Table 1, antibody concentration: 227.7 μg/ml, phospholipid concentration: 0.95 mM, antibody density: 0.096 mol% (HEPES buffer)).

The component composition of the immunoliposomes prepared in Examples 1 to 31 is shown in Table 1.

[Table 1]

| Example No. | Protein | Lipid composition | Antibody density (mol%) |
|---|---|---|---|
| 1 | A | DPPC : Chol : DSPE-PEG$_{3400}$ = 100 : 66 : 1 | 0.0058 |
| 2 | A | DPPC : Chol : DSPE-PEG$_{3400}$ = 100 : 66 : 1 | 0.05 |
| 3 | A | DPPC : Chol : DSPE-PEG$_{3400}$ = 100 : 66 : 1 | 0.102 |
| 4 | A | DPPC : Chol : DSPE-PEG$_{3400}$ = 100 : 66 : 5 | 0.224 |
| 5 | A | DPPC : Chol : DSPE-PEG$_{3400}$ = 100 : 66 : 1 | 0.040 |
| 6 | A | DPPC : Chol : DSPE-PEG$_{3400}$ = 100 : 66 : 1 | 0.167 |
| 7 | A | DPPC : Chol : DSPE-PEG$_{3400}$ = 100 : 66 : 1 | 0.064 |
| 8 | A | DPPC : Chol : DSPE-PEG$_{3400}$ = 100 : 66 : 1 | 0.055 |
| 9 | A | eggPC : Chol : DSPE-PEG$_{3400}$ = 100 : 66 : 1 | 0.065 |
| 10 | A | eggPC : Chol : DSPE-PEG$_{3400}$ = 100 : 66 : 1 | 0.033 |
| 11 | A | DMPC : Chol : DSPE-PEG$_{3400}$ = 100 : 66 : 1 | 0.065 |
| 12 | A | DMPC : Chol : DSPE-PEG$_{3400}$ = 100 : 66 : 1 | 0.028 |
| 13 | A | DOPC : Chol : DSPE-PEG$_{3400}$ = 100 : 66 : 1 | 0.064 |
| 14 | A | DOPC : Chol : DSPE-PEG$_{3400}$ = 100 : 66 : 1 | 0.037 |
| 15 | A | DSPC : Chol : DSPE-PEG$_{3400}$ = 100 : 66 : 1 | 0.099 |
| 16 | A | DSPC : Chol : DSPE-PEG$_{3400}$ = 100 : 66 : 1 | 0.043 |
| 17 | A | DPPC : Chol : DSPE = 100 : 66 : 5 | 0.100 |
| 18 | A | DPPC : Chol : DSPE-PEG$_{3400}$ = 100 : 66 : 1 | 0.035 |
| 19 | B | DPPC : Chol : DSPE-PEG$_{3400}$ = 100 : 66 : 1 | 0.0029 |
| 20 | B | DPPC : Chol : DSPE-PEG$_{3400}$ = 100 : 66 : 1 | 0.0085 |
| 21 | B | DPPC : Chol : DSPE-PEG$_{3400}$ = 100 : 66 : 1 | 0.0053 |
| 22 | B | DPPG : Chol : DSPE-PEG$_{3400}$ = 100 : 66 : 1 | 0.024 |
| 23 | B | DPPC : Chol : DSPE-PEG$_{3400}$ = 100 : 66 : 1 | 0.069 |
| 24 | B | DPPC : Chol : DSPE-PEG$_{3400}$ = 100 : 66 : 1 | 0.013 |
| 25 | B | DPPC : Chol : DSPE-PEG$_{3400}$ = 100 : 66 : 1 | 0.131 |
| 26 | C | DPPC : Chol : DSPE-PEG$_{3400}$ = 100: 66 : 1 | 0.026 |
| 27 | C | DPPC : Chol : DSPE-PEG$_{3400}$ = 100 : 66 : 1 | 0.081 |
| 28 | C | DPPC : Chol : DSPE-PEG$_{3400}$ = 100 : 66 : 1 | 0.103 |
| 29 | D | DPPC : Chol : DSPE-PEG$_{3400}$ = 100 : 66 : 1 | 0.018 |
| 30 | D | DPPC : Chol : DSPE-PEG$_{3400}$ = 100 : 66 : 1 | 0.048 |

(continued)

| Example No. | Protein | Lipid composition | Antibody density (mol%) |
|---|---|---|---|
| 31 | D | DPPC : Chol : DSPE-PEG$_{3400}$ = 100 : 66 : 1 | 0.096 |

A : hTRA-8 Fab', B : hTRA-8 Fullbody, C : hHFE7A Fab', D : hHFE7A Fullbody

Example 32

(1) Thiolation of hTRA-8 F(ab')$_2$

4 mM Traut's Reagent (2-Iminothiolane·HCl, Pierce Biotechnology, Inc.) was added to a F(ab')$_2$ solution (antibody concentration: 3 mg/ml, 50 mM citrate, 150 mM NaCl, 1 mM DTPA, pH 5.0) at a molar ratio of hTRA-8 F(ab')$_2$: Traut's Reagent=1:4, followed by reaction at room temperature for 90 min. Then, the Traut's Reagent was removed by gel filtration purification (column: GE HEALTHCARE INC., NAP-5 Desalting column; PBS) to thiolate the amino groups of some lysine residues in hTRA-8 F(ab')$_2$.

(2) Preparation of liposome

36.71 mg, 33.3 mg, and 2.1 mg (molar ratio 100:66:1) of L-$\alpha$-dipalmitoylphosphatidylcholine (hereinafter, referred to as DPPC; NOF CORPORATION, COATSOME MC6060), cholesterol (Sigma-Aldrich, Inc.), and poly(ethylene glycol)succinyl distearoylphosphatidylethanolamine having a maleimide group at the end of polyethylene glycol of approximately 3400 in molecular weight (hereinafter, referred to as DSPE-PEG3400-Mal; NOF CORPORATION, SUNBRIGHT DSPE-034MA) were weighed, respectively, in an eggplant-shaped flask, to which 3 ml of chloroform (KANTO CHEMICAL CO., INC.) was added for dissolution. Next, the chloroform was distilled off under reduced pressure to thereby form a thin layer of lipids on the interior wall of the flask. To this thin layer of lipids, 5 ml of PBS (20 mM phospha te, 150 mM NaCl, pH 7.4) was added for suspension to obtain a crude liposome (DPPC concentration: 10 mM) dispersion. Subsequently, this liposome dispersion was repeatedly extruded from a polycarbonate membrane (Avanti POLAR LIPID, INC.) having a pore size of 50 nm using an extruder (The Mini-Extruder, Avanti POLAR LIPID, INC.) to produce liposomes with an appropriate particle size.

(3) Binding reaction of antibody with liposome

The hTRA-8 F(ab')$_2$ (1 mg/ml, 400 μl) and the liposome dispersion (2 mM DPPC, 1000 μl) diluted 5-fold with PBS were mixed at a ratio of hTRA-8 F(ab')$_2$:DSPE-PEG3400-Mal=1:5 (molar ratio) to react the thiol group of the antibody with the terminal maleimide group of the PEG chain on the liposome. Antibody-unbound maleimide groups were inactivated by the addition of 10 μl of 20 mM mercaptoethanol (Wako Pure Chemical Industries, Ltd.) which was 10 equivalents with respect to DSPE-PEG3400-Mal and subsequent reaction at room temperature for 30 minutes. Unreacted hTRA-8 F(ab')$_2$ was removed by ultrafiltration through VIVASPIN (Sartorius Mechatronics, molecular cutoff: 300,000) to obtain the present immunoliposome in which the lysine residue on the antibody was bound with the end of PEG on the liposome (liposome composition: No. 32 of Table 2, antibody concentration: 191 μg/ml, phospholipid concentration: 3.38 mM, antibody density: 0.031 mol%, average particle size: 91.5±23.4 nm (PBS)).

Example 33

(1) Thiolation of hTRA-8 F(ab')$_2$

4 mM Traut's Reagent (2-Iminothiolane·HCl, Pierce Biotechnology, Inc.) was added to a F(ab')$_2$ solution (antibody concentration: 3 mg/ml, 20 mM phosphate, 150 mM NaCl, 1 mM DTPA, pH 6.0) at a molar ratio of hTRA-8 F(ab')$_2$:Traut's Reagent=1:4, followed by reaction at room temperature for 90 min. Then, the Traut's Reagent was removed by gel filtration purification (column: GE HEALTHCARE INC., NAP-5 Desalting column; PBS) to thiolate the amino groups of some lysine residues in hTRA-8 F(ab')$_2$.

(2) Preparation of liposome

A liposome dispersion was prepared just before use in the same way as in paragraph (2) of Example 32.

(3) Binding reaction of antibody with liposome

The hTRA-8 F(ab')$_2$ (1 mg/ml, 400 $\mu$l) and the liposome dispersion (2 mM DPPC, 300 $\mu$l) diluted 5-fold with PBS were mixed at a ratio of hTRA-8 F(ab')$_2$:DSPE-PEG3400-Mal=1:1.5 (molar ratio) to react the thiol group of the antibody with the terminal maleimide group of the PEG chain on the liposome. Antibody-unbound maleimide groups were inactivated by the addition of 3 $\mu$l of 20 mM mercaptoethanol (Wako Pure Chemical Industries, Ltd.) which was 10 equivalents with respect to DSPE-PEG3400-Mal and subsequent reaction at room temperature for 30 minutes. Unreacted hTRA-8 F(ab')$_2$ was removed by ultrafiltration through VIVASPIN (Sartorius Mechatronics, molecular cutoff: 300,000) to obtain the present immunoliposome in which the lysine residue on the antibody was bound with the end of PEG on the liposome (liposome composition: No. 33 of Table 2, antibody concentration: 16 $\mu$g/ml, phospholipid concentration: 0.79 mM, antibody density: 0.011 mol%, average particle size: 66.4$\pm$36.5 nm (PBS)).

Example 34

(1) Thiolation of hTRA-8 F(ab')$_2$

4 mM Traut's Reagent (2-Iminothiolane·HCl, Pierce Biotechnology, Inc.) was added to a F(ab')$_2$ solution (antibody concentration: 3 mg/ml, 20 mM phosphate, 150 mM NaCl, 1 mM DTPA, pH 7.0) at a molar ratio of hTRA-8 F(ab')$_2$:Traut's Reagent=1:4, followed by reaction at room temperature for 90 min. Then, the Traut's Reagent was removed by gel filtration purification (column: GE HEALTHCARE INC., NAP-5 Desalting column; PBS) to thiolate the amino groups of some lysine residues in the hTRA-8 F(ab')$_2$.

(2) Preparation of liposome

A liposome dispersion was prepared just before use in the same way as in paragraph (2) of Example 32.

(3) Binding reaction of antibody with liposome

The present immunoliposome in which the lysine residue on the antibody was bound with the end of PEG on the liposome was obtained in the same way as in paragraph (3) of Example 33 (liposome composition: No. 34 of Table 2, antibody concentration: 156 $\mu$g/ml, phospholipid concentration: 1.16 mM, antibody density: 0.073 mol%, average particle size: 68.0$\pm$26.2 nm (PBS)).

Example 35

(1) Thiolation of hTRA-8 F(ab')$_2$

4 mM Traut's Reagent (2-Iminothiolane·HCl, Pierce Biotechnology, Inc.) in dimethyl sulfoxide was added to a F(ab')$_2$ solution (antibody concentration: 3 mg/ml, 20 mM phosphate, 150 mM NaCl, 1 mM DTPA, pH 7.0) at a molar ratio of hTRA-8 F(ab')$_2$:Traut's Reagent=1:4, followed by reaction at room temperature for 90 min. Then, the Traut's Reagent was removed by gel filtration purification (column: GE HEALTHCARE INC., NAP-5 Desalting column; PBS) to thiolate the amino groups of some lysine residues in the hTRA-8 F(ab')$_2$.

(2) Preparation of liposome

A liposome dispersion was prepared just before use in the same way as in paragraph (2) of Example 32.

(3) Binding reaction of antibody with liposome

The present immunoliposome in which the lysine residue on the antibody was bound with the end of PEG on the liposome was obtained in the same way as in paragraph (3) of Example 33 (liposome composition: No. 35 of Table 2, antibody concentration: 233 $\mu$g/ml, phospholipid concentration: 1.16 mM, antibody density: 0.109 mol%, average particle size: 107.7$\pm$58.9 nm (PBS)).

Example 36

(1) Thiolation of Mouse Anti-hDR5 Antibody (MAB631)

20 mM phosphate, 150 mM NaCl, and 1 mM DTPA, pH 8.0 were added to Mouse Anti-hDR5 Antibody (MAB631) (R&D Systems, Inc., 1 mg) to adjust the antibody concentration to 2 mg/ml. 4 mM Traut's Reagent (2-Iminothiolane·HCl, Pierce Biotechnology, Inc.) was added thereto at a molar ratio of MAB631:Traut's Reagent=1:4, followed by reaction at room temperature for 90 min. Then, the Traut's Reagent was removed by gel filtration purification (column: GE HEALTHCARE INC., NAP-5 Desalting column; PBS) to thiolate the amino groups of some lysine residues in the MAB631.

(2) Preparation of liposome

A liposome dispersion was prepared just before use in the same way as in paragraph (2) of Example 32.

(3) Binding reaction of antibody with liposome

The MAB631 (1 mg/ml, 450 μl) and the liposome dispersion (2 mM DPPC, 750 μl) diluted 5-fold with PBS were mixed at a ratio of MAB631:DSPE-PEG3400-Mal=1:5 (molar ratio) to react the thiol group of the antibody with the terminal maleimide group of the PEG chain on the liposome. Antibody-unbound maleimide groups were inactivated by the addition of 7.5 μl of 20 mM mercaptoethanol (Wako Pure Chemical Industries, Ltd.) which was 10 equivalents with respect to DSPE-PEG3400-Mal and subsequent reaction at room temperature for 30 minutes. Unreacted MAB631 was removed by ultrafiltration through VIVASPIN (Sartorius Mechatronics, molecular cutoff: 300,000) to obtain the present immunoliposome in which the lysine residue on the antibody was bound with the end of PEG on the liposome (liposome composition: No. 36 of Table 2, antibody concentration: 232 μg/ml, phospholipid concentration: 4.06 mM, antibody density: 0.023 mol%, average particle size: 105.0±42.4 nm (PBS)).

Example 37

The amino groups of some lysine residues in MAB631 were thiolated in the same way as in paragraph (1) of Example 36. Furthermore, a liposome dispersion was prepared just before use in the same way as in paragraph (2) of Example 32.
The binding reaction of the antibody with the liposome was performed by the following steps: the MAB631 (1 mg/ml, 450 μl) and the liposome dispersion (2 mM DPPC, 225 μl) diluted 5-fold with PBS were mixed at a ratio of MAB631:DSPE-PEG3400-Mal=1:1.5 (molar ratio) to react the thiol group of the antibody with the terminal maleimide group of the PEG chain on the liposome. Antibody-unbound maleimide groups were inactivated by the addition of 2.25 μl of 20 mM mercaptoethanol (Wako Pure Chemical Industries, Ltd.) which was 10 equivalents with respect to DSPE-PEG3400-Mal and subsequent reaction at room temperature for 30 minutes. Unreacted MAB631 was removed by ultrafiltration through VIVASPIN (Sartorius Mechatronics, molecular cutoff: 300,000) to obtain the present immunoliposome in which the lysine residue on the antibody was bound with the end of PEG on the liposome (liposome composition: No. 37 of Table 2, antibody concentration: 141 μg/ml, phospholipid concentration: 0.83 mM, antibody density: 0.068 mol%, average particle size: 110.2±44.9 nm (PBS)).

Example 38

(1) Thiolation of Mouse Anti-hDR5 Antibody (MAB6311)

20 mM phosphate, 150 mM NaCl, and 1 mM DTPA, pH 8.0 were added to Mouse Anti-hDR5 Antibody (MAB6311) (R&D Systems, Inc., 1 mg) to adjust the antibody concentration to 2 mg/ml. 4 mM Traut's Reagent (2-Iminothiolane·HCl, Pierce Biotechnology, Inc.) was added thereto at a molar ratio of MAB6311:Traut's Reagent=1:4, followed by reaction at room temperature for 90 min. Then, the Traut's Reagent was removed by gel filtration purification (column: GE HEALTHCARE INC., NAP-5 Desalting column; PBS) to thiolate the amino groups of some lysine residues in the MAB6311.

(2) Preparation of liposome

A liposome dispersion was prepared just before use in the same way as in paragraph (2) of Example 32.

(3) Binding reaction of antibody with liposome

The MAB6311 (1 mg/ml, 360 μl) and the liposome dispersion (2 mM DPPC, 600 μl) diluted 5-fold with PBS were mixed at a ratio of MAB6311:DSPE-PEG3400-Mal=1:5 (molar ratio) to react the thiol group of the antibody with the terminal maleimide group of the PEG chain on the liposome. Antibody-unbound maleimide groups were inactivated by the addition of 6 μl of 20 mM mercaptoethanol (Wako Pure Chemical Industries, Ltd.) which was 10 equivalents with respect to DSPE-PEG3400-Mal and subsequent reaction at room temperature for 30 minutes. Unreacted MAB6311 was removed by ultrafiltration through VIVASPIN (Sartorius Mechatronics, molecular cutoff: 300,000) to obtain the present immunoliposome in which the lysine residue on the antibody was bound with the end of PEG on the liposome (liposome composition: No. 38 of Table 2, antibody concentration: 223 μg/ml, phospholipid concentration: 3.02 mM, antibody density: 0.029 mol%, average particle size: 124.3±44.6 nm (PBS)).

Example 39

The amino groups of some lysine residues in MAB6311 were thiolated in the same way as in paragraph (1) of Example 38. Furthermore, a liposome dispersion was prepared just before use in the same way as in paragraph (2) of Example 32.
The binding reaction of the antibody with the liposome was performed by the following steps: the MAB6311 (1 mg/ml, 360 μl) and the liposome dispersion (2 mM DPPC, 180 μl) diluted 5-fold with PBS were mixed at a ratio of MAB6311:DSPE-PEG3400-Mal=1:1.5 (molar ratio) to react the thiol group of the antibody with the terminal maleimide group of the PEG chain on the liposome. Antibody-unbound maleimide groups were inactivated by the addition of 1.8 μl of 20 mM mercaptoethanol (Wako Pure Chemical Industries, Ltd.) which was 10 equivalents with respect to DSPE-PEG3400-Mal and subsequent reaction at room temperature for 30 minutes. Unreacted MAB6311 was removed by ultrafiltration through VIVASPIN (Sartorius Mechatronics, molecular cutoff: 300,000) to obtain the present immunoliposome in which the lysine residue on the antibody was bound with the end of PEG on the liposome (liposome composition: No. 39 of Table 2, antibody concentration: 101 μg/ml, phospholipid concentration: 0.54 mM, antibody density: 0.075 mol%, average particle size: 126.0±49.9 nm (PBS)).

Example 40

(1) Thiolation of Mouse Anti-hFas Antibody (DX2)

Mouse Anti-hFas Antibody (DX2) (BD Biosciences Pharmingen, 1.5 mg) was medium-replaced (column: GE HEALTHCARE INC., NAP-5 Desalting column; 20 mM Phosphate, 150 mM NaCl, 1 mM DTPA, pH 8.0) to adjust the antibody concentration to 0.64 mg/ml. 4 mM Traut's Reagent (2-Iminothiolane·HCl, Pierce Biotechnology, Inc.) was added thereto at a molar ratio of DX2:Traut's Reagent=1:4, followed by reaction at room temperature for 90 min. Then, the Traut's Reagent was removed by gel filtration purification (column: GE HEALTHCARE INC., NAP-5 Desalting column; PBS) to thiolate the amino groups of some lysine residues in the DX2.

(2) Preparation of liposome

A liposome dispersion was prepared just before use in the same way as in paragraph (2) of Example 32.

(3) Binding reaction of antibody with liposome

The DX2 (0.25 mg/ml, 1330 μl) and the liposome dispersion (2 mM DPPC, 555 μl) diluted 5-fold with PBS were mixed at a ratio of DX2:DSPE-PEG3400-Mal=1:5 (molar ratio) to react the thiol group of the antibody with the terminal maleimide group of the PEG chain on the liposome. Antibody-unbound maleimide groups were inactivated by the addition of 11.1 μl of 10 mM mercaptoethanol (Wako Pure Chemical Industries, Ltd.) which was 10 equivalents with respect to DSPE-PEG3400-Mal and subsequent reaction at room temperature for 30 minutes. Unreacted DX2 was removed by ultrafiltration through VIVASPIN (Sartorius Mechatronics, molecular cutoff: 300,000) to obtain the present immunoliposome in which the lysine residue on the antibody was bound with the end of PEG on the liposome (liposome composition: No. 40 of Table 1, antibody concentration: 16 μg/ml, phospholipid concentration: 0.87 mM, antibody density: 0.0073 mol%, average particle size: 82.5±33.6 nm (PBS)).

Example 41

(1) Thiolation of hTRA-8 Fullbody

4 mM Traut's Reagent (2-Iminothiolane·HCl, Pierce Biotechnology, Inc.) was added to hTRA-8 Fullbody (antibody concentration: 3.87 mg/ml, 20 mM phosphate, 150 mM NaCl, 1 mM DTPA, pH 8.0) at a molar ratio of hTRA-8: Traut's Reagent=1:4, followed by reaction at room temperature for 90 min. Then, the Traut's Reagent was removed by gel filtration purification (column: GE HEALTHCARE INC., PD-10 Desalting column; PBS) to thiolate the amino groups of some lysine residues in the hTRA-8 Fullbody.

(2) Preparation of liposome

587.2 mg, 206.4 mg, 23.38 mg, and 0.8 ml (molar ratio 100:66:1:0.1) of L-$\alpha$-dipalmitoylphosphatidylcholine (hereinafter, referred to as DPPC; NOF CORPORATION, COATSOME MC-6060), cholesterol (Sigma-Aldrich, Inc.), poly(ethylene glycol)succinyl distearoylphosphatidylethanolamine having a maleimide group at the end of polyethylene glycol of approximately 2000 in molecular weight (hereinafter, referred to as DSPE-PEG2000-Mal; NOF CORPORATION, SUNBRIGHT DSPE-020MA), and Vybrant DiD cell-labeling solution (1 mM in solvent, Invitrogen) were weighed, respectively, in an eggplant-shaped flask, to which 30 ml of chloroform (KANTO CHEMICAL CO., INC.) was added for dissolution. Next, the chloroform was distilled off under reduced pressure to thereby form a thin layer of lipids on the interior wall of the flask. To this thin layer of lipids, 80 ml of PBS (20 mM phosphate, 150 mM NaCl, pH 7.4) was added for suspension to obtain a crude liposome (DPPC concentration: 10 mM) dispersion. Subsequently, this liposome dispersion was repeatedly extruded from a polycarbonate membrane (Avanti POLAR LIPID, INC.) having a pore size of 50 nm using EmulsiFlex C-5 (AVESTIN) to produce liposomes with an appropriate particle size.

(3) Binding reaction of antibody with liposome

The hTRA-8 Fullbody (1 mg/ml, 74.1 ml) and the liposome dispersion (2 mM DPPC, 123.5 ml) diluted 5-fold with PBS were mixed at a ratio of hTRA-8 Fullbody:DSPE-PEG2000-Mal=1:5 (molar ratio) to react the thiol group of the antibody with the terminal maleimide group of the PEG chain on the liposome. Antibody-unbound maleimide groups were inactivated by the addition of 247 $\mu$l of 100 mM mercaptoethanol (Wako Pure Chemical Industries, Ltd.) which was 10 equivalents with respect to DSPE-PEG2000-Mal and subsequent reaction at room temperature for 30 minutes. Unreacted hTRA-8 Fullbody was removed by ultrafiltration (LabScale TFF System (MILLIPORE INC.) through a polyethersulfone membrane (Pellicon XL 50, Biomax 300, molecular cutoff: 300,000, MILLIPORE INC.) to obtain the present immunoliposome in which the cysteine residue on the antibody was bound with the end of PEG on the liposome (liposome composition: No. 41 of Table 2, antibody concentration: 1819 $\mu$g/ml, phospholipid concentration: 10.08 mM, antibody density: 0.072 mol%, average particle size: 91.8 nm (PBS)).

Example 42

(1) Preparation of hTRA-8 Fab'

A F(ab')$_2$ solution (antibody concentration: 5 mg/ml, PBS) was incubated at room temperature for 90 minutes in the presence of 40 mM ($\pm$)-dithiothreitol (hereinafter, referred to as DTT; Wako Pure Chemical Industries, Ltd.) for reduction to Fab'. The DTT was removed by gel filtration purification (column: GE HEALTHCARE INC., PD-10 Desalting column; PBS) to obtain hTRA-8 Fab'.

(2) Preparation of liposome

A liposome dispersion was prepared just before use in the same way as in paragraph (2) of Example 41.

(3) Binding reaction of antibody with liposome

The hTRA-8 Fab' (5 mg/ml, 6.6 ml) and the liposome dispersion (10 mM DPPC, 12 ml) were mixed at a ratio of hTRA-8 Fab':DSPE-PEG2000-Mal=1:2 (molar ratio) to react the thiol group of the antibody with the terminal maleimide group of the PEG chain on the liposome. Antibody-unbound maleimide groups were inactivated by the addition of 0.12 ml of 100 mM mercaptoethanol (Wako Pure Chemical Industries, Ltd.) which was 10 equivalents with respect to DSPE-PEG2000-Mal and subsequent reaction at room temperature for 30 minutes. Unreacted hTRA-8 Fab' was removed by ultrafiltration (LabScale TFF System (MILLIPORE INC.) through a polyethersulfone membrane (Pellicon XL 50, Biomax 300, molecular cutoff: 300,000, MILLIPORE INC.) to obtain the present immunoliposome in which the cysteine residue on the antibody was bound with the end of PEG on the liposome (liposome composition: No. 42 of Table 2, antibody concentration: 1632 $\mu$g/ml, phospholipid concentration: 10.08 mM, antibody density: 0.176 mol%, average particle size: 76.7$\pm$22.9 nm (PBS)).

Example 43

(1) Thiolation of hTRA-8 F(ab')$_2$

4 mM Traut's Reagent (2-Iminothiolane·HCl, Pierce Biotechnology, Inc.) was added to a F(ab')$_2$ solution(antibody concentration: 3.39 mg/ml, 20 mM phosphate, 150 mM NaCl, 1 mM DTPA, pH 8.0) at a molar ratio of hTRA-8 F(ab')$_2$:Traut's Reagent=1:4, followed by reaction at room temperature for 90 min. Then, the Traut's Reagent was removed by gel filtration purification (column: GE HEALTHCARE INC., PD-10 Desalting column; PBS) to thiolate the amino groups of some lysine residues in hTRA-8 F(ab')$_2$.

(2) Preparation of liposome

A liposome dispersion was prepared just before use in the same way as in paragraph (2) of Example 41.

(3) Binding reaction of antibody with liposome

The hTRA-8 F(ab')$_2$ (1 mg/ml, 24.4 ml) and the liposome dispersion (10 mM DPPC, 11 ml) were mixed at a ratio of hTRA-8 F(ab')$_2$:DSPE-PEG2000-Mal=1:5 (molar ratio) to react the thiol group of the antibody with the terminal maleimide group of the PEG chain on the liposome. Antibody-unbound maleimide groups were inactivated by the addition of 110 $\mu$l of 100 mM mercaptoethanol (Wako Pure Chemical Industries, Ltd.) which was 10 equivalents with respect to DSPE-PEG2000-Mal and subsequent reaction at room temperature for 30 minutes. Unreacted hTRA-8 F(ab')$_2$ was removed by ultrafiltration (LabScale TFF System (MILLIPORE INC.)) through a polyethersulfone membrane (Pellicon XL 50, Biomax 300, molecular cutoff: 300,000, MILLIPORE INC.) to obtain the present immunoliposome in which the cysteine residue on the antibody was bound with the end of PEG on the liposome (liposome composition: No. 43 of Table 2, antibody concentration: 1147 $\mu$g/ml, phospholipid concentration: 10.08 mM, antibody density: 0.062 mol%, average particle size: 83.7$\pm$21.8 nm (PBS)).

Example 44

(1) Preparation of hTRA-8 Fab'

hTRA-8 Fab' was prepared just before use in the same way as in paragraph (1) of Example 42.

(2) Preparation of liposome

282.9 mg, 128.9 mg, and 21 mg (molar ratio 100:66:1) of didecanoylphosphatidylcholine (hereinafter, referred to as DDEPC; NOF CORPORATION, COATSOME MC-1010), cholesterol (Sigma-Aldrich, Inc.), and poly(ethylene glycol)succinyl distearoylphosphatidylethanolamine having a maleimide group at the end of polyethylene glycol of approximately 3400 in molecular weight (hereinafter, referred to as DSPE-PEG3400-Mal; NOF CORPORATION, SUNBRIGHT DSPE-034MA)were weighed, respectively, in an eggplant-shaped flask, to which 30 ml of chloroform (KANTO CHEMICAL CO., INC.) was added for dissolution. Next, the chloroform was distilled off under reduced pressure to thereby form a thin layer of lipids on the interior wall of the flask. To this thin layer of lipids, 50 ml of PBS (20 mM phosphate, 150 mM NaCl, pH 7.4) was added for suspension to obtain a crude liposome (DDEPC concentration: 10 mM) dispersion. Subsequently, this liposome dispersion was repeatedly extruded from a polycarbonate membrane (Avanti POLAR LIPID, INC.) having a pore size of 50 nm using an extruder (The Mini-Extruder, Avanti POLAR LIPID, INC.) to produce liposomes with an appropriate particle size.

(3) Binding of antibody to liposome

The hTRA-8 Fab' (1 mg/ml, 0.55 ml) and the liposome dispersion (10 mM DDEPC, 0.5 ml) were mixed at a ratio of hTRA-8 Fab':DSPE-PEG3400-Mal=1:5 (molar ratio) to react the thiol group of the antibody with the terminal maleimide group of the PEG chain on the liposome. Antibody-unbound maleimide groups were inactivated by the addition of 5 $\mu$l of 100 mM mercaptoethanol (Wako Pure Chemical Industries, Ltd.) which was 10 equivalents with respect to DSPE-PEG3400-Mal and subsequent reaction at room temperature for 30 minutes. Unreacted hTRA-8 Fab' was removed by ultrafiltration through VIVASPIN (Sartorius Mechatronics, molecular cutoff: 300,000) to obtain the present immunoliposome in which the cysteine residue on the antibody was bound with the end of PEG on the liposome (liposome composition: No. 44 of Table 2, antibody concentration: 426 μg/ml, phospholipid concentration: 2.31 mM, antibody density: 0.200 mol%, average particle size: 45.7±18.1 nm (PBS)).

Example 45

(1) Preparation of hTRA-8 Fab'

hTRA-8 Fab' was prepared just before use in the same way as in paragraph (1) of Example 42.

(2) Preparation of liposome

310.9 mg, 128.9 mg, and 21.7 mg (molar ratio 100:66:1) of dilauroylphosphatidylcholine (hereinafter, referred to as DLPC; NOF CORPORATION, COATSOME MC-2020), cholesterol (Sigma-Aldrich, Inc.), and poly(ethylene glycol) succinyl distearoylphosphatidylethanolamine having a maleimide group at the end of polyethylene glycol of approximately 3400 in molecular weight (hereinafter, referred to as DSPE-PEG3400-Mal; NOF CORPORATION, SUNBRIGHT DSPE-034MA) were weighed, respectively, in an eggplant-shaped flask, to which 30 ml of chloroform (KANTO CHEMICAL CO., INC.) was added for dissolution. Next, the chloroform was distilled off under reduced pressure to thereby form a thin layer of lipids on the interior wall of the flask. To this thin layer of lipids, 50 ml of PBS (20 mM phosphate, 150 mM NaCl, pH 7.4) was added for suspension to obtain a crude liposome (DLPC concentration: 10 mM) dispersion. Subsequently, this liposome dispersion was repeatedly extruded from a polycarbonate membrane (Avanti POLAR LIPID, INC.) having a pore size of 50 nm using an extruder (The Mini-Extruder, Avanti POLAR LIPID, INC.) to produce liposomes with an appropriate particle size.

(3) Binding of antibody to liposome

The hTRA-8 Fab' (1 mg/ml, 0.55 ml) and the liposome dispersion (10 mM DLPC, 0.5 ml) were mixed at a ratio of hTRA-8 Fab':DSPE-PEG3400-Mal=1:5 (molar ratio) to react the thiol group of the antibody with the terminal maleimide group of the PEG chain on the liposome. Antibody-unbound maleimide groups were inactivated by the addition of 5 μl of 100 mM mercaptoethanol (Wako Pure Chemical Industries, Ltd.) which was 10 equivalents with respect to DSPE-PEG3400-Mal and subsequent reaction at room temperature for 30 minutes. Unreacted hTRA-8 Fab' was removed by ultrafiltration through VIVASPIN (Sartorius Mechatronics, molecular cutoff: 300,000) to obtain the present immunoliposome in which the cysteine residue on the antibody was bound with the end of PEG on the liposome (liposome composition: No. 45 of Table 2, antibody concentration: 600 μg/ml, phospholipid concentration: 2.31 mM, antibody density: 0.135 mol%, average particle size: 65.6±25.7 nm (PBS)).

Example 46

(1) Preparation of hTRA-8 Fab'

hTRA-8 Fab' was prepared just before use in the same way as in paragraph (1) of Example 42.

(2) Preparation of liposome

45.12 mg, 12.89 mg, and 2.17 mg (molar ratio 100:66:1) of didocosanoylphosphatidylcholine (hereinafter, referred to as DDOPC; Sigma-Aldrich, Inc.), cholesterol (Sigma-Aldrich, Inc.), and poly(ethylene glycol)succinyl distearoylphosphatidylethanolamine having a maleimide group at the end of polyethylene glycol of approximately 3400 in molecular weight (hereinafter, referred to as DSPE-PEG3400-Mal; NOF CORPORATION, SUNBRIGHT DSPE-034MA) were weighed, respectively, in an eggplant-shaped flask, to which 3 ml of chloroform (KANTO CHEMICAL CO., INC.) was added for dissolution. Next, the chloroform was distilled off under reduced pressure to thereby form a thin layer of lipids on the interior wall of the flask. To this thin layer of lipids, 5 ml of PBS (20 mM phosphate, 150 mM NaCl, pH 7.4) was added for suspension to obtain a crude liposome (DDOPC concentration: 10 mM) dispersion. Subsequently, this liposome dispersion was repeatedly extruded from a polycarbonate membrane (Avanti POLAR LIPID, INC.) having a pore size of 400 nm using an extruder (The Mini-Extruder, Avanti POLAR LIPID, INC.). Then, liposomes with an appropriate particle size were produced by probe sonication (ULTRASONIC PROCESSOR SH-7600, SEIKO INSTRUMENT & ELECTRONICS LTD).

(3) Binding of antibody to liposome

The hTRA-8 Fab' (1 mg/ml, 0.55 ml) and the liposome dispersion (10 mM DDOPC, 0.5 ml) were mixed at a ratio of hTRA-8 Fab':DSPE-PEG3400-Mal=1:5 (molar ratio) to react the thiol group of the antibody with the terminal maleimide group of the PEG chain on the liposome.
Antibody-unbound maleimide groups were inactivated by the addition of 5 μl of 100 mM mercaptoethanol (Wako Pure Chemical Industries, Ltd.) which was 10 equivalents with respect to DSPE-PEG3400-Mal and subsequent reaction at room temperature for 30 minutes. Unreacted hTRA-8 Fab' was removed by ultrafiltration through VIVASPIN (Sartorius Mechatronics, molecular cutoff: 300,000) to obtain the present immunoliposome in which the cysteine residue on the antibody was bound with the end of PEG on the liposome (liposome composition: No. 46 of Table 2, antibody concentration: 183 μg/ml, phospholipid concentration: 2.11 mM, antibody density: 0.094 mol%, average particle size: 147.7±56.7 nm (PBS)).

Example 47

hTRA-8 Fab' was prepared just before use in the same way as in paragraph (1) of Example 42. Furthermore, a liposome dispersion was prepared just before use in the same way as in paragraph (2) of Example 44 and repeatedly extruded from a polycarbonate membrane (Avanti POLAR LIPID, INC.) having a pore size of 30 nm to produce liposomes with an appropriate particle size.
The binding reaction of the antibody with the liposome was performed in the same way as in paragraph (3) of Example 44 to obtain the present immunoliposome in which the cysteine residue on the antibody was bound with the end of PEG on the liposome (liposome composition: No. 47 of Table 2, antibody concentration: 458 μg/ml, phospholipid concentration: 7.29 mM, antibody density: 0.068 mol%, average particle size: 39.8±17.9 nm (PBS)).

Example 48

(1) Preparation of hTRA-8 Fab'

hTRA-8 Fab' was prepared just before use in the same way as in paragraph (1) of Example 42.

(2) Preparation of liposome

36.7 mg, 12.89 mg, and 2.17 mg (molar ratio 100:66:1) of L-α-dipalmitoylphosphatidylcholine (hereinafter, referred to as DPPC; NOF CORPORATION, COATSOME MC-6060), cholesterol (Sigma-Aldrich, Inc.), and poly(ethylene glycol)succinyl distearoylphosphatidylethanolamine having a maleimide group at the end of polyethylene glycol of approximately 3400 in molecular weight (hereinafter, referred to as DSPE-PEG3400-Mal; NOF CORPORATION, SUNBRIGHT DSPE-034MA) were weighed, respectively, in an eggplant-shaped flask, to which 3 ml of chloroform (KANTO CHEMICAL CO., INC.) was added for dissolution. Next, the chloroform was distilled off under reduced pressure to thereby form a thin layer of lipids on the interior wall of the flask. To this thin layer of lipids, 5 ml of PBS (20 mM phosphate, 150 mM NaCl, pH 7.4) was added for suspension to obtain a crude liposome (DPPC concentration: 10 mM) dispersion. Subsequently, this liposome dispersion was repeatedly extruded from a polycarbonate membrane (Avanti POLAR LIPID, INC.) having a pore size of 400 nm using an extruder (The Mini-Extruder, Avanti POLAR LIPID, INC.) to produce liposomes with an appropriate particle size.

(3) Binding of antibody to liposome

The hTRA-8 Fab' (1 mg/ml, 0.55 ml) and the liposome dispersion (10 mM DPPC, 0.5 ml) were mixed at a ratio of hTRA-8 Fab':DSPE-PEG3400-Mal=1:5 (molar ratio) to react the thiol group of the antibody with the terminal maleimide group of the PEG chain on the liposome. Antibody-unbound maleimide groups were inactivated by the addition of 5 μl of 100 mM mercaptoethanol (Wako Pure Chemical Industries, Ltd.) which was 10 equivalents with respect to DSPE-PEG3400-Mal and subsequent reaction at room temperature for 30 minutes. Unreacted hTRA-8 Fab' was removed by ultrafiltration through VIVASPIN (Sartorius Mechatronics, molecular cutoff: 300,000) to obtain the present immunoliposome in which the cysteine residue on the antibody was bound with the end of PEG on the liposome (liposome composition: No. 48 of Table 2, antibody concentration: 124 μg/ml, phospholipid concentration: 1.14 mM, antibody density: 0.118 mol%, average particle size: 75.2±9.1 nm, 232.2±29.3 nm (PBS)).

Example 49

(1) Preparation of hTRA-8 Fab'

hTRA-8 Fab' was prepared just before use in the same way as in paragraph (1) of Example 42.

(2) Preparation of liposome

44.92 mg, 12.89 mg, and 2.17 mg (molar ratio 100:66:1) of dierucoylphosphatidylcholine (hereinafter, referred to as DEPC; NOF CORPORATION, COATSOME MC-2121AL), cholesterol (Sigma-Aldrich, Inc.), and poly(ethylene glycol)succinyl distearoylphosphatidylethanolamine having a maleimide group at the end of polyethylene glycol of approximately 3400 in molecular weight (hereinafter, referred to as DSPE-PEG3400-Mal; NOF CORPORATION, SUNBRIGHT DSPE-034MA) were weighed, respectively, in an eggplant-shaped flask, to which 3 ml of chloroform (KANTO CHEMICAL CO., INC.) was added for dissolution. Next, the chloroform was distilled off under reduced pressure to thereby form a thin layer of lipids on the interior wall of the flask. To this thin layer of lipids, 5 ml of PBS (20 mM phosphate, 150 mM NaCl, pH 7.4) was added for suspension to obtain a crude liposome (DEPC concentration: 10 mM) dispersion. Subsequently, this liposome dispersion was repeatedly extruded from a polycarbonate membrane (Avanti POLAR LIPID, INC.) having a pore size of 800 nm using an extruder (The Mini-Extruder, Avanti POLAR LIPID, INC.) to produce liposomes with an appropriate particle size.

(3) Binding of antibody to liposome

The hTRA-8 Fab' (1 mg/ml, 0.55 ml) and the liposome dispersion (10 mM DEPC, 0.5 ml) were mixed at a ratio of hTRA-8 Fab':DSPE-PEG3400-Mal=1:5 (molar ratio) to react the thiol group of the antibody with the terminal maleimide group of the PEG chain on the liposome.
Antibody-unbound maleimide groups were inactivated by the addition of 5 μl of 100 mM mercaptoethanol (Wako Pure Chemical Industries, Ltd.) which was 10 equivalents with respect to DSPE-PEG3400-Mal and subsequent reaction at room temperature for 30 minutes. Unreacted hTRA-8 Fab' was removed by ultrafiltration through VIVASPIN (Sartorius Mechatronics, molecular cutoff: 300,000) to obtain the present immunoliposome in which the cysteine residue on the antibody was bound with the end of PEG on the liposome (liposome composition: No. 49 of Table 2, antibody concentration: 94 μg/ml, phospholipid concentration: 1.50 mM, antibody density: 0.068 mol%, average particle size: 130.0±43.2 nm (PBS)).

Example 50

(1) Preparation of hTRA-8 Fullbody

The amino groups of some lysine residues in hTRA-8 Fullbody were thiolated in the same way as in paragraph

(1) of Example 41.

(2) Preparation of liposome

36.7 mg, 12.89 mg, and 6.3 mg (molar ratio 100:66:3) of L-α-dipalmitoylphosphatidylcholine (hereinafter, referred to as DPPC; NOF CORPORATION, COATSOME MC-6060), cholesterol (Sigma-Aldrich, Inc.), and poly(ethylene glycol)succinyl distearoylphosphatidylethanolamine having a maleimide group at the end of polyethylene glycol of approximately 3400 in molecular weight (hereinafter, referred to as DSPE-PEG3400-Mal; NOF CORPORATION, SUNBRIGHT DSPE-034MA) were weighed, respectively, in an eggplant-shaped flask, to which 3 ml of chloroform (KANTO CHEMICAL CO., INC.) was added for dissolution. Next, the chloroform was distilled off under reduced pressure to thereby form a thin layer of lipids on the interior wall of the flask. To this thin layer of lipids, 5 ml of PBS (20 mM phosphate, 150 mM NaCl, pH 7.4) was added for suspension to obtain a crude liposome (DPPC concentration: 10 mM) dispersion. Subsequently, this liposome dispersion was repeatedly extruded from a polycarbonate membrane (Avanti POLAR LIPID, INC.) having a pore size of 50 nm using an extruder (The Mini-Extruder, Avanti POLAR LIPID, INC.) to produce liposomes with an appropriate particle size.

(3) Binding of antibody to liposome

The hTRA-8 Fullbody (1 mg/ml, 360 μl) and the liposome dispersion (2 mM DPPC, 200 μl) diluted 5-fold with PBS were mixed at a ratio of hTRA-8 Fullbody:DSPE-PEG3400-Mal=1:5 (molar ratio) to react the thiol group of the antibody with the terminal maleimide group of the PEG chain on the liposome. Antibody-unbound maleimide groups were inactivated by the addition of 2 $\mu$l of 60 mM mercaptoethanol (Wako Pure Chemical Industries, Ltd.) which was 10 equivalents with respect to DSPE-PEG3400-Mal and subsequent reaction at room temperature for 30 minutes. Unreacted hTRA-8 Fullbody was removed by ultrafiltration through VIVASPIN (Sartorius Mechatronics, molecular cutoff: 300,000) to obtain the present immunoliposome in which the lysine residue on the antibody was bound with the end of PEG on the liposome (liposome composition: No. 50 of Table 2, antibody concentration: 81 $\mu$g/ml, phospholipid concentration: 1.17 mM, antibody density: 0.027 mol%, average particle size: 57.3 nm (PBS)).

Example 51

The amino groups of some lysine residues in hTRA-8 Fullbody were thiolated in the same way as in paragraph (1) of Example 41. Furthermore, a liposome dispersion was prepared just before use in the same way as in the paragraph (2) of Example 50.

The binding reaction of the antibody with the liposome was performed by the following steps: the hTRA-8 Fullbody (1 mg/ml, 1200 $\mu$l) and the liposome dispersion (2 mM DPPC, 200 $\mu$l) diluted 5-fold with PBS were mixed at a ratio of hTRA-8 Fullbody:DSPE-PEG3400-Mal=1:1.5 (molar ratio) to react the thiol group of the antibody with the terminal maleimide group of the PEG chain on the liposome. Antibody-unbound maleimide groups were inactivated by the addition of 2 $\mu$l of 60 mM mercaptoethanol (Wako Pure Chemical Industries, Ltd.) which was 10 equivalents with respect to DSPE-PEG3400-Mal and subsequent reaction at room temperature for 30 minutes. Unreacted hTRA-8 Fullbody was removed by ultrafiltration through VIVASPIN (Sartorius Mechatronics, molecular cutoff: 300,000) to obtain the present immunoliposome in which the lysine residue on the antibody was bound with the end of PEG on the liposome (liposome composition: No. 51 of Table 2, antibody concentration: 156 $\mu$g/ml, phospholipid concentration: 0.73 mM, antibody density: 0.084 mol%, average particle size: 60.8 nm (PBS)).

Example 52

(1) Preparation of hTRA-8 Fullbody

The amino groups of some lysine residues in hTRA-8 Fullbody were thiolated in the same way as in paragraph

(1) of Example 41.

(2) Preparation of liposome

36.7 mg, 12.89 mg, and 21 mg (molar ratio 100:66:10) of L-$\alpha$-dipalmitoylphosphatidylcholine (hereinafter, referred to as DPPC; NOF CORPORATION, COATSOME MC-6060), cholesterol (Sigma-Aldrich, Inc.), and poly(ethylene glycol)succinyl distearoylphosphatidylethanolamine having a maleimide group at the end of polyethylene glycol of approximately 3400 in molecular weight (hereinafter, referred to as DSPE-PEG3400-Mal; NOF CORPORATION, SUNBRIGHT DSPE-034MA) were weighed, respectively, in an eggplant-shaped flask, to which 3 ml of chloroform (KANTO CHEMICAL CO., INC.) was added for dissolution. Next, the chloroform was distilled off under reduced pressure to thereby form a thin layer of lipids on the interior wall of the flask. To this thin layer of lipids, 5 ml of PBS (20 mM phosphate, 150 mM NaCl, pH 7.4) was added for suspension to obtain a crude liposome (DPPC concentration: 10 mM) dispersion. Subsequently, this liposome dispersion was repeatedly extruded from a polycarbonate membrane (Avanti POLAR LIPID, INC.) having a pore size of 50 nm using an extruder (The Mini-Extruder, Avanti POLAR LIPID, INC.) to produce liposomes with an appropriate particle size.

(3) Binding of antibody to liposome

The hTRA-8 Fullbody (1 mg/ml, 1200 $\mu$l) and the liposome dispersion (2 mM DPPC, 200 $\mu$l) diluted 5-fold with PBS were mixed at a ratio of hTRA-8 Fullbody:DSPE-PEG3400-Mal=1:5 (molar ratio) to react the thiol group of the antibody with the terminal maleimide group of the PEG chain on the liposome. Antibody-unbound maleimide groups were inactivated by the addition of 6.67 $\mu$l of 60 mM mercaptoethanol (Wako Pure Chemical Industries, Ltd.) which was 10 equivalents with respect to DSPE-PEG3400-Mal and subsequent reaction at room temperature for 30 minutes. Unreacted hTRA-8 Fullbody was removed by ultrafiltration through VIVASPIN (Sartorius Mechatronics, molecular cutoff: 300,000) to obtain the present immunoliposome in which the lysine residue on the antibody was bound with the end of PEG on the liposome (liposome composition: No. 52 of Table 2, antibody concentration: 161 $\mu$g/ml, phospholipid concentration: 0.66 mM, antibody density: 0.092 mol%, average particle size: 74.1 nm (PBS)).

Example 53

hTRA-8 Fab' was prepared just before use in the same way as in paragraph (1) of Example 42. Furthermore, a liposome dispersion was prepared just before use in the same way as in paragraph (2) of Example 50.

The binding reaction of the antibody with the liposome was performed by the following steps: the hTRA-8 Fab' (1 mg/ml, 0.44 ml) and the liposome dispersion (2 mM DPPC, 0.2 ml) diluted 5-fold with PBS were mixed at a ratio of hTRA-8 Fab':DSPE-PEG3400-Mal=1:1.5 (molar ratio) to react the thiol group of the antibody with the terminal maleimide group of the PEG chain on the liposome. Antibody-unbound maleimide groups were inactivated by the addition of 6.67 $\mu$l of 60 mM mercaptoethanol (Wako Pure Chemical Industries, Ltd.) which was 10 equivalents with respect to DSPE-PEG3400-Mal and subsequent reaction at room temperature for 30 minutes. Unreacted hTRA-8 Fab' was removed by ultrafiltration through VIVASPIN (Sartorius Mechatronics, molecular cutoff: 300,000) to obtain the present immunoliposome in which the cysteine residue on the antibody was bound with the end of PEG on the liposome (liposome composition: No. 53 of Table 2, antibody concentration: 125 $\mu$g/ml, phospholipid concentration: 0.45 mM, antibody density: 0.297 mol%, average particle size: 53.9 nm (PBS)).

Example 54

(1) Preparation of hTRA-8 Fab'

hTRA-8 Fab' was prepared just before use in the same way as in paragraph (1) of Example 42.

(2) Preparation of liposome

36.7 mg, 12.89 mg, and 1.47 mg (molar ratio 100:66:1) of L-$\alpha$-dipalmitoylphosphatidylcholine (hereinafter, referred to as DPPC; NOF CORPORATION, COATSOME MC-6060), cholesterol (Sigma-Aldrich, Inc.), and poly(ethylene glycol)succinyl distearoylphosphatidylethanolamine having a maleimide group at the end of polyethylene glycol of approximately 2000 in molecular weight (hereinafter, referred to as DSPE-PEG2000-Mal; NOF CORPORATION, SUNBRIGHT DSPE-020MA) were weighed, respectively, in an eggplant-shaped flask, to which 3 ml of chloroform (KANTO CHEMICAL CO., INC.) was added for dissolution. Next, the chloroform was distilled off under reduced pressure to thereby form a thin layer of lipids on the interior wall of the flask. To this thin layer of lipids, 5 ml of PBS (20 mM phosphate, 150 mM NaCl, pH 7.4) was added for suspension to obtain a crude liposome (DPPC concentration: 10 mM) dispersion. Subsequently, this liposome dispersion was repeatedly extruded from a polycarbonate membrane (Avanti POLAR LIPID, INC.) having a pore size of 50 nm using an extruder (The Mini-Extruder, Avanti POLAR LIPID, INC.) to produce liposomes with an appropriate particle size.

(3) Binding of antibody to liposome

The hTRA-8 Fab' (1 mg/ml, 0.44 ml) and the liposome dispersion (10 mM DPPC, 0.4 ml) were mixed at a ratio of hTRA-8 Fab':DSPE-PEG2000-Mal=1:5 (molar ratio) to react the thiol group of the antibody with the terminal maleimide group of the PEG chain on the liposome. Antibody-unbound maleimide groups were inactivated by the addition of 4 $\mu$l of 100 mM mercaptoethanol (Wako Pure Chemical Industries, Ltd.) which was 10 equivalents with respect to DSPE-PEG2000-Mal and subsequent reaction at room temperature for 30 minutes. Unreacted hTRA-8 Fab' was removed by ultrafiltration through VIVASPIN (Sartorius Mechatronics, molecular cutoff: 300,000) to obtain the present immunoliposome in which the cysteine residue on the antibody was bound with the end of PEG on the liposome (liposome composition: No. 54 of Table 2, antibody concentration: 354 $\mu$g/ml, phospholipid concentration: 4.54 mM, antibody density: 0.085 mol%, average particle size: 106.5$\pm$33.4 nm (PBS)).

Example 55

(1) Preparation of hTRA-8 Fab'

hTRA-8 Fab' was prepared just before use in the same way as in paragraph (1) of Example 42.

(2) Preparation of liposome

36.7 mg, 12.89 mg, and 2.99 mg (molar ratio 100:66:1) of L-$\alpha$-dipalmitoylphosphatidylcholine (hereinafter, referred to as DPPC; NOF CORPORATION, COATSOME MC-6060), cholesterol (Sigma-Aldrich, Inc.), and poly(ethylene glycol)succinyl distearoylphosphatidylethanolamine having a maleimide group at the end of polyethylene glycol of approximately 5000 in molecular weight (hereinafter, referred to as DSPE-PEG5000-Mal; NOF CORPORATION, SUNBRIGHT DSPE-050MA) were weighed, respectively, in an eggplant-shaped flask, to which 3 ml of chloroform (KANTO CHEMICAL CO., INC.) was added for dissolution. Next, the chloroform was distilled off under reduced pressure to thereby form a thin layer of lipids on the interior wall of the flask. To this thin layer of lipids, 5 ml of PBS (20 mM phosphate, 150 mM NaCl, pH 7.4) was added for suspension to obtain a crude liposome (DPPC concentration: 10 mM) dispersion. Subsequently, this liposome dispersion was repeatedly extruded from a polycarbonate membrane (Avanti POLAR LIPID, INC.) having a pore size of 50 nm using an extruder (The Mini-Extruder, Avanti POLAR LIPID, INC.) to produce liposomes with an appropriate particle size.

(3) Binding of antibody to liposome

The hTRA-8 Fab' (1 mg/ml, 0.66 ml) and the liposome dispersion (10 mM DPPC, 0.6 ml) were mixed at a ratio of hTRA-8 Fab':DSPE-PEG5000-Mal=1:5 (molar ratio) to react the thiol group of the antibody with the terminal maleimide group of the PEG chain on the liposome. Antibody-unbound maleimide groups were inactivated by the addition of 6 $\mu$l of 100 mM mercaptoethanol (Wako Pure Chemical Industries, Ltd.) which was 10 equivalents with respect to DSPE-PEG5000-Mal and subsequent reaction at room temperature for 30 minutes. Unreacted hTRA-8 Fab' was removed by ultrafiltration through VIVASPIN (Sartorius Mechatronics, molecular cutoff: 300,000) to obtain the present immunoliposome in which the cysteine residue on the antibody was bound with the end of PEG on the liposome (liposome composition: No. 55 of Table 2, antibody concentration: 128 $\mu$g/ml, phospholipid concentration: 3.02 mM, antibody density: 0.046 mol%, particle size: 84.9$\pm$36.9 nm (PBS)).

Example 56

(1) Preparation of hTRA-8 Fab'

hTRA-8 Fab' was prepared just before use in the same way as in paragraph (1) of Example 42.

(2) Preparation of liposome

36.7 mg, 4.84 mg, and 2.17 mg (molar ratio 100:25:1) of L-$\alpha$-dipalmitoylphosphatidylcholine (hereinafter, referred to as DPPC; NOF CORPORATION, COATSOME MC-6060), cholesterol (Sigma-Aldrich, Inc.), and poly(ethylene glycol)succinyl distearoylphosphatidylethanolamine having a maleimide group at the end of polyethylene glycol of approximately 3400 in molecular weight (hereinafter, referred to as DSPE-PEG3400-Mal; NOF CORPORATION, SUNBRIGHT DSPE-034MA) were weighed, respectively, in an eggplant-shaped flask, to which 3 ml of chloroform (KANTO CHEMICAL CO., INC.) was added for dissolution. Next, the chloroform was distilled off under reduced pressure to thereby form a thin layer of lipids on the interior wall of the flask. To this thin layer of lipids, 5 ml of PBS (20 mM phosphate, 150 mM NaCl, pH 7.4) was added for suspension to obtain a crude liposome (DPPC concentration: 10 mM) dispersion. Subsequently, this liposome dispersion was repeatedly extruded from a polycarbonate membrane (Avanti POLAR LIPID, INC.) having a pore size of 50 nm using an extruder (The Mini-Extruder, Avanti POLAR LIPID, INC.) to produce liposomes with an appropriate particle size.

(3) Binding of antibody to liposome

The hTRA-8 Fab' (1 mg/ml, 0.44 ml) and the liposome dispersion (10 mM DPPC, 0.4 ml) were mixed at a ratio of hTRA-8 Fab':DSPE-PEG3400-Mal=1:5 (molar ratio) to react the thiol group of the antibody with the terminal maleimide group of the PEG chain on the liposome. Antibody-unbound maleimide groups were inactivated by the addition of 4 $\mu$l of 100 mM mercaptoethanol (Wako Pure Chemical Industries, Ltd.) which was 10 equivalents with respect to DSPE-PEG3400-Mal and subsequent reaction at room temperature for 30 minutes. Unreacted hTRA-8 Fab' was removed by ultrafiltration through VIVASPIN (Sartorius Mechatronics, molecular cutoff: 300,000) to obtain the present immunoliposome in which the cysteine residue on the antibody was bound with the end of PEG on the liposome (liposome composition: No. 56 of Table 2, antibody concentration: 214 $\mu$g/ml, phospholipid concentration: 1.95 mM, antibody density: 0.156 mol%, particle size: 78.9$\pm$41.4 nm (PBS)).

Example 57

(1) Preparation of hTRA-8 Fab'

hTRA-8 Fab' was prepared just before use in the same way as in paragraph (1) of Example 42.

(2) Preparation of liposome

36.7 mg, 19.35 mg, and 2.17 mg (molar ratio 100:100:1) of L-α-dipalmitoylphosphatidylcholine (hereinafter, referred to as DPPC; NOF CORPORATION, COATSOME MC-6060), cholesterol (Sigma-Aldrich, Inc.), and poly(ethylene glycol)succinyl distearoylphosphatidylethanolamine having a maleimide group at the end of polyethylene glycol of approximately 3400 in molecular weight (hereinafter, referred to as DSPE-PEG3400-Mal; NOF CORPORATION, SUNBRIGHT DSPE-034MA) were weighed, respectively, in an eggplant-shaped flask, to which 3 ml of chloroform (KANTO CHEMICAL CO., INC.) was added for dissolution. Next, the chloroform was distilled off under reduced pressure to thereby form a thin layer of lipids on the interior wall of the flask. To this thin layer of lipids, 5 ml of PBS (20 mM phosphate, 150 mM NaCl, pH 7.4) was added for suspension to obtain a crude liposome (DPPC concentration: 10 mM) dispersion. Subsequently, this liposome dispersion was repeatedly extruded from a polycarbonate membrane (Avanti POLAR LIPID, INC.) having a pore size of 50 nm using an extruder (The Mini-Extruder, Avanti POLAR LIPID, INC.) to produce liposomes with an appropriate particle size.

(3) Binding of antibody to liposome

The binding was performed in the same way as in paragraph (3) of Example 56 to obtain the present immunoliposome in which the cysteine residue on the antibody was bound with the end of PEG on the liposome (liposome composition: No. 57 of Table 2, antibody concentration: 456 μg/ml, phospholipid concentration: 4.88 mM, antibody density: 0.084 mol%, particle size: 89.1±31.9 nm (PBS)).

Example 58

(1) Preparation of hTRA-8 scFv

hTRA-8 scFv 5B (buffer: 50 mM Tris-HCl (pH 7.5), 500 mM NaCl, 10% glycerol, 10 mM CHAPS, 1 mM DTT) was buffer-replaced with a phosphate buffer and 500 mM NaCl, pH 7.4 by gel filtration purification (column: GE HEALTHCARE INC., PD-10 Desalting column).

(2) Preparation of liposome

22 mg, 7.73 mg, and 1.7 mg (molar ratio 100:66:1.5) of L-α-dipalmitoylphosphatidylcholine (hereinafter, referred to as DPPC; NOF CORPORATION, COATSOME MC-6060), cholesterol (Sigma-Aldrich, Inc.), and poly(ethylene glycol) succinyl distearoylphosphatidylethanolamine having a maleimide group at the end of polyethylene glycol of approximately 3400 in molecular weight (hereinafter, referred to as DSPE-PEG3400-Mal; NOF CORPORATION, SUNBRIGHT DSPE-034MA) were weighed, respectively, in an eggplant-shaped flask, to which 3 ml of chloroform (KANTO CHEMICAL CO., INC.) was added for dissolution. Next, the chloroform was distilled off under reduced pressure to thereby form a thin layer of lipids on the interior wall of the flask. To this thin layer of lipids, 3 ml of a phosphate buffer and 500 mM NaCl, pH 7.4 were added for suspension to obtain a crude liposome (DPPC concentration: 10 mM) dispersion. Subsequently, this liposome dispersion was repeatedly extruded from a polycarbonate membrane (Avanti POLAR LIPID, INC.) having a pore size of 100 nm using an extruder (The Mini-Extruder, Avanti POLAR LIPID, INC.) to produce liposomes with an appropriate particle size.

(3) Binding reaction of antibody with liposome

The hTRA-8 scFv (125 μg/ml, 800 μl) and the liposome dispersion (10 mM DPPC, 200 μl) were mixed at a ratio of hTRA-8 scFv:DSPE-PEG3400-Mal=1:7.5 (molar ratio) to react the thiol group of the antibody with the terminal maleimide group of the PEG chain on the liposome. Antibody-unbound maleimide groups were inactivated by the addition of 2 μl of 100 mM mercaptoethanol (Wako Pure Chemical Industries, Ltd.) which was 10 equivalents with respect to DSPE-PEG3400-Mal and subsequent reaction at room temperature for 30 minutes. Unreacted hTRA-8 scFv was removed by gel filtration chromatography (AKTA explorer 10S (GE HEALTHCARE INC.); column (GE HEALTHCARE INC., HiLoad Superdex 200 16/60 prep grade); HEPES buffer (pH 7.4); 4°C; 2 ml/min; detection wavelength: UV 280 nm) to separate immunoliposome fractions (36-48 ml fractions). Ultrafiltration concentration was performed using Amicon Ultra (MILLIPORE INC., molecular cutoff: 50,000) to obtain the present immunoliposome in which the cysteine residue on the antibody was bound with the end of PEG on the liposome (liposome composition: No. 58 of Table 2, antibody concentration: 14 μg/ml, phospholipid concentration: 1.3 mM, antibody density: 0.025 mol%, average particle size: 91±16 nm (phosphate buffer, 500 mM NaCl, pH 7.4)).

Example 59

(1) Thiolation of Recombinant soluble TRAIL

Recombinant soluble TRAIL (Peprotech Inc.) (hereinafter, referred to as rsTRAIL(P)) (150 μg) was dissolved in 10 mM sodium phosphate, 140 mM NaCl, and 1 mM DTPA, pH 7.4 to adjust the concentration to 2 mg/mL. 400 μM Traut's Reagent (2-Iminothiolane·HCl, Pierce Biotechnology, Inc.) in dimethyl sulfoxide was added thereto at a molar ratio of rsTRAIL(P):Traut's Reagent=1:1, followed by reaction at room temperature for 90 min. Then, the Traut's Reagent was removed by ultrafiltration (1000-fold dilution) using Microcon (MILLIPORE INC., molecular cutoff: 3,000; PBS, pH 7.4) to thiolate the amino groups of some lysine residues in the rsTRAIL(P).

(2) Preparation of liposome

A liposome dispersion was prepared just before use in the same way as in paragraph (2) of Example 1.

(3) Binding reaction of rsTRAIL(P) with liposome

The rsTRAIL(P) was bound to the liposome as follows: the rsTRAIL(P) (1 mg/ml, 19.6 μl) and the liposome dispersion (10 mM DPPC, 50 μl) were mixed at a ratio of rsTRAIL(P):DSPE-PEG3400-Mal=1:15 (molar ratio) to react the thiol group of the rsTRAIL(P) with the terminal maleimide group of the PEG chain on the liposome. rsTRAIL(P)-unbound maleimide groups were inactivated by the addition of 5 μl of 10 mM mercaptoethanol (Wako Pure Chemical Industries, Ltd.) which was 10 equivalents with respect to DSPE-PEG3400-Mal and subsequent reaction at room temperature for 30 minutes. Unreacted rsTRAIL(P) was removed by ultrafiltration through VIVASPIN (Sartorius Mechatronics, molecular cutoff: 300,000) repeated until the stock solution was diluted 1000-fold. As a result, the present immunoliposome in which the lysine residue on the rsTRAIL(P) was bound with the end of PEG on the liposome was obtained (liposome composition: No. 59 of Table 2, protein concentration: 6 μg/ml, phospholipid concentration: 1.8 mM, rsTRAIL (P) density: 0.011 mol%, average particle size: 62 nm (PBS, pH 7.4)).

Example 60

(1) Thiolation of rsTRAIL(P)

The amino groups of some lysine residues in rsTRAIL(P) were thiolated in the same way as in Example 59.

(2) Preparation of liposome

A liposome dispersion was prepared just before use in the same way as in paragraph (2) of Example 1.

(3) Binding reaction of rsTRAIL(P) with liposome

The rsTRAIL was bound to the liposome as follows: the rsTRAIL(P) (1 mg/ml, 58.8 μl) and the liposome dispersion (10 mM DPPC, 50 μl) were mixed at a ratio of rsTRAIL(P):DSPE-PEG3400-Mal=1:5 (molar ratio) to react the thiol group of the rsTRAIL(P) with the terminal maleimide group of the PEG chain on the liposome. rsTRAIL(P)-unbound maleimide groups were inactivated by the addition of 5 μl of 10 mM mercaptoethanol (Wako Pure Chemical Industries, Ltd.) which was 10 equivalents with respect to DSPE-PEG3400-Mal and subsequent reaction at room temperature for 30 minutes. Unreacted rsTRAIL(P) was removed by ultrafiltration through VIVASPIN (Sartorius Mechatronics, molecular cutoff: 300,000) repeated until the stock solution was diluted 1000-fold. As a result, the present immunoliposome in which the lysine residue on the rsTRAIL(P) was bound with the end of PEG on the liposome was obtained (liposome composition: No. 60 of Table 2, protein concentration: 9 μg/ml, phospholipid concentration: 1.1 mM, rsTRAIL (P) density: 0.024 mol%, average particle size: 73 nm (PBS, pH 7.4)).

Example 61

(1) Thiolation of Recombinant soluble TRAIL

Recombinant soluble TRAIL (Biodesign) (hereinafter, referred to as rsTRAIL(B)) (200 μg) was dissolved in 10 mM sodium phosphate, 140 mM NaCl, and 1 mM DTPA, pH 7.4 to adjust the concentration to 2 mg/mL. 400 μM Traut's Reagent (2-Iminothiolane·HCl, Pierce Biotechnology, Inc.) in dimethyl sulfoxide was added thereto at a molar ratio of rsTRAIL(B):Traut's Reagent=1:1, followed by reaction at room temperature for 90 min. Then, the Traut's Reagent was removed by ultrafiltration (1000-fold dilution) using Microcon (MILLIPORE INC., molecular cutoff: 3,000; PBS, pH 7.4) to thiolate the amino groups of some lysine residues in the rsTRAIL(B).

(2) Preparation of liposome

A liposome dispersion was prepared just before use in the same way as in paragraph (2) of Example 1.

(3) Binding reaction of rsTRAIL(B) with liposome

The rsTRAIL(B) was bound to the liposome as follows: the rsTRAIL(B) (1 mg/ml, 47 μl) and the liposome dispersion (10 mM DPPC, 40 μl) were mixed at a ratio of rsTRAIL:DSPE-PEG3400-Mal=1:15 (molar ratio) to react the thiol group of the rsTRAIL(B) with the terminal maleimide group of the PEG chain on the liposome. rsTRAIL(B)-unbound maleimide groups were inactivated by the addition of 4 μl of 10 mM mercaptoethanol (Wako Pure Chemical Industries, Ltd.) which was 10 equivalents with respect to DSPE-PEG3400-Mal and subsequent reaction at room temperature for 30 minutes. Unreacted rsTRAIL(B) was removed by ultrafiltration through VIVASPIN (Sartorius Mechatronics, molecular cutoff: 300,000) repeated until the stock solution was diluted 1000-fold. As a result, the present immunoliposome in which the lysine residue on the rsTRAIL(B) was bound with the end of PEG on the liposome was obtained (liposome composition: No. 61 of Table 2, protein concentration: 4 μg/ml, phospholipid concentration: 1.2 mM, rsTRAIL density: 0.009 mol%, average particle size: 63 nm (PBS, pH 7.4)).

Example 62

(1) Thiolation of Mouse Anti-hDR4 Antibody (m2E12)

4 mM Traut's Reagent (2-Iminothiolane·HCl, Pierce Biotechnology, Inc.) was added to a mouse anti-human DR4 antibody m2E12 (antibody concentration: 1.8 mg/ml, 20 mM phosphate buffer, 150 mM NaCl, 1 mM DTPA, pH 8.0) produced by hybridoma m2E12 described in the pamphlet of WO2003/37913, at a molar ratio of m2E12:Traut's Reagent=1:4, followed by reaction at room temperature for 90 min. Then, the Traut's Reagent was removed by gel filtration chromatography (column: GE HEALTHCARE INC., PD-10 Desalting column; PBS, pH 7.4) to thiolate the amino groups of some lysine residues in the m2E12.

(2) Preparation of liposome

A liposome dispersion was prepared just before use in the same way as in paragraph (2) of Example 1.

(3) Binding reaction of antibody with liposome

The m2E12 (0.77 mg/ml, 97.4 μl) and the liposome dispersion (2 mM DPPC, 1000 μl) were mixed at a ratio of m2E12:DSPE-PEG3400-Mal=1:40 (molar ratio) to react the thiol group of the antibody with the terminal maleimide group of the PEG chain on the liposome. Antibody-unbound maleimide groups were inactivated by the addition of 2 μl of 100 mM mercaptoethanol (Wako Pure Chemical Industries, Ltd.) which was 10 equivalents with respect to DSPE-PEG3400-Mal and subsequent reaction at room temperature for 30 minutes. Unreacted m2E12 was removed by ultrafiltration through VIVASPIN (Sartorius Mechatronics, molecular cutoff: 300,000) repeated until the stock solution was diluted 1000-fold. As a result, the present immunoliposome in which the lysine residue on the antibody was bound with the end of PEG on the liposome was obtained (liposome composition: No. 62 of Table 2, antibody concentration: 25 μg/ml, phospholipid concentration: 1.7 mM, antibody density: 0.0059 mol%, average particle size: 68±25 nm (PBS, pH 7.4)).

Example 63

The amino groups of some lysine residues in m2E12 were thiolated in the same way as in Example 62. Furthermore, a liposome dispersion was prepared just before use in the same way as in paragraph (2) of Example 1.
The binding reaction of the antibody with the liposome was performed by the following steps: the m2E12 (0.77 mg/ml, 195 μl) and the liposome dispersion (2 mM DPPC, 1000 μl) were mixed at a ratio of m2E12 Fullbody:DSPE-PEG3400-Mal=1:20 (molar ratio) to react the thiol group of the antibody with the terminal maleimide group of the PEG chain on the liposome. Antibody-unbound maleimide groups were inactivated by the addition of 2 μl of 100 mM mercaptoethanol (Wako Pure Chemical Industries, Ltd.) which was 10 equivalents with respect to DSPE-PEG3400-Mal and subsequent reaction at room temperature for 30 minutes. Unreacted m2E12 was removed by ultrafiltration through VIVASPIN (Sartorius Mechatronics, molecular cutoff: 300,000) repeated until the stock solution was diluted 1000-fold. As a result, the present immunoliposome in which the lysine residue on the antibody was bound with the end of PEG on the liposome was obtained (liposome composition: No. 63 of Table 2, antibody concentration: 43 μg/ml, phospholipid concentration: 1.4 mM, antibody density: 0.0122 mol%, average particle size: 53±24 nm (PBS, pH 7.4)).

Example 64

The amino groups of some lysine residues in m2E12 were thiolated in the same way as in Example 62. Furthermore, a liposome dispersion was prepared just before use in the same way as in paragraph (2) of Example 1.
The binding reaction of the antibody with the liposome was performed by the following steps: the m2E12 (0.77 mg/ml, 390 μl) and the liposome dispersion (2 mM DPPC, 1000 μl) were mixed at a ratio of m2E12:DSPE-PEG3400-Mal=1:10 (molar ratio) to react the thiol group of the antibody with the terminal maleimide group of the PEG chain on the liposome. Antibody-unbound maleimide groups were inactivated by the addition of 2 μl of 100 mM mercaptoethanol (Wako Pure Chemical Industries, Ltd.) which was 10 equivalents with respect to DSPE-PEG3400-Mal and subsequent reaction at room temperature for 30 minutes. Unreacted m2E12 was removed by ultrafiltration through VIVASPIN (Sartorius Mechatronics, molecular cutoff: 300,000) repeated until the stock solution was diluted 1000-fold. As a result, the present immunoliposome in which the lysine residue on the antibody was bound with the end of PEG on the liposome was obtained (liposome composition: No. 64 of Table 2, antibody concentration: 72 μg/ml, phospholipid concentration: 1.2 mM, antibody density: 0.0247 mol%, average particle size: 63±26 nm (PBS, pH 7.4)).

Example 65

The amino groups of some lysine residues in m2E12 were thiolated in the same way as in Example 62. Furthermore, a liposome dispersion was prepared just before use in the same way as in paragraph (2) of Example 1.
The binding reaction of the antibody with the liposome was performed by the following steps: the m2E12 (0.77 mg/ml, 780 μl) and the liposome dispersion (2 mM DPPC, 1000 μl) were mixed at a ratio of m2E12:DSPE-PEG3400-Mal=1:5 (molar ratio) to react the thiol group of the antibody with the terminal maleimide group of the PEG chain on the liposome. Antibody-unbound maleimide groups were inactivated by the addition of 2 μl of 100 mM mercaptoethanol (Wako Pure Chemical Industries, Ltd.) which was 10 equivalents with respect to DSPE-PEG3400-Mal and subsequent reaction at room temperature for 30 minutes. Unreacted m2E12 was removed by ultrafiltration through VIVASPIN (Sartorius Mechatronics, molecular cutoff: 300,000) repeated until the stock solution was diluted 1000-fold. As a result, the present immunoliposome in which the lysine residue on the antibody was bound with the end of PEG on the liposome was obtained (liposome composition: No. 65 of Table 2, antibody concentration: 159 μg/ml, phospholipid concentration: 1.2 mM, antibody density: 0.0510 mol%, average particle size: 65±27 nm (PBS, pH 7.4)).

Example 66

The amino groups of some lysine residues in m2E12 were thiolated in the same way as in Example 62. Furthermore, a liposome dispersion was prepared just before use in the same way as in paragraph (2) of Example 1. The binding reaction of the antibody with the liposome was performed by the following steps: the m2E12 (0.77 mg/ml, 1950 μl) and the liposome dispersion (2 mM DPPC, 1000 μl) were mixed at a ratio of m2E12:DSPE-PEG3400-Mal=1:2 (molar ratio) to react the thiol group of the antibody with the terminal maleimide group of the PEG chain on the liposome. Antibody-unbound maleimide groups were inactivated by the addition of 2 μl of 100 mM mercaptoethanol (Wako Pure Chemical Industries, Ltd.) which was 10 equivalents with respect to DSPE-PEG3400-Mal and subsequent reaction at room temperature for 30 minutes. Unreacted m2E12 was removed by ultrafiltration through VIVASPIN (Sartorius Mechatronics, molecular cutoff: 300,000) repeated until the stock solution was diluted 1000-fold. As a result, the present immunoliposome in which the lysine residue on the antibody was bound with the end of PEG on the liposome was obtained (liposome composition: No. 66 of Table 2, antibody concentration: 281 μg/ml, phospholipid concentration: 0.86 mM, antibody density: 0.1296 mol%, average particle size: 76±29 nm (PBS, pH 7.4)).

Example 67

(1) Preparation of hTRA-8 Fab'

hTRA-8 Fab' was prepared just before use in the same way as in paragraph (1) of Example 42.

(2) Preparation of liposome

45.12 mg, 12.89 mg, and 2.17 mg (molar ratio 100:66:1) of didocosanoylphosphatidylcholine (hereinafter, referred to as DDOPC; Sigma-Aldrich, Inc.), cholesterol (Sigma-Aldrich, Inc.), and poly(ethylene glycol)succinyl distearoylphosphatidylethanolamine having a maleimide group at the end of polyethylene glycol of approximately 3400 in molecular weight (hereinafter, referred to as DSPE-PEG3400-Mal; NOF CORPORATION, SUNBRIGHT DSPE-034MA) were weighed, respectively, in an eggplant-shaped flask, to which 3 ml of chloroform (KANTO CHEMICAL CO., INC.) was added for dissolution. Next, the chloroform was distilled off under reduced pressure to thereby form a thin layer of lipids on the interior wall of the flask. To this thin layer of lipids, 5 ml of PBS (20 mM phosphate, 150 mM NaCl, pH 7.4) was added for suspension to obtain a crude liposome (DDOPC concentration: 10 mM) dispersion. Subsequently, this liposome dispersion was repeatedly extruded from a polycarbonate membrane (Avanti POLAR LIPID, INC.) having a pore size of 800 nm using an extruder (The Mini-Extruder, Avanti POLAR LIPID, INC.) to produce liposomes with an appropriate particle size.

(3) Binding of antibody to liposome

The hTRA-8 Fab' (1 mg/ml, 0.66 ml) and the liposome dispersion (10 mM DDOPC, 0.6 ml) were mixed at a ratio of hTRA-8 Fab':DSPE-PEG3400-Mal=1:5 (molar ratio) to react the thiol group of the antibody with the terminal maleimide group of the PEG chain on the liposome. Antibody-unbound maleimide groups were inactivated by the addition of 6 μl of 100 mM mercaptoethanol (Wako Pure Chemical Industries, Ltd.) which was 10 equivalents with respect to DSPE-PEG3400-Mal and subsequent reaction at room temperature for 30 minutes. Unreacted hTRA-8 Fab' was removed by ultrafiltration through VIVASPIN (Sartorius Mechatronics, molecular cutoff: 300,000) to obtain the present immunoliposome in which the cysteine residue on the antibody was bound with the end of PEG on the liposome (liposome composition: No. 67 of Table 2, antibody concentration: 77 μg/ml, phospholipid concentration: 0.15 mM, antibody density: 0.556 mol%, average particle size: 896.8±70.8 nm (PBS)).

Example 68

(1) Preparation of hTRA-8 Fab'

hTRA-8 Fab' was prepared just before use in the same way as in paragraph (1) of Example 42.

(2) Preparation of liposome

44.92 mg, 12.89 mg, and 2.17 mg (molar ratio 100:66:1) of dierucoylphosphatidylcholine (hereinafter, referred to as DEPC; NOF CORPORATION, COATSOME MC-2121AL), cholesterol (Sigma-Aldrich, Inc.), and poly(ethylene glycol)succinyl distearoylphosphatidylethanolamine having a maleimide group at the end of polyethylene glycol of approximately 3400 in molecular weight (hereinafter, referred to as DSPE-PEG3400-Mal; NOF CORPORATION, SUNBRIGHT DSPE-034MA) were weighed, respectively, in an eggplant-shaped flask, to which 3 ml of chloroform (KANTO CHEMICAL CO., INC.) was added for dissolution. Next, the chloroform was distilled off under reduced pressure to thereby form a thin layer of lipids on the interior wall of the flask. To this thin layer of lipids, 5 ml of PBS (20 mM phosphate, 150 mM NaCl, pH 7.4) was added for suspension to obtain a crude liposome (DEPC concentration: 10 mM) dispersion. Subsequently, this liposome dispersion was repeatedly extruded from a polycarbonate membrane (Avanti POLAR LIPID, INC.) having a pore size of 100 nm using an extruder (The Mini-Extruder, Avanti POLAR LIPID, INC.) to produce liposomes with an appropriate particle size.

(3) Binding of antibody to liposome

The hTRA-8 Fab' (1 mg/ml, 0.66 ml) and the liposome dispersion (10 mM DEPC, 0.6 ml) were mixed at a ratio of hTRA-8 Fab':DSPE-PEG3400-Mal=1:5 (molar ratio) to react the thiol group of the antibody with the terminal maleimide group of the PEG chain on the liposome. Antibody-unbound maleimide groups were inactivated by the addition of 6 μl of 100 mM mercaptoethanol (Wako Pure Chemical Industries, Ltd.) which was 10 equivalents with respect to DSPE-PEG3400-Mal and subsequent reaction at room temperature for 30 minutes. Unreacted hTRA-8 Fab' was removed by ultrafiltration through VIVASPIN (Sartorius Mechatronics, molecular cutoff: 300,000) to obtain the present immunoliposome in which the cysteine residue on the antibody was bound with the end of PEG on the liposome (liposome composition: No. 68 of Table 2, antibody concentration: 393 μg/ml, phospholipid concentration: 5.64 mM, antibody density: 0.076 mol%, average particle size: 91.7±33.7 nm (PBS)).

Example 69

(1) Thiolation of hTRA-8 F(ab')$_2$

The amino groups of some lysine residues in F(ab')$_2$ were thiolated in the same way as in paragraph (1) of Example 43. Furthermore, a liposome dispersion was prepared just before use in the same way as in paragraph (2) of Example 41.

The binding reaction of the antibody with the liposome was performed by the following steps: the hTRA-8 F(ab')$_2$ (1 mg/ml, 2.686 ml) and the liposome dispersion (10 mM DPPC, 11 ml) were mixed at a ratio of hTRA-8 F(ab')$_2$:DSPE-PEG2000-Mal=1:45 (molar ratio) to react the thiol group of the antibody with the terminal maleimide group of the PEG chain on the liposome. Antibody-unbound maleimide groups were inactivated by the addition of 110 $\mu$l of 100 mM mercaptoethanol (Wako Pure Chemical Industries, Ltd.) which was 10 equivalents with respect to DSPE-PEG2000-Mal and subsequent reaction at room temperature for 30 minutes. Unreacted hTRA-8 F(ab')$_2$ was removed by ultrafiltration (LabScale TFF System (MILLIPORE INC.)) through a polyethersulfone membrane (Pellicon XL 50, Biomax 300, molecular cutoff: 300,000, MILLIPORE INC.) to obtain the present immunoliposome in which the lysine residue on the antibody was bound with the end of PEG on the liposome (liposome composition: No. 69 of Table 2, antibody concentration: 128 $\mu$g/ml, phospholipid concentration: 10.08 mM, antibody density: 0.0069 mol%, average particle size: 87.6$\pm$18.1 nm (PBS)).

Example 70

(1) Thiolation of m2E12 Fullbody and hTRA-8 Fullbody

4 mM Traut's Reagent (2-Iminothiolane·HCl, Pierce Biotechnology, Inc.) was added to m2E12 Fullbody and hTRA-8 Fullbody (antibody concentration: 1.8 mg/ml, 20 mM phosphate buffer, 150 mM NaCl, 2 mM DTPA, pH 8.0) at a molar ratio of each antibody (m2E12 or hTRA-8):Traut's Reagent=1:4, followed by reaction at room temperature for 90 min. Then, the Traut's Reagent was removed by gel filtration chromatography (column: GE HEALTHCARE INC., PD-10 Desalting column; PBS, pH 7.4) to thiolate the amino groups of some lysine residues in the m2E12 Fullbody and the hTRA-8 Fullbody.

(2) Preparation of liposome

44 mg, 15.4 mg, and 2.6 mg (molar ratio 100:66:1) of L-$\alpha$-dipalmitoylphosphatidylcholine (hereinafter, referred to as DPPC; NOF CORPORATION, COATSOME MC-6060), cholesterol (Sigma-Aldrich, Inc.), and poly(ethylene glycol) succinyl distearoylphosphatidylethanolamine having a maleimide group at the end of polyethylene glycol of approximately 3400 in molecular weight (hereinafter, referred to as DSPE-PEG3400-Mal; NOF CORPORATION, SUNBRIGHT DSPE-034MA) were weighed, respectively, in an eggplant-shaped flask, to which 6 ml of chloroform (KANTO CHEMICAL CO., INC.) was added for dissolution. Next, the chloroform was distilled off under reduced pressure to thereby form a thin layer of lipids on the interior wall of the flask. To this thin layer of lipids, 6 ml of PBS (20 mM phosphate, 150 mM NaCl, pH 7.4) was added for suspension to obtain a crude liposome (DPPC concentration: 10 mM) dispersion. Subsequently, this liposome dispersion was repeatedly extruded from a polycarbonate membrane (Avanti POLAR LIPID, INC.) having a pore size of 100 nm using an extruder (The Mini-Extruder, Avanti POLAR LIPID, INC.) to produce liposomes with an appropriate particle size. The concentration of this 10 mM DPPC dispersion was adjusted to 2 mM with PBS, pH 7.4.

(3) Binding reaction of antibody with liposome

The m2E12 Fullbody (0.77 mg/ml, 195 $\mu$l) and the liposome dispersion (2 mM DPPC, 1000 $\mu$l) were mixed at a ratio of m2E12 Fullbody:DSPE-PEG3400-Mal=1:20 (molar ratio) and reacted at room temperature for 2 hr to bind the thiol group of the antibody with the terminal maleimide group of the PEG chain on the liposome. Unreacted m2E12 was removed by ultrafiltration through VIVASPIN (Sartorius Mechatronics, molecular cutoff: 300,000) repeated until the stock solution was diluted 1000-fold, to adjust the volume of the dispersion to 1 ml. At this stage, an aliquot of the dispersion was collected, and the protein level of the liposome-bound m2E12 and the lipid level of the liposome were quantified. Subsequently, the hTRA-8 Fullbody (0.77 mg/ml, 584 $\mu$l) and the resulting liposome dispersion were mixed at a ratio of hTRA-8 Fullbody:DSPE-PEG3400-Mal=3:20 (molar ratio) and reacted at room temperature for 2 hr to bind the thiol group of the antibody with the terminal maleimide group of the PEG chain on the liposome. Antibody-unbound maleimide groups were inactivated by the addition of 2 $\mu$l of 100 mM mercaptoethanol (Wako Pure Chemical Industries, Ltd.) which was 10 equivalents with respect to DSPE-PEG3400-Mal and subsequent reaction at room temperature for 30 minutes. Unreacted hTRA-8 Fullbody was removed by ultrafiltration through VIVASPIN (Sartorius Mechatronics, molecular cutoff:

300,000) repeated until the stock solution was diluted 1000-fold, to obtain the present immunoliposome in which the lysine residues on the two antibodies were bound with the end of PEG on the liposome. The total antibody concentration and lipid concentration of the present immunoliposome dispersion were quantified, and each antibody level was calculated from the total antibody level and the m2E12 antibody level (liposome composition: No. 70 of Table 2, antibody concentration: 40 μg/ml (m2E12) and 139 μg/ml (hTRA-8), antibody ratio of m2E12:hTRA-8=22:78, phospholipid concentration: 1.15 mM, total antibody density: 0.062 mol% (PBS)).

Example 71

m2E12 Fullbody and hTRA-8 Fullbody were subjected to thiolation in the same way as in paragraph (1) of Example 70. Furthermore, a liposome dispersion was prepared just before use in the same way as in paragraph (2) of Example 70.

(3) Binding reaction of antibody with liposome

The hTRA-8 Fullbody (0.77 mg/ml, 195 μl) and the liposome dispersion (2 mM DPPC, 1000 μl) were mixed at a ratio of hTRA-8 Fullbody:DSPE-PEG3400-Mal=1:20 (molar ratio) and reacted at room temperature for 2 hr to bind the thiol group of the antibody with the terminal maleimide group of the PEG chain on the liposome. Unreacted hTRA-8 was removed by ultrafiltration through VIVASPIN (Sartorius Mechatronics, molecular cutoff: 300,000) repeated until the stock solution was diluted 1000-fold, to adjust the volume of the dispersion to 1 ml. At this stage, an aliquot of the dispersion was collected, and the protein level of the liposome-bound hTRA-8 and the lipid level of the liposome were quantified. Subsequently, the m2E12 Fullbody (0.77 mg/ml, 584 μl) and the resulting liposome dispersion were mixed at a ratio of m2E12 Fullbody:DSPE-PEG3400-Mal=3:20 (molar ratio) and reacted at room temperature for 2 hr to bind the thiol group of the antibody with the terminal maleimide group of the PEG chain on the liposome. Antibody-unbound maleimide groups were inactivated by the addition of 2 μl of 100 mM mercaptoethanol (Wako Pure Chemical Industries, Ltd.) which was 10 equivalents with respect to DSPE-PEG3400-Mal and subsequent reaction at room temperature for 30 minutes. Unreacted m2E12 Fullbody was removed by ultrafiltration through VIVASPIN (Sartorius Mechatronics, molecular cutoff: 300,000) repeated until the stock solution was diluted 1000-fold, to obtain the present immunoliposome in which the lysine residues on the two antibodies were bound with the end of PEG on the liposome. The total antibody concentration and lipid concentration of the present immunoliposome dispersion were quantified, and each antibody level was calculated from the total antibody level and the hTRA-8 antibody level (liposome composition: No. 71 of Table 2, antibody concentration: 93 μg/ml (m2E12) and 74 μg/ml (hTRA-8), antibody ratio of m2E12:hTRA-8=56:44, phospholipid concentration: 1.14 mM, total antibody density: 0.058 mol% (PBS)).

Example 72

m2E12 Fullbody and hTRA-8 Fullbody were subjected to thiolation in the same way as in paragraph (1) of Example 70. Furthermore, a liposome dispersion was prepared just before use in the same way as in paragraph (2) of Example 70.

(3) Binding reaction of antibody with liposome

The hTRA-8 Fullbody (0.77 mg/ml, 78 μl) and the liposome dispersion (2 mM DPPC, 1000 μl) were mixed at a ratio of hTRA-8 Fullbody:DSPE-PEG3400-Mal=1:50 (molar ratio) and reacted at room temperature for 2 hr to bind the thiol group of the antibody with the terminal maleimide group of the PEG chain on the liposome. Unreacted hTRA-8 was removed by ultrafiltration through VIVASPIN (Sartorius Mechatronics, molecular cutoff: 300,000) repeated until the stock solution was diluted 1000-fold, to adjust the volume of the dispersion to 1 ml. At this stage, an aliquot of the dispersion was collected, and the protein level of the liposome-bound hTRA-8 and the lipid level of the liposome were quantified. Subsequently, the m2E12 Fullbody (0.77 mg/ml, 701 μl) and the resulting liposome dispersion were mixed at a ratio of m2E12 Fullbody:DSPE-PEG3400-Mal=9:50 (molar ratio) and reacted at room temperature for 2 hr to bind the thiol group of the antibody with the terminal maleimide group of the PEG chain on the liposome. Antibody-unbound maleimide groups were inactivated by the addition of 2 μl of 100 mM mercaptoethanol (Wako Pure Chemical Industries, Ltd.) which was 10 equivalents with respect to DSPE-PEG3400-Mal and subsequent reaction at room temperature for 30 minutes. Unreacted m2E12 Fullbody was removed by ultrafiltration through VIVASPIN (Sartorius Mechatronics, molecular cutoff: 300,000) repeated until the stock solution was diluted 1000-fold, to obtain the present immunoliposome in which the lysine residues on the two antibodies were bound with the end of PEG on the liposome. The total antibody concentration and lipid concentration of the present immunoliposome dispersion were quantified, and each antibody level was calculated from the total antibody level and the hTRA-8 antibody level (liposome composition: No. 72 of Table 2, antibody concen-

tration: 155 μg/ml (m2E12) and 39 μg/ml (hTRA-8), antibody ratio of m2E12:hTRA-8=80:20, phospholipid concentration: 1.57 mM, total antibody density: 0.049 mol% (PBS)).

The component composition of the immunoliposomes prepared in Examples 32 to 72 is shown in Table 2.

[Table 2]

| Example No. | Protein | Lipid composition | Antibody density (mol%) |
|---|---|---|---|
| 32 | E | DPPC : Chol : DSPE-PEG$_{3400}$ = 100 : 66 : 1 | 0.031 |
| 33 | E | DPPC : Chol : DSPE-PEG$_{3400}$ = 100 : 66 : 1 | 0.011 |
| 34 | E | DPPC : Chol : DSPE-PEG$_{3400}$ = 100 : 66 : 1 | 0.073 |
| 35 | E | DPPC : Chol : DSPE-PEG$_{3400}$ = 100 : 66 : 1 | 0.109 |
| 36 | G | DPPC : Chol : DSPE-PEG$_{3400}$ = 100 : 66 : 1 | 0.023 |
| 37 | G | DPPC : Chol : DSPE-PEG$_{3400}$ = 100 : 66 : 1 | 0.068 |
| 38 | H | DPPC : Chol : DSPE-PEG$_{3400}$ = 100 : 66 : 1 | 0.029 |
| 39 | H | DPPC : Chol : DSPE-PEG$_{3400}$ = 100 : 66 : 1 | 0.075 |
| 40 | I | DPPC : Chol : DSPE-PEG$_{3400}$ = 100 : 66 : 1 | 0.0073 |
| 41 | B | DPPC : Chol : DSPE-PEG$_{2000}$ = 100 : 66 : 1 | 0.072 |
| 42 | A | DPPC : Chol : DSPE-PEG$_{2000}$ = 100 : 66 : 1 | 0.176 |
| 43 | E | DPPC : Chol : DSPE-PEG$_{2000}$ = 100 : 66 : 1 | 0.062 |
| 44 | A | DDEPC : Chol : DSPE-PEG$_{3400}$ = 100 : 66 : 1 | 0.2 |
| 45 | A | DLPC : Chol : DSPE-PEG$_{3400}$ = 100 : 66 : 1 | 0.135 |
| 46 | A | DDOPC : Chol : DSPE-PEG$_{3400}$ = 100 : 66 : 1 | 0.094 |
| 47 | A | DDEPC : Chol : DSPE-PEG$_{3400}$ = 100 : 66 : 1 | 0.068 |
| 48 | A | DPPC : Chol : DSPE-PEG$_{3400}$ = 100 : 66 : 1 | 0.118 |
| 49 | A | DEPC : Chol : DSPE-PEG$_{3400}$ = 100: 66 : 1 | 0.068 |
| 50 | B | DPPC : Chol : DSPE-PEG$_{3400}$ = 100 : 66 : 3 | 0.027 |
| 51 | B | DPPC : Chol : DSPE-PEG$_{3400}$ = 100 : 66 : 3 | 0.084 |
| 52 | B | DPPC : Chol : DSPE-PEG$_{3400}$ = 100 : 66 : 10 | 0.092 |
| 53 | A | DPPC : Chol : DSPE-PEG$_{3400}$ = 100 : 66 : 3 | 0.297 |
| 54 | A | DPPC : Chol : DSPE-PEG$_{2000}$ = 100 : 66 : 1 | 0.085 |
| 55 | A | DPPC : Chol : DSPE-PEG$_{5000}$ = 100 : 66 : 1 | 0.046 |
| 56 | A | DPPC : Chol : DSPE-PEG$_{3400}$ = 100 : 25 : 1 | 0.158 |
| 57 | A | DPPC : Chol : DSPE-PEG$_{3400}$ = 100 : 100 : 1 | 0.084 |
| 58 | F | DPPC : Chol : DSPE-PEG$_{3400}$ = 100 : 66: 1 | 0.025 |
| 59 | K | DPPC : Chol : DSPE-PEG$_{3400}$ = 100 : 66 : 1 | 0.011 |
| 60 | K | DPPC : Chol : DSPE-PEG$_{3400}$ = 100 : 66 : 1 | 0.024 |
| 61 | L | DPPC : Chol : DSPE-PEG$_{3400}$ = 100 : 66 : 1 | 0.009 |
| 62 | J | DPPC : Chol : DSPE-PEG$_{3400}$ = 100 : 66 : 1 | 0.006 |
| 63 | J | DPPC : Chol : DSPE-PEG$_{3400}$ = 100 : 66 : 1 | 0.012 |
| 64 | J | DPPC : Chol : DSPE-PEG$_{3400}$ = 100 : 66 : 1 | 0.025 |
| 65 | J | DPPC : Chol : DSPE-PEG$_{3400}$ = 100 : 66 : 1 | 0.051 |
| 66 | J | DPPC : Chol : DSPE-PEG$_{3400}$ = 100 : 66 : 1 | 0.13 |
| 67 | A | DDOPC : Chol : DSPE-PEG$_{3400}$ = 100 : 66 : 1 | 0.556 |
| 68 | A | DEPC : Chol : DSPE-PEG$_{3400}$ = 100 : 66 : 1 | 0.076 |
| 69 | E | DPPC : Chol : DSPE-PEG$_{3400}$ = 100: 66 : 1 | 0.0069 |
| 70 | B&J | DPPC : Chol : DSPE-PEG$_{3400}$ = 100 : 66 : 1 | 0.062 (J : B = 22 : 78) |
| 71 | B&J | DPPC : Chol: DSPE-PEG$_{3400}$ = 100 : 66 : 1 | 0.058 (J : B = 56 : 44) |
| 72 | B&J | DPPC : Chol : DSPE-PEG$_{3400}$ = 100 : 66 : 1 | 0.049 (J : B = 80 : 20) |

A : hTRA-8 Fab', B : hTRA-8 Fullbody, C: hHFE7A Fab', D : hHFE7A Fullbody
E : hTRA-8 F(ab')2, F: hTRA-8 scFv, G : MAB631 Fullbody, H : MAB6311 Fullbody
I : DX2 Fullbody, J: 2E12 Fullbody, K : rsTRAIL(peprotech), L : rsTRAIL (biodesign)

Test Example 1

Measurement of apoptosis-inducing activities of immunoliposomes prepared in Examples 1, 2, and 3 against Jurkat cells

Jurkat cells were counted by a trypan blue staining method, and the concentration was then adjusted to $1 \times 10^5$ cells/ml with a DMEM medium (manufactured by Invitrogen Corp.; hereinafter, referred to as a DMEM medium) containing 10% fetal calf serum (manufactured by Hyclone Laboratories, Inc.). An immunoliposome solution whose antibody concentration was adjusted in advance to 2000, 200, 20, or 2 ng/ml (final concentration: 1000, 100, 10, or 1 ng/ml) with a DMEM medium, or a hTRA-8 solution whose antibody concentration was adjusted in advance to 2000, 200, 20, or 2 ng/ml (final concentration: 1000, 100, 10, or 1 ng/ml) with a 1 μg/ml secondary antibody solution (goat anti-human IgG Fc antibody, manufactured by MP Biomedicals Inc.) was added in an amount of 50 μl/well for three rows per group to a 96-well microplate (manufactured by Corning Inc.). To this microplate, the cell suspension was inoculated in an amount of 50 μl ($5 \times 10^3$ cells)/well. The plate was cultured at 37°C for 72 hr in the presence of 5% carbon dioxide, and the ATP level of each well was measured. In the ATP level measurement, a luciferase luminescent reagent (CellTiter Glo, manufactured by Promega Corp.) was used, and the measurement was performed according to the protocol included therein. Specifically, the test solution consisting of a cell lysate component and a luminescent substrate component was added in an amount of 100 μl/well to the plate and stirred. Then, the supernatant was transferred in an amount of 100 μl/well to a 96-well white microplate (manufactured by Corning Inc.), and luminescence from each well was measured using a luminometer (manufactured by Molecular Devices Corp.). Wells supplemented with a DMEM medium and a cell suspension were used as negative control wells, and wells supplemented only with a DMEM medium were used as background wells. The cell viability of each well was calculated according to the following equation:

```
Cell viability (%) = (Luminescence intensity of test wells - Average luminescence intensity of background

wells) / (Average luminescence intensity of negative control wells - Average luminescence intensity of

background wells) × 100.
```

The results are shown in Figure 1. The immunoliposomes prepared in Examples 1, 2, and 3 exhibited a stronger apoptosis-inducing activity than that of secondary antibody-cross-linked hTRA-8 against Jurkat cells. The immunoliposomes prepared in Examples 2 and 3 exhibited 20% or smaller cell viability at the concentration of 1, 10, 100, or 1000 ng/ml.

Test Example 2

Measurement of apoptosis-inducing activities of immunoliposomes prepared in Examples 19, 20, and 21 against A375 cells

The apoptosis-inducing activities against human malignant melanoma cell strain A375 cells were measured according to the method described in Test Example 1. However, the antibody concentrations of the immunoliposome and hTRA-8 were adjusted to 2000, 200, or 20 ng/ml (final concentration: 1000, 100, or 10 ng/ml), and the experiment was conducted using two rows per group.

The results are shown in Figure 2. The immunoliposomes prepared in Examples 19, 20, and 21 exhibited a stronger apoptosis-inducing activity than that of secondary antibody-cross-linked hTRA-8 against A375 cells. The apoptosis-inducing activity exhibited by hTRA-8 against A375 cells was weak but could be enhanced by conjugating the hTRA-8 to an immunoliposome. The immunoliposomes prepared in Examples 19, 20, and 21 exhibited 80% or smaller cell viability at the concentration of 100 ng/ml and 40% or smaller cell viability at the concentration of 1000 ng/ml. The immunoliposome prepared in Example 19 exhibited 20% or smaller cell viability at the concentration of 1000 ng/ml.

Test Example 3

Measurement of apoptosis-inducing activities of immunoliposomes prepared in Examples 8, 10, 12, 14, and 16 against A375 cells

The apoptosis-inducing activities against human malignant melanoma cell strain A375 cells were measured according to the method described in Test Example 1. However, the antibody concentrations of the immunoliposome and hTRA-8 were adjusted to 2000, 200, or 20 ng/ml (final concentration: 1000, 100, or 10 ng/ml), and the experiment was conducted using two rows per group.

The results are shown in Figure 3. The immunoliposomes prepared in Examples 8, 10, 12, 14, and 16 exhibited a stronger apoptosis-inducing activity than that of secondary antibody-cross-linked hTRA-8 against A375 cells. The apoptosis-inducing activity exhibited by hTRA-8 against A375 cells was weak but could be enhanced by conjugating the hTRA-8 to an immunoliposome. The immunoliposomes prepared in Examples 8, 10, 12, 14, and 16 exhibited 60% or smaller cell viability at the concentration of 1000 ng/ml. The immunoliposomes prepared in Examples 8 and 16 exhibited 40% or smaller cell viability at the concentration of 1000 ng/ml. The immunoliposome prepared in Example 8 exhibited 20% or smaller cell viability at the concentration of 1000 ng/ml.

Test Example 4

Measurement of apoptosis-inducing activities of immunoliposomes prepared in Examples 7, 9, 11, 13, and 15 against A2058 cells

The apoptosis-inducing activities against human malignant melanoma cell strain A2058 cells were measured according to the method described in Test Example 1. However, the antibody concentrations of the immunoliposome and hTRA-8 were adjusted to 2000, 200, or 20 ng/ml (final concentration: 1000, 100, or 10 ng/ml), and the experiment was conducted using two rows per group.

The results are shown in Figure 4. The immunoliposomes prepared in Examples 7, 9, 11, 13, and 15 exhibited a stronger apoptosis-inducing activity than that of secondary antibody-cross-linked hTRA-8 against A2058 cells. The apoptosis-inducing activity exhibited by hTRA-8 against A2058 cells was weak but could be enhanced by conjugating the hTRA-8 to an immunoliposome. The immunoliposomes prepared in Examples 9, 11, and 15 exhibited 60% or smaller cell viability at the concentration of 100 ng/ml. The immunoliposomes prepared in Examples 11 and 15 exhibited 40% or smaller cell viability at the concentration of 100 ng/ml. The immunoliposome prepared in Example 15 exhibited 20% or smaller cell viability at the concentration of 100 ng/ml. The immunoliposomes prepared in Examples 7, 9, 11, 13, and 15 exhibited 20% or smaller cell viability at the concentration of 1000 ng/ml.

Test Example 5

Measurement of apoptosis-inducing activities of immunoliposomes prepared in Examples 26, 27, 28, 29, 30, and 31 against Jurkat cells

The apoptosis-inducing activities against T-cell leukemia-lymphoma cell line Jurkat cells were measured according to the method described in Test Example 1. However, the medium used was a RPMI medium (manufactured by Invitrogen Corp.) containing 10% fetal calf serum (manufactured by Hyclone Laboratories, Inc.), and the antibody concentrations of the immunoliposome and hHFE7A were adjusted to 26, 2.6, 0.26, or 0.026 nM (final concentration: 13, 1.3, 0.13, or 0.013 nM). A secondary antibody (goat anti-human IgG Fc antibody) used for cross-linking hHFE7A was manufactured by BioSource International Inc.

The results are shown in Figure 5. The immunoliposomes prepared in Examples 26, 27, 28, 29, 30, and 31 exhibited an apoptosis-inducing activity equivalent to or stronger than that of secondary antibody-cross-linked hHFE7A against Jurkat cells.

Test Example 6

Measurement of apoptosis-inducing activities of immunoliposomes prepared in Examples 4, 17, and 18 against Jurkat cells

Jurkat cells were counted by a trypan blue staining method, and the concentration was then adjusted to $2 \times 10^5$ cells/ml with a RPMI medium (manufactured by Invitrogen Corp.) containing 10% fetal calf serum (manufactured by Hyclone Laboratories, Inc.). An immunoliposome solution whose antibody concentration was adjusted in advance to 2000, 200, 20, or 2 ng/ml (final concentration: 1000, 100, 10, or 1 ng/ml) with a RPMI medium, or a hTRA-8 solution whose antibody concentration was adjusted in advance to 2000, 200, 20, or 2 ng/ml (final concentration: 1000, 100, 10, or 1 ng/ml) with a 1 $\mu$g/ml secondary antibody solution (goat anti-human IgG Fc antibody, manufactured by MP Biomedicals Inc.) was added in an amount of 50 $\mu$l/well for three rows per group to a 96-well microplate (manufactured by Corning Inc.). To this microplate, the cell suspension was inoculated in an amount of 50 $\mu$l ($1 \times 10^4$ cells)/well. The plate was cultured at 37°C for 72 hr in the presence of 5% carbon dioxide, and the intracellular dehydrogenase activity of the cells in each well was measured to thereby calculate a live cell count. In the intracellular dehydrogenase activity measurement, WST-8 Reagent (live cell counting reagent SF, Nacalai Tesque) was used, and the measurement was performed according to the protocol included therein. Specifically, WST-8 was added at a concentration of 10 $\mu$l/well, and the amount of formazan produced by reducing WST-8 with the intracellular dehydrogenase was quantified by measuring the absorbance of formazan at 450 nm using an absorptiometer (manufactured by Molecular Devices Corp.). Wells supplemented with a RPMI1640 medium and a cell suspension were used as negative control wells, and wells supplemented only with a RPMI1640 medium were used as background wells. The cell viability of each well was calculated according to the following equation:

```
Cell viability (%) = (Absorbance of test wells - Average Absorbance of background wells) / (Average Absorbance of

negative control wells - Average Absorbance of background

wells) × 100.
```

The results are shown in Figure 6. The immunoliposomes prepared in Examples 4, 17, and 18 exhibited a stronger apoptosis-inducing activity than that of secondary antibody-cross-linked hTRA-8 against Jurkat cells. The immunoliposomes prepared in Examples 4, 17, and 18 exhibited 60% or smaller cell viability at the concentration of 10 ng/ml and 20% or smaller cell viability at the concentration of 100 or 1000 ng/ml.

Test Example 7

Measurement of apoptosis-inducing activities of immunoliposomes prepared in Examples 22 and 23 against Jurkat cells

The apoptosis-inducing activities against T-cell leukemia-lymphoma cell line Jurkat cells were measured according to the method described in Test Example 1. However, the medium used was a RPMI medium (manufactured by Invitrogen Corp.) containing 10% fetal calf serum (manufactured by Hyclone Laboratories, Inc.); the antibody concentrations of the immunoliposome and hTRA-8 were adjusted to 2000, 200, 20, 2, 0.2, or 0.02 ng/ml (final concentration: 1000, 100, 10, 1, 0.1, or 0.01 ng/ml); and the experiment was conducted using three rows per group.
The results are shown in Figure 7. The immunoliposomes prepared in Examples 22 and 23 exhibited a stronger apoptosis-inducing activity than that of secondary antibody-cross-linked hTRA-8 against Jurkat cells. The immunoliposomes prepared in Examples 22 and 23 exhibited 60% or smaller cell viability at the concentration of 0.1 ng/ml and 20% or smaller cell viability at the concentration of 1, 10, 100, or 1000 ng/ml.

Test Example 8

Measurement of apoptosis-inducing activity of immunoliposome prepared in Example 24 against Jurkat cells

The apoptosis-inducing activity against T-cell leukemia-lymphoma cell line Jurkat cells was measured according to the method described in Test Example 1. However, the medium used was a RPMI medium (manufactured by Invitrogen Corp.) containing 10% fetal calf serum (manufactured by Hyclone Laboratories, Inc.); the antibody concentrations of the immunoliposome and hTRA-8 were adjusted to 200, 20, 2, or 0.2 ng/ml (final concentration: 100, 10, 1, or 0.1 ng/ml); and the experiment was conducted using three rows per group.
The results are shown in Figure 8. The immunoliposome prepared in Example 24 exhibited a stronger apoptosis-inducing activity than that of secondary antibody-cross-linked hTRA-8 against Jurkat cells. The immunoliposome prepared in Example 24 exhibited 60% or smaller cell viability at the concentration of 1 ng/ml and 20% or smaller cell viability at the concentration of 10 or 100 ng/ml.

Test Example 9

Measurement of apoptosis-inducing activity of immunoliposome prepared in Example 25 against Jurkat cells

The apoptosis-inducing activity against T-cell leukemia-lymphoma cell line Jurkat cells was measured according to the method described in Test Example 1. However, the medium used was a RPMI medium (manufactured by Invitrogen Corp.) containing 10% fetal calf serum (manufactured by Hyclone Laboratories, Inc.); the antibody concentrations of the immunoliposome and hTRA-8 were adjusted to 200, 20, 2, or 0.2 ng/ml (final concentration: 100, 10, 1, or 0.1 ng/ml); and the experiment was conducted using three rows per group.

The results are shown in Figure 9. The immunoliposome prepared in Example 25 exhibited a stronger apoptosis-inducing activity than that of secondary antibody-cross-linked hTRA-8 against Jurkat cells. The immunoliposome prepared in Example 25 exhibited 70% or smaller cell viability at the concentration of 0.1 ng/ml and 20% or smaller cell viability at the concentration of 1, 10, or 100 ng/ml.

Test Example 10

Measurement of apoptosis-inducing activities of immunoliposomes prepared in Examples 5 and 6 against Jurkat cells

The apoptosis-inducing activities against T-cell leukemia-lymphoma cell line Jurkat cells were measured according to the method described in Test Example 1. However, the medium used was a RPMI medium (manufactured by Invitrogen Corp.) containing 10% fetal calf serum (manufactured by Hyclone Laboratories, Inc.); the antibody concentrations of the immunoliposome and hTRA-8 were adjusted to 200, 20, 2, or 0.2 ng/ml (final concentration: 100, 10, 1, or 0.1 ng/ml); and the experiment was conducted using three rows per group.

The results are shown in Figure 10. The immunoliposomes prepared in Examples 5 and 6 exhibited a stronger apoptosis-inducing activity than that of secondary antibody-cross-linked hTRA-8 against Jurkat cells. The immunoliposome prepared in Example 5 exhibited 70% or smaller cell viability at the concentration of 0.1 ng/ml. The immunoliposome prepared in Example 6 exhibited 40% or smaller cell viability at the concentration of 0.1 ng/ml. Moreover, the immunoliposomes prepared in Examples 5 and 6 exhibited 20% or smaller cell viability at the concentration of 1, 10, or 100 ng/ml.

Test Example 11

Measurement of apoptosis-inducing activities of immunoliposomes prepared in Examples 3 and 20 against synovial cells derived from articular rheumatism patients

Frozen synovial cells derived from articular rheumatism patients (Cell Applications, Inc.) were suspended in a synovial cell growth medium (manufactured by Cell Applications, Inc., hereinafter, referred to as a medium) and counted by a trypan blue staining method, and the concentration was then adjusted to $2 \times 10^4$ cells/ml with a medium. The cell suspension was inoculated in an amount of 50 $\mu$l ($1 \times 10^3$ cells)/well to a 96-well microplate (manufactured by Corning Inc.). Simultaneously, an immunoliposome solution or hTRA-8 diluted with a medium to 2000, 200, 20, or 2 ng/ml in terms of antibody concentrations was added in an amount of 50 $\mu$l/well to the plate. The plate was cultured overnight at 37°C in the presence of 5% carbon dioxide, and the ATP level of each well was then measured. In the ATP level measurement, a luciferase luminescent reagent (CellTiter Glo, manufactured by Promega Corp.) was used, and the measurement was performed according to the protocol included therein. Specifically, the test solution consisting of a cell lysate component and a luminescent substrate component was added in an amount of 100 $\mu$l/well to the plate and stirred. Then, the supernatant was transferred in an amount of 100 $\mu$l/well to a 96-well white microplate (manufactured by Corning Inc.), and luminescence from each well was measured using a luminometer (manufactured by Molecular Devices Corp.). Wells supplemented with a medium instead of the immunoliposome solution were used as negative control wells, and wells supplemented only with a medium without being inoculated with the cells were used as background wells. The cell viability of each well was calculated according to the following equation:

$$\text{Cell viability (\%) = (Luminescence intensity of test}$$

$$\text{wells - Average luminescence intensity of background}$$

$$\text{wells) / (Average luminescence intensity of negative}$$

$$\text{control wells - Average luminescence intensity of}$$

$$\text{background wells)} \times 100.$$

The results are shown in Figure 11. The immunoliposomes prepared in Examples 3 and 20 exhibited a stronger apoptosis-inducing activity than that of secondary antibody-cross-linked hTRA-8 against synovial cells derived from articular rheumatism patients. The immunoliposome prepared in Example 3 exhibited 50% or smaller cell viability at the concentration of 1000 ng/ml.

Test Example 12

Measurement of antitumor activity of immunoliposome prepared in Example 5 against human colon cancer strain COLO 205-transplanted nude mice

The in-vivo antitumor activity of the immunoliposome prepared in Example 5 was studied. $2 \times 10^6$ cells of a human colon cancer strain COLO 205 (purchased from American Type Culture Collection) which was confirmed by quarantine to have no detectable mouse pathogenic microorganisms were hypodermically transplanted to the axillary region of nude mice BALB/cA Jcl-nu (CLEA Japan, Inc.). The immunoliposome of Example 5 was diluted, immediately before its administration, with saline (Otsuka Pharmaceutical Co., Ltd.) to 1 and 0.33 mg/ml in terms of antibody concentrations. Seven days after the transplantation, the administration of the immunoliposome of Example 5 to COLO 205 cancer-bearing mice in which the tumor successfully grafted was started. Specifically, on Days 7, 9, 11, 14, 16, 18, 21, 23, and 25, the liposome solution was intravenously administered to the tails of the cancer-bearing mice at a dose of 0.1 ml per 10 g of mouse body weight.
The major axis and minor axis of the transplanted tumor were measured two to three times a week using an electronic vernier caliper (CD-15C; Mitutoyo Corp.). The tumor volume was determined according to the following calculation formula:
$$\text{Tumor volume (mm}^3\text{) = 1/2} \times \text{minor axis}^2 \text{ (mm)} \times \text{major}$$

$$\text{axis (mm).}$$

Moreover, the rate of tumor growth (mm$^3$/day) of each individual was calculated, and the statistically significant difference was tested by a Dunnett test using the value. In this context, a P value less than 0.05 was regarded as being a significant difference among groups.
The test results are shown in Figure 12. The immunoliposome of Example 5 exhibited a dose-dependent antitumor activity and exhibited an antitumor activity with a statistically significant difference at the dose of 10 mg/kg.

Test Example 13

Measurement of apoptosis-inducing activities of immunoliposomes prepared in Examples 32, 33, 34, and 35 against Jurkat cells

The apoptosis-inducing activities against T-cell leukemia-lymphoma cell line Jurkat cells were measured according to the method described in Test Example 1. However, the medium used was a RPMI medium (manufactured by Invitrogen Corp.) containing 10% fetal calf serum (manufactured by Hyclone Laboratories, Inc.); the antibody concentrations of the immunoliposome and hTRA-8 were adjusted to 2000, 200, 20, 2, 0.2, or 0.02 ng/ml (final concentration: 1000, 100, 10, 1, 0.1, or 0.01 ng/ml); and the experiment was conducted using three rows per group.
The results are shown in Figure 13. The immunoliposomes prepared in Examples 32, 33, 34, and 35 exhibited a stronger apoptosis-inducing activity than that of secondary antibody-cross-linked hTRA-8 against Jurkat cells and exhibited 70% or smaller cell viability at the concentration of 0.1 ng/ml and 30% or smaller cell viability at the concentration of 1, 10, 100, or 1000 ng/ml.

Test Example 14

Measurement of apoptosis-inducing activities of immunoliposomes prepared in Examples 44, 45, 46, 47, 48, and 49 against Jurkat cells

The apoptosis-inducing activities against T-cell leukemia-lymphoma cell line Jurkat cells were measured according to the method described in Test Example 1. However, the medium used was a RPMI medium (manufactured by Invitrogen Corp.) containing 10% fetal calf serum (manufactured by Hyclone Laboratories, Inc.); the antibody concentrations of the immunoliposome and hTRA-8 were adjusted to 2000, 200, 20, 2, 0.2, or 0.02 ng/ml (final concentration: 1000, 100, 10, 1, 0.1, or 0.01 ng/ml); and the experiment was conducted using three rows per group.
The results are shown in Figure 14. The immunoliposomes prepared in Examples 44, 45, 46, 47, 48, and 49 exhibited a stronger apoptosis-inducing activity than that of secondary antibody-cross-linked hTRA-8 against Jurkat cells and exhibited 60% or smaller cell viability at the concentration of 1 ng/ml and 20% or smaller cell viability at the concentration of 10, 100, or 1000 ng/ml.

Test Example 15

Measurement of apoptosis-inducing activities of immunoliposomes prepared in Examples 50, 51, and 52 against Jurkat cells

The apoptosis-inducing activities against T-cell leukemia-lymphoma cell line Jurkat cells were measured according to the method described in Test Example 1. However, the medium used was a RPMI medium (manufactured by Invitrogen Corp.) containing 10% fetal calf serum (manufactured by Hyclone Laboratories, Inc.); the antibody concentrations of the immunoliposome and hTRA-8 were adjusted to 2000, 200, 20, 2, 0.2, or 0.02 ng/ml (final concentration: 1000, 100, 10, 1, 0.1, or 0.01 ng/ml); and the experiment was conducted using three rows per group.
The results are shown in Figure 15. The immunoliposomes prepared in Examples 50, 51, and 52 exhibited a stronger apoptosis-inducing activity than that of secondary antibody-cross-linked hTRA-8 against Jurkat cells and exhibited 60% or smaller cell viability at the concentration of 0.1 ng/ml and 20% or smaller cell viability at the concentration of 1, 10, 100, or 1000 ng/ml.

Test Example 16

Measurement of apoptosis-inducing activities of immunoliposomes prepared in Examples 53, 54, 55, 56, and 57 against Jurkat cells

The apoptosis-inducing activities against T-cell leukemia-lymphoma cell line Jurkat cells were measured according to the method described in Test Example 1. However, the medium used was a RPMI medium (manufactured by Invitrogen Corp.) containing 10% fetal calf serum (manufactured by Hyclone Laboratories, Inc.); the antibody concentrations of the immunoliposome and hTRA-8 were adjusted to 2000, 200, 20, 2, 0.2, or 0.02 ng/ml (final concentration: 1000, 100, 10, 1, 0.1, or 0.01 ng/ml); and the experiment was conducted using three rows per group.
The results are shown in Figure 16. The immunoliposomes prepared in Examples 53, 54, 55, 56, and 57 exhibited a stronger apoptosis-inducing activity than that of secondary antibody-cross-linked hTRA-8 against Jurkat cells and exhibited 40% or smaller cell viability at the concentration of 1 ng/ml and 5% or smaller cell viability at the concentration of 10, 100, or 1000 ng/ml.

Test Example 17

Measurement of apoptosis-inducing activity of immunoliposome prepared in Example 58 against Jurkat cells

The apoptosis-inducing activity against T-cell leukemia-lymphoma cell line Jurkat cells was measured according to the method described in Test Example 1. However, the medium used was a RPMI medium (manufactured by Invitrogen Corp.) containing 10% fetal calf serum (manufactured by Hyclone Laboratories, Inc.); the antibody concentrations of the immunoliposome and hTRA-8 were adjusted to 2000, 200, 20, 2, or 0.2 ng/ml (final concentration: 1000, 100, 10, 1, or 0.1 ng/ml); and the experiment was conducted using three rows per group.
The results are shown in Figure 17. The immunoliposome prepared in Example 58 exhibited a stronger apoptosis-inducing activity than that of secondary antibody-cross-linked hTRA-8 against Jurkat cells and exhibited 30% or smaller cell viability at the concentration of 1 ng/ml and 5% or smaller cell viability at the concentration of 10, 100, or 1000 ng/ml.

Test Example 18

Measurement of apoptosis-inducing activities of immunoliposomes prepared in Examples 36 and 37 against Jurkat cells

The apoptosis-inducing activities against T-cell leukemia-lymphoma cell line Jurkat cells were measured according to the method described in Test Example 1. However, the medium used was a RPMI medium (manufactured by Invitrogen Corp.) containing 10% fetal calf serum (manufactured by Hyclone Laboratories, Inc.); the antibody concentrations were adjusted to 2000, 20, or 0.2 ng/ml (final concentration: 1000, 10, or 0.1 ng/ml) for the immunoliposome and adjusted with a 1 μg/mL secondary antibody solution (anti-mouse IgG Fc) to 2000, 20, or 0.2 ng/mL (final concentration: 1000, 10, or 0.1 ng/mL) for MAB631; and the experiment was conducted using two rows per group.
The results are shown in Figure 18. The immunoliposomes prepared in Examples 36 and 37 exhibited a stronger apoptosis-inducing activity than that of secondary antibody-cross-linked MAB631 against Jurkat cells and exhibited 50% or smaller cell viability at the concentration of 0.1 ng/ml and 5% or smaller cell viability at the concentration of 10 or 1000 ng/ml.

Test Example 19

Measurement of apoptosis-inducing activities of immunoliposomes prepared in Examples 38 and 39 against Jurkat cells

The apoptosis-inducing activities against T-cell leukemia-lymphoma cell line Jurkat cells were measured according to the method described in Test Example 1. However, the medium used was a RPMI medium (manufactured by Invitrogen Corp.) containing 10% fetal calf serum (manufactured by Hyclone Laboratories, Inc.); the antibody concentrations were adjusted to 2000, 20, or 0.2 ng/ml (final concentration: 1000, 10, or 0.1 ng/ml) for the immunoliposome and adjusted with a 1 μg/mL secondary antibody solution (anti-mouse IgG Fc) to 2000, 20, or 0.2 ng/mL (final concentration: 1000, 10, or 0.1 ng/mL) for MAB6311; and the experiment was conducted using two rows per group.
The results are shown in Figure 19. The immunoliposomes prepared in Examples 38 and 39 exhibited a stronger apoptosis-inducing activity than that of secondary antibody-cross-linked MAB6311 against Jurkat cells and exhibited 40% or smaller cell viability at the concentration of 0.1 ng/ml and 5% or smaller cell viability at the concentration of 10 or 1000 ng/ml.

Test Example 20

Measurement of apoptosis-inducing activities of immunoliposomes prepared in Examples 59 and 60 against Jurkat cells

The apoptosis-inducing activities against T-cell leukemia-lymphoma cell line Jurkat cells were measured according to the method described in Test Example 1. However, the medium used was a RPMI medium (manufactured by Invitrogen Corp.) containing 10% fetal calf serum (manufactured by Hyclone Laboratories, Inc.); the protein concentrations of the immunoliposome and rsTRAIL were adjusted to 2000, 200, 20, or 2 ng/ml (final concentration: 1000, 100, 10, or 1 ng/ml); and the experiment was conducted using two rows per group.
The results are shown in Figure 20. The immunoliposomes prepared in Examples 59 and 60 exhibited a stronger apoptosis-inducing activity than that of rsTRAIL against Jurkat cells and exhibited 70% or smaller cell viability at the concentration of 1 ng/ml and 20% or smaller cell viability at the concentration of 10, 100, or 1000 ng/ml.

Test Example 21

Measurement of apoptosis-inducing activity of immunoliposome prepared in Example 61 against Jurkat cells

The apoptosis-inducing activity against T-cell leukemia-lymphoma cell line Jurkat cells was measured according to the method described in Test Example 1. However, the medium used was a RPMI medium (manufactured by Invitrogen Corp.) containing 10% fetal calf serum (manufactured by Hyclone Laboratories, Inc.); the protein concentrations of the immunoliposome and rsTRAIL were adjusted to 2000, 200, 20, or 2 ng/ml (final concentration: 1000, 100, 10, or 1 ng/ml); and the experiment was conducted using two rows per group.
The results are shown in Figure 21. The immunoliposome prepared in Example 61 exhibited a stronger apoptosis-inducing activity than that of rsTRAIL against Jurkat cells and exhibited 20% or smaller cell viability at the concentration of 10, 100, or 1000 ng/ml.

Test Example 22

Measurement of apoptosis-inducing activities of immunoliposomes prepared in Examples 41, 42, 43, and 69 against Jurkat cells

The apoptosis-inducing activities against T-cell leukemia-lymphoma cell line Jurkat cells were measured according to the method described in Test Example 1. However, the medium used was a RPMI medium (manufactured by Invitrogen Corp.) containing 10% fetal calf serum (manufactured by Hyclone Laboratories, Inc.); the antibody concentrations of the immunoliposome and hTRA-8 were adjusted to 2000, 200, 20, 2, 0.2, or 0.02 ng/ml (final concentration: 1000, 100, 10, 1, 0.1, or 0.01 ng/ml); and the experiment was conducted using three rows per group.
The results are shown in Figure 22. The immunoliposomes prepared in Examples 41, 42, 43, and 69 exhibited a stronger apoptosis-inducing activity than that of secondary antibody-cross-linked hTRA-8 against Jurkat cells and exhibited 80% or smaller cell viability at the concentration of 0.1 ng/ml, 40% or smaller cell viability at the concentration of 1 ng/ml, and 10% or smaller cell viability at the concentration of 10, 100, or 1000 ng/ml.

Test Example 23

Measurement of apoptosis-inducing activities of immunoliposomes prepared in Examples 67 and 68 against Jurkat cells

The apoptosis-inducing activities against T-cell leukemia-lymphoma cell line Jurkat cells were measured according to the method described in Test Example 1. However, the medium used was a RPMI medium (manufactured by Invitrogen Corp.) containing 10% fetal calf serum (manufactured by Hyclone Laboratories, Inc.); the antibody concentrations of the immunoliposome and hTRA-8 were adjusted to 2000, 200, 20, 2, or 0.2 ng/ml (final concentration: 1000, 100, 10, 1, or 0.1 ng/ml); and the experiment was conducted using three rows per group.
The results are shown in Figure 23. The immunoliposome prepared in Example 68 exhibited a stronger apoptosis-inducing activity than that of secondary antibody-cross-linked hTRA-8 against Jurkat cells and exhibited 30% or smaller cell viability at the concentration of 1 ng/ml and 5% or smaller cell viability at the concentration of 10, 100, or 1000 ng/ml.

Test Example 24

Measurement of apoptosis-inducing activity of immunoliposome prepared in Example 40 against WR19L12a cells

WR19L12a cells were counted by a trypan blue staining method, and the concentration was then adjusted to $1 \times 10^5$ cells/ml with a RPMI medium (manufactured by Invitrogen Corp.) containing 10% fetal calf serum (manufactured by Hyclone Laboratories, Inc.). An immunoliposome solution whose antibody concentration was adjusted in advance to 10, 1, 0.1, or 0.01 $\mu$g/mL (final concentration: 5, 0.5, 0.05, or 0.005 $\mu$g/mL) with a RPMI medium, or a DX2 solution whose antibody concentration was adjusted in advance to 10, 1, 0.1, or 0.01 $\mu$g/mL (final concentration: 5, 0.5, 0.05, or 0.005 $\mu$g/mL) with 0.6 $\mu$g/mL rProtein G (manufactured by Sigma-Aldrich, Inc.) was added in an amount of 50 $\mu$l/well for three rows per group to a 96-well microplate (manufactured by Corning Inc.). To this microplate, the cell suspension was inoculated in an amount of 50 $\mu$l ($5 \times 10^3$ cells)/well. The plate was cultured at 37°C for 72 hr in the presence of 5% carbon dioxide, and the ATP level of each well was measured according to the method described in Test Example 1. However, wells supplemented with a RPMI medium containing PBS and a cell suspension were used as negative control wells, and wells supplemented only with a RPMI medium were used as background wells.
The results are shown in Figure 24. The immunoliposome prepared in Example 40 exhibited a stronger apoptosis-inducing activity than that of DX2 against WR19L12a cells and exhibited 20% or smaller cell viability at the concentration of 500 or 5000 ng/ml.

Test Example 25

Measurement of apoptosis-inducing activities of immunoliposomes prepared in Examples 62, 64, 65, and 66 against Alexander cells

Alexander cells were counted by a trypan blue staining method, and the concentration was then adjusted to $2 \times 10^4$ cells/ml with a RPMI medium (manufactured by Invitrogen Corp.) containing 10% fetal calf serum (manufactured by Hyclone Laboratories, Inc.). An immunoliposome solution whose antibody concentration was adjusted in advance to 2000, 200, 20, or 2 ng/mL (final concentration: 1000, 100, 10, or 1 ng/mL) with a RPMI medium, or a m2E12 solution whose antibody concentration was adjusted in advance to 2000, 200, 20, or 2 ng/mL (final concentration: 1000, 100, 10, or 1 ng/mL) with a 3.6 $\mu$g/mL secondary antibody solution (goat anti-mouse IgG Fc, manufactured by Jackson ImmunoResearch Laboratories, Inc.) was added in an amount of 50 $\mu$l/well for three rows per group to a 96-well microplate (manufactured by Corning Inc.). To this microplate, the cell suspension was inoculated in an amount of 50 $\mu$l ($1 \times 10^3$ cells)/well. The plate was cultured at 37°C for 72 hr in the presence of 5% carbon dioxide, and the ATP level of each well was measured according to the method described in Test Example 1.

The results are shown in Figure 25. The immunoliposomes prepared in Examples 62, 64, 65, and 66 exhibited a stronger apoptosis-inducing activity than that of m2E12 against Alexander cells and exhibited 70% or smaller cell viability at the concentration of 100 ng/ml and 50% or smaller cell viability at the concentration of 1000 ng/ml.

Test Example 26

Measurement of apoptosis-inducing activities of immunoliposomes prepared in Examples 70, 71, and 72 against Alexander cells

Alexander cells were counted by a trypan blue staining method, and the concentration was then adjusted to $2 \times 10^4$ cells/mL with a RPMI medium (manufactured by Invitrogen Corp.) containing 10% fetal calf serum (manufactured by Hyclone Laboratories, Inc.). An immunoliposome solution whose antibody concentration was adjusted in advance to 2000, 200, 20, or 2 ng/mL (final concentration: 1000, 100, 10, or 1 ng/mL) with a RPMI medium, or a m2E12/hTRA-8 mixed solution of a m2E12 solution whose antibody concentration was adjusted in advance to 2000 ng/mL with a 3.6 $\mu$g/mL secondary antibody solution (goat anti-mouse IgG Fc, manufactured by Jackson ImmunoResearch Laboratories, Inc.) and a hTRA-8 solution whose antibody concentration was adjusted in advance to 2000 ng/mL with a 1 $\mu$g/mL secondary antibody solution (goat anti-human IgG Fc, manufactured by MP Biomedicals Inc.). The m2E12 solution and the hTRA-8 solutions were mixed at a ratio of 22:78, 56:44, or 80:20, and the concentration was adjusted to 2000, 200, 20, or 2 ng/mL (final concentration: 1000, 100, 10, or 1 ng/mL) was added in an amount of 50 $\mu$l/well for three rows per group to a 96-well microplate (manufactured by Corning Inc.). To this microplate, the cell suspension was inoculated in an amount of 50 $\mu$l ($1 \times 10^3$ cells)/well. The plate was cultured at 37°C for 24 hr in the presence of 5% carbon dioxide, and the ATP level of each well was measured according to the method described in Test Example 1. The results are shown in Figure 26. The immunoliposomes prepared in Examples 70, 71, and 72 exhibited a stronger apoptosis-inducing activity than that of the antibodies adjusted to the corresponding ratio against Alexander cells and exhibited 60% or smaller cell viability at the concentration of 10 ng/ml and 10% or smaller cell viability at the concentration of 100 or 1000 ng/ml.

Test Example 27

The concentrations at which cell viability was decreased to 50% by the immunoliposomes or control antibodies were calculated from Test Examples 1 to 26. The results are shown in Table 3 (only Test Examples 1, 6 to 10, 13 to 23, and 26 are described since the 50% cell viability of the control antibodies could be determined for these examples). In the table, $EC_{50}$ represents a concentration exhibiting 50% cell viability. Furthermore, $EC_{50}$ Ab/IL is defined as representing a value obtained by dividing the concentration for 50% cell viability of the control antibody by the concentration for 50% cell viability of each immunoliposome. Most of the immunoliposomes exhibited a concentration for 50% cell viability which was 1/10 or smaller than that of the control antibody. Even in Test Example 19 in which the immunoliposomes exhibited a low apoptosis-inducing activity, the concentration for 50% cell viability of the immunoliposomes was 1/2.5 or smaller than that of the control antibodies. In Test Examples 7, 9, 13 to 16, 18, and 22, it was 1/100 or smaller than that of the control antibodies.

[Table 3]

| Test Example No. | Example No. of compound | $EC_{50}$ (ng/mL) | $EC_{50}$ Ab/II | Cross-linked Antibody $EC_{50}$ (ng/mL) |
|---|---|---|---|---|
| **1** | 3 | 0.57 | **30.9** | 17.6 |
| | 2 | 0.93 | **18.9** | |
| | 1 | 9.2 | **1.9** | |
| **6** | 4 | 8.61 | **31.4** | 270.4 |
| | 17 | 6.33 | **42.7** | |
| | 18 | 10.7 | **25.3** | |

(continued)

| Test Example No. | Example No. of compound | EC$_{50}$ (ng/mL) | EC$_{50}$ Ab/II | Cross-linked Antibody EC$_{50}$ (ng/mL) |
|---|---|---|---|---|
| 7 | 22 | 0.075 | **228.0** | 17.1 |
|  | 23 | 0.1 | **171.0** |  |
| 8 | 24 | 1.32 | **55.8** | 73.7 |
| 9 | 25 | 0.168 | **186.9** | 31.4 |
| 10 | 5 | 0.189 | **77.2** | 14.6 |
|  | 6 | 0.079 | **184.8** |  |
| 12 | 32 | 0.048 | **614.6** | 29.5 |
|  | 33 | 0.158 | **186.7** |  |
|  | 34 | 0.0514 | **573.9** |  |
|  | 35 | 0.274 | **107.7** |  |
| 13 | 44 | 0.0627 | **575.8** | 36.1 |
|  | 45 | 0.211 | **171.1** |  |
|  | 46 | 0.477 | **75.7** |  |
|  | 47 | 0.0276 | **1308** |  |
|  | 48 | 0.158 | **228.5** |  |
|  | 49 | 1.02 | **35.4** |  |
| 14 | 50 | 0.128 | **230.5** | 29.5 |
|  | 51 | 0.0757 | **389.7** |  |
|  | 52 | 0.125 | **236.0** |  |
| 15 | 53 | 0.235 | **153.7** | 36.12 |
|  | 54 | 0.13 | **277.8** |  |
|  | 55 | 0.332 | **108.8** |  |
|  | 56 | 0.489 | **73.9** |  |
|  | 57 | 0.127 | **284.4** |  |
| 16 | 58 | 0.343 | **99.7** | 34.19 |
| 17 | 36 | 0.069 | **193.6** | 13.36 |
|  | 37 | 0.089 | **150.1** |  |
| 18 | 38 | 0.075 | **31.9** | 2.39 |
|  | 39 | 0.083 | **28.8** |  |
| 19 | 59 | 1.9 | **2.8** | 5.24 |
|  | 60 | 1.24 | **4.2** |  |
| 20 | 61 | 4.24 | **3.7** | 15.5 |
| 21 | 41 | 0.057 | **378.8** | 21.59 |
|  | 42 | 0.144 | **149.9** |  |
|  | 43 | 0.094 | **229.7** |  |
|  | 69 | 0.371 | **58.2** |  |

(continued)

| Test Example No. | Example No. of compound | EC$_{50}$ (ng/mL) | EC$_{50}$ Ab/II | Cross-linked Antibody EC$_{50}$ (ng/mL) |
|---|---|---|---|---|
| 22 | 67 | 7.04 | **2.7** | 18.7 |
| | 68 | 0.233 | **80.3** | |
| 25 | 70 | 1.69 | **21.5** | 36.3 |
| | 71 | 2.93 | **26.6** | 77.8 |
| | 72 | 10.7 | **8.2** | 87.4 |

Industrial Applicability

The present invention provides a pharmaceutical composition for cancer treatment and a pharmaceutical composition for autoimmune disease or inflammatory disease treatment comprising an immunoliposome which contains an antibody specifically binding to a cell surface receptor involved in apoptosis induction.

[Sequence Listing Free Text]

SEQ ID NO: 1 - Description of artificial sequence: the heavy chain amino acid sequence of humanized TRA-8.
SEQ ID NO: 2 - Description of artificial sequence: the light chain amino acid sequence of humanized TRA-8.
SEQ ID NO: 3 - Description of artificial sequence: the heavy chain amino acid sequence of humanized HFE7A.
SEQ ID NO: 4 - Description of artificial sequence: the light chain amino acid sequence of humanized HFE7A.

**Claims**

1. An immunoliposome which contains the following components (a) and (b) and specifically binds to a cell surface receptor involved in apoptosis induction:

   (a) an antibody specifically binding to the cell surface receptor involved in apoptosis induction, a functional fragment of the antibody, or a polypeptide comprising heavy and light chain complementarity-determining regions of the antibody and specifically binding to the cell surface receptor; and
   (b) an amphiphilic vesicle-forming lipid.

2. The immunoliposome according to claim 1, **characterized in that** the immunoliposome exhibits an apoptosis-inducing activity against a cell expressing the cell surface receptor involved in apoptosis induction.

3. The immunoliposome according to claim 1 or 2, **characterized in that** the immunoliposome exhibits, in vitro against a cell expressing the cell surface receptor involved in apoptosis induction, an apoptosis-inducing activity equivalent to or stronger than that exhibited in vitro, through cross-linking by a secondary antibody or an antibody-binding protein such as protein G or A, of full-length molecules of the antibody specifically binding to the cell surface receptor.

4. The immunoliposome according to any one of claims 1 to 3, **characterized in that** the immunoliposome exhibits, against a cell expressing the cell surface receptor involved in apoptosis induction, a concentration for 50% cell viability that is 1/2.5 or smaller than that exhibited in vitro, through cross-linking by a secondary antibody or an antibody-binding protein such as protein G or A, of full-length molecules of the antibody specifically binding to the cell surface receptor.

5. The immunoliposome according to any one of claims 1 to 4, **characterized in that** the immunoliposome exhibits, against a cell expressing the cell surface receptor involved in apoptosis induction, a concentration for 50% cell viability that is 1/10 or smaller than that exhibited *in vitro,* through cross-linking by a secondary antibody or an antibody-binding protein such as protein G or A, of full-length molecules of the antibody specifically binding to the cell surface receptor.

6. The immunoliposome according to any one of claims 1 to 5, **characterized in that** the immunoliposome contains an antibody specifically binding to the cell surface receptor involved in apoptosis induction, wherein the antibody is a full-length antibody molecule.

7. The immunoliposome according to claim 6, **characterized in that** the immunoliposome contains the full-length antibody molecule at an antibody density of 0.000014 mol% to 0.23 mol% with respect to the number of moles of total lipids.

8. The immunoliposome according to claim 7, **characterized in that** the immunoliposome contains the full-length antibody molecule at an antibody density of 0.002 mol% to 0.14 mol% with respect to the number of moles of total lipids.

9. The immunoliposome according to any one of claims 1 to 5, **characterized in that** the functional fragment of the antibody specifically binding to the cell surface receptor involved in apoptosis induction is $F(ab')_2$.

10. The immunoliposome according to claim 9, **characterized in that** the immunoliposome contains the $F(ab')_2$ at an antibody density of 0.000014 mol% to 0.23 mol% with respect to the number of moles of total lipids.

11. The immunoliposome according to claim 10, **characterized in that** the immunoliposome contains the $F(ab')_2$ at an antibody density of 0.006 mol% to 0.11 mol% with respect to the number of moles of total lipids.

12. The immunoliposome according to any one of claims 1 to 5, **characterized in that** the functional fragment of the antibody specifically binding to the cell surface receptor involved in apoptosis induction is Fab'.

13. The immunoliposome according to claim 12, **characterized in that** the immunoliposome contains the Fab' at an antibody density of 0.000014 mol% to 0.94 mol% with respect to the number of moles of total lipids.

14. The immunoliposome according to claim 13, **characterized in that** the immunoliposome contains the Fab' at an antibody density of 0.005 mol% to 0.56 mol% with respect to the number of moles of total lipids.

15. The immunoliposome according to any one of claims 1 to 14, **characterized in that** the antibody specifically binding to the cell surface receptor involved in apoptosis induction is a chimeric antibody.

16. The immunoliposome according to any one of claims 1 to 14, **characterized in that** the antibody specifically binding to the cell surface receptor involved in apoptosis induction is a humanized antibody.

17. The immunoliposome according to any one of claims 1 to 14, **characterized in that** the antibody specifically binding to the cell surface receptor involved in apoptosis induction is a human antibody.

18. The immunoliposome according to any one of claims 1 to 5, **characterized in that** the polypeptide specifically binding to the cell surface receptor involved in apoptosis induction is a single-chain variable fragment antibody.

19. The immunoliposome according to claim 18, **characterized in that** the immunoliposome contains the single-chain variable fragment antibody at an antibody density of 0.000014 mol% to 1.8 mol% with respect to the number of moles of total lipids.

20. The immunoliposome according to claim 19, **characterized in that** the immunoliposome contains the single-chain variable fragment antibody at an antibody density of 0.005 mol% to 0.56 mol% with respect to the number of moles of total lipids.

21. The immunoliposome according to any one of claims 1 to 20, **characterized in that** the cell surface receptor involved in apoptosis induction is a death domain-containing receptor.

22. The immunoliposome according to claim 21, wherein the death domain-containing receptor is selected from the group consisting of Fas, DR4, DR5, and a TNF receptor.

23. The immunoliposome according to claim 22, wherein the death domain-containing receptor is DR5.

24. The immunoliposome according to claim 22, wherein the death domain-containing receptor is DR4.

25. The immunoliposome according to claim 22, wherein the death domain-containing receptor is Fas.

26. The immunoliposome according to claim 23, **characterized in that** the antibody molecule or the functional fragment of the antibody comprises a heavy chain variable region sequence consisting of amino acid residues 1 to 118 of the amino acid sequence of SEQ ID NO: 1 described in the sequence listing and a light chain variable region sequence consisting of amino acid residues 1 to 107 of the amino acid sequence of SEQ ID NO: 2 described in the sequence listing.

27. The immunoliposome according to claim 24, **characterized in that** the antibody molecule or the functional fragment of the antibody comprises heavy and light chain variable regions of an antibody selected from an antibody produced by hybridoma m2E12, an antibody binding to the same epitope as that for the antibody, and a humanized antibody of the antibody.

28. The immunoliposome according to claim 25, **characterized in that** the antibody molecule or the functional fragment of the antibody comprises a heavy chain variable region sequence consisting of amino acid residues 1 to 139 of the amino acid sequence of SEQ ID NO: 3 described in the sequence listing and a light chain variable region sequence consisting of amino acid residues 1 to 131 of the amino acid sequence of SEQ ID NO: 4 described in the sequence listing.

29. An immunoliposome which contains the following components (a) and (b) and specifically binds to a cell surface receptor involved in apoptosis induction:

   (a) an endogenous ligand specifically binding to the cell surface receptor and having an activity of inducing the apoptosis of a cell via the receptor, or a functional fragment of the endogenous ligand; and
   (b) an amphiphilic vesicle-forming lipid.

30. The immunoliposome according to claim 29, **characterized in that** the immunoliposome exhibits an apoptosis-inducing activity stronger than that of the endogenous ligand against a cell expressing the cell surface receptor.

31. The immunoliposome according to claim 29 or 30, **characterized in that** the cell surface receptor is a death domain-containing receptor.

32. The immunoliposome according to claim 31, wherein the death domain-containing receptor is selected from the group consisting of Fas, DR4, DR5, and a TNF receptor.

33. The immunoliposome according to claim 32, wherein the endogenous ligand for the death domain-containing receptor is TRAIL or a Fas ligand.

34. The immunoliposome according to any one of claims 1 to 33, **characterized in that** the immunoliposome contains a sterol and a phospholipid as constituent lipids.

35. The immunoliposome according to claim 34, **characterized in that** the sterol as one of the constituent lipids of the immunoliposome is cholesterol.

36. The immunoliposome according to claim 35, **characterized in that** the immunoliposome contains the cholesterol as one of the constituent lipids of the immunoliposome at a content of 20 mol% to 50 mol% with respect to the number of moles of total lipids.

37. The immunoliposome according to any one of claims 34 to 36, **characterized in that** the phospholipid as one of the constituent lipids of the immunoliposome is phosphatidylcholine.

38. The immunoliposome according to claim 37, **characterized in that** the phosphatidylcholine as the constituent lipid of the immunoliposome contains an acyl group having 14 to 22 carbon atoms.

39. The immunoliposome according to claim 38, **characterized in that** the phosphatidylcholine as the constituent lipid of the immunoliposome contains an acyl group having 14 to 18 carbon atoms.

40. The immunoliposome according to any one of claims 34 to 39, wherein the immunoliposome further contains a hydrophilic polymer or a lipid derivative of the hydrophilic polymer as a constituent lipid.

41. The immunoliposome according to claim 40, **characterized in that** the hydrophilic polymer in the lipid derivative of the hydrophilic polymer has an average molecular weight of 500 to 20000.

42. The immunoliposome according to claim 41, **characterized in that** the hydrophilic polymer in the lipid derivative of the hydrophilic polymer has an average molecular weight of 2000 to 5000.

43. The immunoliposome according to any one of claims 40 to 42, **characterized in that** the immunoliposome contains the lipid derivative of the hydrophilic polymer at a content of 0.1 mol% to 10 mol% with respect to the number of moles of total lipids.

44. The immunoliposome according to claim 43, **characterized in that** the immunoliposome contains the lipid derivative of the hydrophilic polymer at a content of 1 mol% to 6 mol% with respect to the number of moles of total lipids.

45. The immunoliposome according to any one of claims 40 to 44, **characterized in that** the lipid derivative of the hydrophilic polymer as one of the constituent lipids of the immunoliposome is a polyethylene glycol-modified lipid.

46. The immunoliposome according to any one of claims 34 to 44, **characterized in that** the liposome is conjugated with the antibody via a thioether linkage using a lipid or a hydrophilic polymer having a terminal maleimide group.

47. The immunoliposome according to any one of claims 1 to 46, **characterized in that** the immunoliposome has an average particle size of 30 to 1000 nm.

48. The immunoliposome according to claim 47, **characterized in that** the immunoliposome has an average particle size of 30 to 200 nm.

49. The immunoliposome according to any one of claims 1 to 48, **characterized in that** a drug having an antitumor activity is encapsulated in the liposome.

50. The immunoliposome according to any one of claims 1 to 48, **characterized in that** a drug having a therapeutic effect on autoimmune disease or inflammatory disease is encapsulated in the liposome.

51. A pharmaceutical composition comprising an immunoliposome according to any one of claims 1 to 50 as an active ingredient.

52. An antitumor agent comprising an immunoliposome according to any one of claims 1 to 49 as an active ingredient.

53. An immunoliposome according to any one of claims 1 to 48 and 50 as an active.

[Figure 1]

[Figure 2]

[Figure 3]

[Figure 4]

[Figure 5]

[Figure 6]

[Figure 7]

[Figure 8]

[Figure 9]

[Figure 10]

[Figure 11]

[Figure 12]

[Figure 13]

[Figure 14]

[Figure 15]

[Figure 16]

[Figure 17]

[Figure 18]

[Figure 19]

[Figure 20]

EP 2 177 230 A1

[Figure 21]

[Figure 22]

96

[Figure 23]

[Figure 24]

[Figure 25]

[Figure 26]

## INTERNATIONAL SEARCH REPORT

| International application No. |
| --- |
| PCT/JP2008/063958 |

A. CLASSIFICATION OF SUBJECT MATTER
*A61K39/395*(2006.01)i, *A61K9/127*(2006.01)i, *A61K47/24*(2006.01)i, *A61K47/28*
(2006.01)i, *A61K47/42*(2006.01)i, *A61K47/44*(2006.01)i, *A61P29/00*(2006.01)i,
*A61P35/00*(2006.01)i, *A61P37/02*(2006.01)i
According to International Patent Classification (IPC) or to both national classification and IPC

B. FIELDS SEARCHED

Minimum documentation searched (classification system followed by classification symbols)
A61K39/395, A61K9/127, A61K47/24, A61K47/28, A61K47/42, A61K47/44,
A61P29/00, A61P35/00, A61P37/02

Documentation searched other than minimum documentation to the extent that such documents are included in the fields searched
Jitsuyo Shinan Koho        1922-1996   Jitsuyo Shinan Toroku Koho   1996-2008
Kokai Jitsuyo Shinan Koho   1971-2008   Toroku Jitsuyo Shinan Koho   1994-2008

Electronic data base consulted during the international search (name of data base and, where practicable, search terms used)
BIOSIS(STN), CAplus(STN), EMBASE(STN), MEDLINE(STN)

C. DOCUMENTS CONSIDERED TO BE RELEVANT

| Category* | Citation of document, with indication, where appropriate, of the relevant passages | Relevant to claim No. |
| --- | --- | --- |
| A | JP 9-110722 A  (Toray Industries, Inc.), 28 April, 1997 (28.04.97), Claims 1 to 9 (Family: none) | 1-52 |
| A | Sajid HUSSAIN, Mol. Cancer Ther., Vol.5, No.12, 2006, Pages 3170-3180 | 1-52 |
| A | Gabriella PAGNAN, Int. J. Cancer, Vol.81, 1999, Pages 268-274 | 1-52 |
| A | JP 2003-520603 A  (Medizinische Hochschule Hannover), 08 July, 2003 (08.07.03), Claim 18 & US 2003/0118553 A1    & EP 1254229 A & WO 2001/055385 A1 | 1-52 |

| ☒ | Further documents are listed in the continuation of Box C. | ☐ | See patent family annex. |

| * | Special categories of cited documents: | "T" | later document published after the international filing date or priority date and not in conflict with the application but cited to understand the principle or theory underlying the invention |
| "A" | document defining the general state of the art which is not considered   to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 15 August, 2008 (15.08.08) | 02 September, 2008 (02.09.08) |

| Name and mailing address of the ISA/ | Authorized officer |
| --- | --- |
| Japanese Patent Office | |
| Facsimile No. | Telephone No. |

Form PCT/ISA/210 (second sheet) (April 2007)

**INTERNATIONAL SEARCH REPORT**

International application No.

PCT/JP2008/063958

C (Continuation).    DOCUMENTS CONSIDERED TO BE RELEVANT

| Category* | Citation of document, with indication, where appropriate, of the relevant passages | Relevant to claim No. |
|---|---|---|
| A | JP 2002-537318 A  (Georgetown University), 05 November, 2002 (05.11.02), Claim 1 & US 2007/0065432 A1    & EP 1154756 A & WO 2000/050008 A2 | 1-52 |

Form PCT/ISA/210 (continuation of second sheet) (April 2007)

# EP 2 177 230 A1

| INTERNATIONAL SEARCH REPORT | International application No. |
|---|---|
| | PCT/JP2008/063958 |

**Box No. II    Observations where certain claims were found unsearchable (Continuation of item 2 of first sheet)**

This international search report has not been established in respect of certain claims under Article 17(2)(a) for the following reasons:

1. ☐  Claims Nos.:
   because they relate to subject matter not required to be searched by this Authority, namely:

2. ☒  Claims Nos.: 53
   because they relate to parts of the international application that do not comply with the prescribed requirements to such an extent that no meaningful international search can be carried out, specifically:
   In claim 53, it is merely described "an immunoliposome as claimed in any one of claims 1 to 48 or claim 50 is effective", which makes the scope of the invention according to claim 53 extremely unclear.

3. ☐  Claims Nos.:
   because they are dependent claims and are not drafted in accordance with the second and third sentences of Rule 6.4(a).

**Box No. III    Observations where unity of invention is lacking (Continuation of item 3 of first sheet)**

This International Searching Authority found multiple inventions in this international application, as follows:

1. ☐  As all required additional search fees were timely paid by the applicant, this international search report covers all searchable claims.

2. ☐  As all searchable claims could be searched without effort justifying additional fees, this Authority did not invite payment of additional fees.

3. ☐  As only some of the required additional search fees were timely paid by the applicant, this international search report covers only those claims for which fees were paid, specifically claims Nos.:

4. ☐  No required additional search fees were timely paid by the applicant.    Consequently, this international search report is restricted to the invention first mentioned in the claims; it is covered by claims Nos.:

**Remark on Protest**
the

☐ The additional search fees were accompanied by the applicant's protest and, where applicable, payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2007)

<table>
<tr><td>**INTERNATIONAL SEARCH REPORT**</td><td>International application No.<br>PCT/JP2008/063958</td></tr>
</table>

\<Subject of search\>

Claims 1 to 52 relate to "an immunoliposome binding specifically to a cell surface receptor participating in apoptosis induction" which comprises, as a constituting element thereof, a substance defined by a desired property, i.e., "an antibody binding specifically to a cell surface receptor participating in apoptosis induction, a functional fragment of this antibody, or a polypeptide containing the complementarity determining regions of the heavy chain and light chain of this antibody and binding specifically to the cell surface receptor" or "an endogenous ligand binding specifically to the cell surface receptor and inducing apoptosis into a cell via the receptor or a functional fragment of the endogenous ligand". Although claims 1 to 52 involve any substances having such properties, only small part of the claimed compounds are merely disclosed in the meaning within PCT Article 5 (for example, "TRA-8", (HFE7A", "MAB631", "rsTRAIL", etc.). Therefore, it is recognized that these claims are not supported by the disclosure in the description in the meaning within PCT Article 6.
Although the common technical knowledge at the point of the application is taken into consideration, the scopes of substances having the properties as "an antibody binding specifically to a cell surface receptor participating in apoptosis induction, a functional fragment of this antibody, or a polypeptide containing the complementarity determining regions of the heavy chain and light chain of this antibody and binding specifically to the cell surface receptor" or "an endogenous ligand binding specifically to the cell surface receptor and inducing apoptosis into a cell via the receptor or a functional fragment of the endogenous ligand" cannot be specified. Thus, claims 1 to 52 do not comply with the requirement of clearness in the meaning within PCT Article 6 too.
Such being the case, the search was made on the relationship between "an antibody binding specifically to the cell surface receptor participating in apoptosis induction" or "an endogenous ligand binding specifically to the cell surface receptor and inducing apoptosis into a cell via the receptor" and an immunoliposome and on the reasonable range based on the statements in Examples.

In claim 53, it is merely described "an immunoliposome as claimed in any one of claims 1 to 48 or claim 50 is effective", which makes the scope of the invention according to claim 53 extremely unclear. Therefore, no search was made on claim 53.

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9007861 A
- WO 9201047 A
- WO 9220791 A
- WO 9306213 A
- WO 9311236 A
- WO 9319172 A
- WO 9501438 A
- WO 9515388 A
- US 4946778 A
- US 5260203 A
- US 5091513 A
- US 5455030 A
- WO 2004061104 A

- WO 9851793 A
- WO 200183560 A
- WO 200294880 A
- WO 200354216 A
- WO 200683971 A
- WO 200722157 A
- US 6972323 B
- WO 2002097033 A
- WO 2005051351 A
- WO 2005026372 A
- US 5395619 A
- US 5213804 A
- WO 200337913 A

**Non-patent literature cited in the description**

- **D.D. Lasic.** Liposomes: From Physics to Applications. Elsevier Science Publishers, 1993
- *Cell Death and Differentiation,* 2003, vol. 10, 66-75
- **Wiley SR et al.** *Immunity,* December 1995, vol. 3 (6), 673-82
- **Golstein, P. et al.** *Cell,* 1995, vol. 81, 185-186
- **White, K et al.** *Science,* 1994, vol. 264, 677-683
- **Delgi-Esposti MA et al.** *Immunity,* December 1997, vol. 7 (6), 813-20
- **Chaudhary PM et al.** *Immunity,* December 1997, vol. 7 (6), 821-30
- *Journal of Cellular Physiology,* 2006, vol. 209, 1021-1028
- *Leukemia,* April 2007, vol. 21 (4), 805-812
- *Blood,* 2002, vol. 99, 1666-1675
- *Cellular Immunology,* January 1994, vol. 153 (1), 184-193
- *Journal of Immunology,* 1992, vol. 149, 3166-3173
- *European Journal of Immunology,* October 1993, vol. 23 (10), 2676-2681
- **Kohler ; Milstein.** *Nature,* 1975, vol. 256, 495-497
- Monoclonal Antibodies. Plenum Press, 1980, 365-367
- **Saiki, R.K. et al.** *Science,* 1988, vol. 239, 487-489
- **Gluzman, Y.** *Cell,* 1981, vol. 23, 175-182
- **Urlaub, G. ; Chasin, L.A.** *Proc. Natl. Acad. Sci. USA,* 1980, vol. 77, 4126-4220

- **Weir, D.M.** Handbook of Experimental Immunology. Blackwell Scientific Publications, 1987, vol. I. II. I
- **Kabat, E.A. ; Mayer, M.M.** Experimental Immunochemistry. Charles C Thomas Publisher, 1964
- **Kohler et al.** *Nature,* 1975, vol. 256, 495
- **Kohler et al.** *Eur. J. Immunol.,* 1977, vol. 6, 511
- **Milstein et al.** *Nature,* 1977, vol. 266, 550
- **Walsh.** *Nature,* 1977, vol. 266, 495
- **Barbara, B.M. ; Stanley, M.S.** Selected Methods in Cellular Immunology. W.H. Freeman and Company, 1980
- **Weir, D.M.** Handbook of Experimental Immunology. Blackwell Scientific Publications, 1978, vol. I, II, I
- *Proc. Natl. Acad. Sci. U.S.A.,* 1984, vol. 81, 6851-6855
- *Nature,* 1986, vol. 321, 522-525
- **Tomizuka, K. et al.** *Nature Genetics,* 1997, vol. 16, 133-143
- **Kuroiwa, Y. et al.** *Nucl. Acids Res.,* 1998, vol. 26, 3447-3448
- **Yoshida, H. et al.** Animal Cell Technology: Basic and Applied Aspects. Kluwer Academic Publishers, 1999, vol. 10, 69-73
- **Tomizuka, K. et al.** *Proc. Natl. Acad. Sci. USA,* 2000, vol. 97, 722-727

- **Wormstone, I.M. et al.** *Investigative Ophthalmology & Visual Science,* 2002, vol. 43 (7), 2301-2308
- **Carmen, S. et al.** *Briefings in Functional Genomics and Proteomics,* 2002, vol. 1 (2), 189-203
- **Siriwardena, D. et al.** *Ophthalmology,* 2002, vol. 109 (3), 427-431
- *Nature Biotechnology,* 2005, vol. 23 (9), 1105-1116
- *Annu. Rev. Immunol,* 1994, vol. 12, 433-455
- **Gluzman.** *Y. Cell,* 1981, vol. 23, 175-182
- **Urlaub, G. ; Chasin, L.A.** *Proc. Natl. Acad. Sci. U.S.A.,* 1980, vol. 77, 4126-4220
- **Millstein et al.** *Nature,* 1983, vol. 305, 537-539
- **Pluckthun.** The Pharmacology of Monoclonal Antibodies. Springer Verlag, 1994, vol. 113, 269-315
- *Nature Biotechnology,* 2005, vol. 23, 1126-1136
- **Huston, J.S. et al.** *Proc. Natl. Acad. Sci. U.S.A.,* 1988, vol. 85, 5879-5883
- *Nature Biotechnology,* 2005, vol. 23, 1137-1146
- Strategies for Protein Purification and Characterization: A Laboratory Course Manual. Cold Spring Harbor Laboratory Press, 1996
- Antibodies: A Laboratory Manual. Cold Spring Harbor Laboratory, 1988
- **D.D. Lasic.** Liposomes: From Physics to Applications. Elsevier Science Publishers, 1993, 1-171
- Liposomes: From Physics to Applications. Chemistry of lipids and liposomes
- **Yoshimori et al.** *Apoptosis,* 2005, vol. 10 (2), 323-329
- The Merck Manual of Diagnosis and Therapy. 1987
- **Rahway, N.J. ; Sladek et al.** *Metabolism and Action of Anti-Cancer Drugs,* 1987
- **Senior et al.** *Biochim. Biophys. Acta,* 1991, vol. 1062, 77-82
- **Blume et al.** *Biochim. Biophys. Acta,* 1990, vol. 1029, 91-97
- *Proc. Intern. Symp. Control. Rel. Bioact. Mater.,* 1990, vol. 17, 77-78
- *Proc. Natl. Acad. Sci. USA,* 1991, vol. 88, 11460-11464
- *Biochim. Biophys. Acta.,* 1991, vol. 1066, 29-36
- *Biochim. Biophys. Acta.,* 1992, vol. 1105, 193-200
- *Period. Biol.,* 1992, vol. 93, 349-352
- *Biochimica et Biophysica Acta,* 1985, vol. 812, 116
- *Mol Med.,* 2001, vol. 7 (10), 723
- *Biochimica et Biophysica Acta,* 1981, vol. 640, 66
- *J. Immunol Methods.,* 1983, vol. 65 (3), 295-306
- *Journal of Immunological Method,* 1984, vol. 75, 351
- *Biochemical and Biophysical Research Communications,* 1983, vol. 117, 399
- **Carlsson, J. et al.** *Biochem. J.,* 1978, vol. 173, 723
- **Traut, R.R. et al.** *Biochemistry,* 1973, vol. 12, 3266
- **Martin, F.J. et al.** *Biochemistry,* 1981, vol. 20, 4229
- **Miller et al.** *J. Biol. Chem,* 1965, vol. 257, 286
- *Biochimica et Biophysica Acta,* 1999, vol. 1420, 153-167
- *Biochemical and Biophysical Research Communications,* 1979, vol. 89, 1114
- *Liposome Technology,* 1983, vol. 155
- *Biochemistry,* 1992, vol. 31, 2850-2855
- *Antisense and Nucleic Acid Drug Development,* 2002, vol. 12, 311-325
- *Biochimica et Biophysica Acta,* 1995, vol. 1239, 133-144
- *Molecular Pharmaceutics,* vol. 3 (5), 525-530
- *BMC Immunology,* 2006, vol. 7, 24
- *Int J Oncol.,* 2003, vol. 23 (4), 1159-65
- **Dmitri Kirpotin et al.** *Biochemistry,* 1997, vol. 36, 66-75
- **Bo B. Lundberg et al.** *Int. J. Pharm,* 2000, vol. 205, 101-108
- **Maruyama K. et al.** *Biochimica et Biophysica,* 1995, vol. 1234, 74-80
- BMC Immunology. 2006, vol. 7, 24
- **G. Gregoriadis.** Liposome Technology Liposome Preparation and Related Techniques. CRC Press, vol. I-III
- **Paul S. Uster et al.** *FEBS letters,* 1996, vol. 386, 243-246
- **T. Ishida et al.** *FEBS letters,* 1999, vol. 460, 129-133
- **Maehama T et al.** *Anal Biochem.,* 2000, vol. 279 (2), 248
- **Serunian L.A. et al.** *Methods Enzymol.,* 1991, vol. 198, 78
- **Hoving EB et al.** *J Chromatogr B Biomed Appl.,* 1995, vol. 15, 671 (1-2), 341
- **Wenk M.R. et al.** *Nat Biotechnol.,* 2003, vol. 21 (7), 813
- **Bartlett GR et al.** *J Biol Chem.,* 1959, vol. 234 (3), 469
- **John Charles et al.** *Anal Biochem.,* 1980, vol. 1, 104 (1), 10
- **Takayama M et al.** *Clin Chim Acta.,* 1977, vol. 15, 79 (1), 93
- **Allain CC et al.** *Clin Chem.,* 1974, vol. 20 (4), 470

- Comprehensive Polymer Science. 1989
- *Int. J. Pharm.,* 2000, vol. 203, 255
- **You WW et al.** *Anal Biochem.,* vol. 15, 244 (2), 277
- **Allen et al.** *Biochimica et Biophysica Acta,* 1995
- **Raghupathy et al.** *The Journal of Biological Chemistry,* 1971
- **Hoedemaeker et al.** *J. Biol. Chem,* 1997
- *J. Clin. Invest.,* 1996, vol. 98 (2), 271-278
- *Int. Immunol.,* 1996, vol. 8 (10), 1595-1602
- **Sambrook, J. ; Fritsch, E.F. ; Maniatis, T.** Molecular Cloning. Cold Spring Harbor Laboratory Press, 1989
- *Nature Med.,* 2001, vol. 7 (8), 954-60
- *Int. Immunol.,* 2000, vol. 12 (4), 555-62

[Sequence Listing]

SEQUENCE LISTING

<110>   Daiichi Sankyo Co., Ltd.

<120>   Immunoliposome which can induce apoptosis to the cells
        expressing death domain-containing receptors

<130>   FP0830

<160>   4

<170>   PatentIn version 3.4

<210>   1
<211>   449
<212>   PRT
<213>   Artificial

<220>
<223>   Heavy chain amino acid sequence of humanized TRA-8

<220>
<223>   Inventor: Morita, Koji; Niwa,Takako; Ichikawa, Kimihisa
<223>   Inventor: Yoshida, Hiroko

<220>

<400>   1

Glu Val Gln Leu Val Glu Ser Gly Gly Gly Leu Val Gln Pro Gly Gly
1               5                   10                  15


Ser Leu Arg Leu Ser Cys Ala Ala Ser Gly Phe Thr Phe Ser Ser Tyr
            20                  25                  30


Val Met Ser Trp Val Arg Gln Ala Pro Gly Lys Gly Leu Glu Trp Val
        35                  40                  45


Ala Thr Ile Ser Ser Gly Gly Ser Tyr Thr Tyr Tyr Pro Asp Ser Val
    50                  55                  60


Lys Gly Arg Phe Thr Ile Ser Arg Asp Asn Ala Lys Asn Thr Leu Tyr
65                  70                  75                  80


Leu Gln Met Asn Ser Leu Arg Ala Glu Asp Thr Ala Val Tyr Tyr Cys
                85                  90                  95


Ala Arg Arg Gly Asp Ser Met Ile Thr Thr Asp Tyr Trp Gly Gln Gly
            100                 105                 110


Thr Leu Val Thr Val Ser Ser Ala Ser Thr Lys Gly Pro Ser Val Phe
        115                 120                 125

```
Pro Leu Ala Pro Ser Ser Lys Ser Thr Ser Gly Gly Thr Ala Ala Leu
    130                 135             140

Gly Cys Leu Val Lys Asp Tyr Phe Pro Glu Pro Val Thr Val Ser Trp
145                 150             155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
                180                 185                 190

Ser Ser Leu Gly Thr Gln Thr Tyr Ile Cys Asn Val Asn His Lys Pro
        195                 200                 205

Ser Asn Thr Lys Val Asp Lys Arg Val Glu Pro Lys Ser Cys Asp Lys
    210                 215                 220

Thr His Thr Cys Pro Pro Cys Pro Ala Pro Glu Leu Leu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
                260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
        290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Pro Ala Pro Ile Glu Lys
                325                 330                 335

Thr Ile Ser Lys Ala Lys Gly Gln Pro Arg Glu Pro Gln Val Tyr Thr
                340                 345                 350

Leu Pro Pro Ser Arg Glu Glu Met Thr Lys Asn Gln Val Ser Leu Thr
        355                 360                 365
```

```
Cys Leu Val Lys Gly Phe Tyr Pro Ser Asp Ile Ala Val Glu Trp Glu
    370             375             380

Ser Asn Gly Gln Pro Glu Asn Asn Tyr Lys Thr Thr Pro Pro Val Leu
385             390             395             400

Asp Ser Asp Gly Ser Phe Phe Leu Tyr Ser Lys Leu Thr Val Asp Lys
            405             410             415

Ser Arg Trp Gln Gln Gly Asn Val Phe Ser Cys Ser Val Met His Glu
            420             425             430

Ala Leu His Asn His Tyr Thr Gln Lys Ser Leu Ser Leu Ser Pro Gly
        435             440             445

Lys
```

```
<210>  2
<211>  213
<212>  PRT
<213>  Artificial

<220>
<223>  Light chain amino acid sequence of humanized TRA-8

<400>  2
```

```
Asp Ile Gln Met Thr Gln Ser Pro Ser Ser Leu Ser Ala Ser Val Gly
1               5               10              15

Asp Arg Val Thr Ile Thr Cys Lys Ala Ser Gln Asp Val Gly Thr Ala
            20              25              30

Val Ala Trp Tyr Gln Gln Lys Pro Gly Lys Ala Pro Lys Leu Leu Ile
            35              40              45

Tyr Trp Ala Ser Thr Arg His Thr Gly Val Pro Ser Arg Phe Ser Gly
    50              55              60

Ser Gly Ser Gly Thr Asp Phe Thr Leu Thr Ile Ser Ser Leu Gln Pro
65              70              75              80

Glu Asp Phe Ala Thr Tyr Tyr Cys Gln Gln Tyr Ser Ser Tyr Arg Thr
            85              90              95

Phe Gly Gln Gly Thr Lys Val Glu Ile Lys Arg Thr Val Ala Ala Pro
            100             105             110
```

Ser Val Phe Ile Phe Pro Pro Ser Asp Glu Gln Leu Lys Ser Gly Thr
              115                 120                 125

Ala Ser Val Val Cys Leu Leu Asn Asn Phe Tyr Pro Arg Glu Ala Lys
    130                 135                 140

Val Gln Trp Lys Val Asp Asn Ala Leu Gln Ser Gly Asn Ser Gln Glu
145                 150                 155                 160

Ser Val Thr Glu Gln Asp Ser Lys Asp Ser Thr Tyr Ser Leu Ser Ser
                165                 170                 175

Thr Leu Thr Leu Ser Lys Ala Asp Tyr Glu Lys His Lys Val Tyr Ala
              180                 185                 190

Cys Glu Val Thr His Gln Gly Leu Ser Ser Pro Val Thr Lys Ser Phe
        195                 200                 205

Asn Arg Gly Glu Cys
        210


<210>  3
<211>  470
<212>  PRT
<213>  Artificial

<220>
<223>  Heavy chain amino acid sequence of humanized HFE7A

<400>  3

Met Gly Trp Ser Cys Ile Ile Leu Phe Leu Val Ala Thr Ala Thr Gly
1               5                   10                  15

Val His Ser Gln Val Gln Leu Val Gln Ser Gly Ala Glu Val Lys Lys
              20                  25                  30

Pro Gly Ala Ser Val Lys Val Ser Cys Lys Ala Ser Gly Tyr Thr Phe
        35                  40                  45

Thr Ser Tyr Trp Met Gln Trp Val Lys Gln Ala Pro Gly Gln Gly Leu
        50                  55                  60

Glu Trp Met Gly Glu Ile Asp Pro Ser Asp Ser Tyr Thr Asn Tyr Asn
65                  70                  75                  80

Gln Lys Phe Lys Gly Lys Ala Thr Leu Thr Val Asp Thr Ser Thr Ser
              85                  90                  95

```
Thr Ala Tyr Met Glu Leu Ser Ser Leu Arg Ser Glu Asp Thr Ala Val
        100             105             110

Tyr Tyr Cys Ala Arg Asn Arg Asp Tyr Ser Asn Asn Trp Tyr Phe Asp
        115             120             125

Val Trp Gly Glu Gly Thr Leu Val Thr Val Ser Ser Ala Ser Thr Lys
    130             135             140

Gly Pro Ser Val Phe Pro Leu Ala Pro Ser Ser Lys Ser Thr Ser Gly
145             150             155             160

Gly Thr Ala Ala Leu Gly Cys Leu Val Lys Asp Tyr Phe Pro Glu Pro
                165             170             175

Val Thr Val Ser Trp Asn Ser Gly Ala Leu Thr Ser Gly Val His Thr
            180             185             190

Phe Pro Ala Val Leu Gln Ser Ser Gly Leu Tyr Ser Leu Ser Ser Val
        195             200             205

Val Thr Val Pro Ser Ser Ser Leu Gly Thr Gln Thr Tyr Ile Cys Asn
        210             215             220

Val Asn His Lys Pro Ser Asn Thr Lys Val Asp Lys Arg Val Glu Pro
225             230             235             240

Lys Ser Cys Asp Lys Thr His Thr Cys Pro Pro Cys Pro Ala Pro Glu
            245             250             255

Leu Leu Gly Gly Pro Ser Val Phe Leu Phe Pro Pro Lys Pro Lys Asp
            260             265             270

Thr Leu Met Ile Ser Arg Thr Pro Glu Val Thr Cys Val Val Val Asp
        275             280             285

Val Ser His Glu Asp Pro Glu Val Lys Phe Asn Trp Tyr Val Asp Gly
    290             295             300

Val Glu Val His Asn Ala Lys Thr Lys Pro Arg Glu Glu Gln Tyr Asn
305             310             315             320

Ser Thr Tyr Arg Val Val Ser Val Leu Thr Val Leu His Gln Asp Trp
            325             330             335
```

```
Leu Asn Gly Lys Glu Tyr Lys Cys Lys Val Ser Asn Lys Ala Leu Pro
            340             345             350

Ala Pro Ile Glu Lys Thr Ile Ser Lys Ala Lys Gly Gln Pro Arg Glu
            355             360             365

Pro Gln Val Tyr Thr Leu Pro Pro Ser Arg Glu Glu Met Thr Lys Asn
        370             375             380

Gln Val Ser Leu Thr Cys Leu Val Lys Gly Phe Tyr Pro Ser Asp Ile
385             390             395             400

Ala Val Glu Trp Glu Ser Asn Gly Gln Pro Glu Asn Asn Tyr Lys Thr
                405             410             415

Thr Pro Pro Val Leu Asp Ser Asp Gly Ser Phe Phe Leu Tyr Ser Lys
            420             425             430

Leu Thr Val Asp Lys Ser Arg Trp Gln Gln Gly Asn Val Phe Ser Cys
            435             440             445

Ser Val Met His Glu Ala Leu His Asn His Tyr Thr Gln Lys Ser Leu
        450             455             460

Ser Leu Ser Pro Gly Lys
465             470


<210>  4
<211>  237
<212>  PRT
<213>  Artificial

<220>
<223>  Light chain amino acid sequence of humanized HFE7A

<400>  4

Glu Thr Asp Thr Ile Leu Leu Trp Val Leu Leu Leu Trp Val Pro Gly
1               5               10              15

Ser Thr Gly Glu Ile Val Leu Thr Gln Ser Pro Gly Thr Leu Ser Leu
            20              25              30

Ser Pro Gly Glu Arg Ala Thr Leu Ser Cys Lys Ala Ser Gln Ser Val
        35              40              45

Asp Tyr Asp Gly Asp Ser Tyr Met Asn Trp Tyr Gln Gln Lys Pro Gly
        50              55              60
```

```
Gln Ala Pro Arg Leu Leu Ile Tyr Ala Ala Ser Asn Leu Glu Ser Gly
65                  70                  75                  80


Ile Pro Asp Arg Phe Ser Gly Ser Gly Ser Gly Thr Asp Phe Thr Leu
                85                  90                  95


Thr Ile Ser Arg Leu Glu Pro Glu Asp Phe Ala Val Tyr Tyr Cys Gln
            100                 105                 110


Gln Ser Asn Glu Asp Pro Arg Thr Phe Gly Gln Gly Thr Lys Leu Glu
            115                 120                 125


Ile Lys Arg Thr Val Ala Ala Pro Ser Val Phe Ile Phe Pro Pro Ser
        130                 135                 140


Asp Glu Gln Leu Lys Ser Gly Thr Ala Ser Val Val Cys Leu Leu Asn
145                 150                 155                 160


Asn Phe Tyr Pro Arg Glu Ala Lys Val Gln Trp Lys Val Asp Asn Ala
                165                 170                 175


Leu Gln Ser Gly Asn Ser Gln Glu Ser Val Thr Glu Gln Asp Ser Lys
            180                 185                 190


Asp Ser Thr Tyr Ser Leu Ser Ser Thr Leu Thr Leu Ser Lys Ala Asp
        195                 200                 205


Tyr Glu Lys His Lys Val Tyr Ala Cys Glu Val Thr His Gln Gly Leu
    210                 215                 220


Ser Ser Pro Val Thr Lys Ser Phe Asn Arg Gly Glu Cys
225                 230                 235
```

**84**